# FASCICULE DE BREVET EUROPEEN

(11) **EP 1 000 067 B1**
(45) Date de publication et mention de la délivrance du brevet: **23.10.2002**
(21) Numéro de dépôt: 98940327.4
(22) Date de dépôt: 24.07.1998
(51) Int. Cl.: C07D 513/06, A61K 31/55, C07D 517/06, C07D 223/16, C07D 281/02, C07D 243/14

(54) **THIAZOLOBENZOHETEROCYCLES, LEUR PREPARATION ET LES MEDICAMENTS LES CONTENANT**
THIAZOLOBENZOHETEROCYCLEN, IHRE HERSTELLUNG UND DIESE ENTHALTENDE PHATRMAZEUTISCHE ZUBEREITUNGEN
THIAZOLOBENZOHETEROCYCLES, PREPARATION AND MEDICINES CONTAINING SAME

(30) Priorité: 28.07.1997 FR 9709556
(43) Date de publication de la demande: 17.05.2000
(73) Titulaire: Aventis Pharma S.A., 92160 Antony (FR)
(72) Inventeur: HARDY, Jean-Claude, F-95800 Cergy Saint Christophe (FR); BOUQUEREL, Jean, F-93700 Drancy (FR); NEMECEK, Patrick, F-94320 Thiais (FR); PEYRONEL, Jean-François, F-91120 Palaiseau (FR)
(86) Numéro de dépôt international: FR9801638
(87) Numéro de publication internationale: WO99005147

(56) Documents cités:
- EP-A- 0 374 040

## Description

La présente invention concerne des composés de formule : 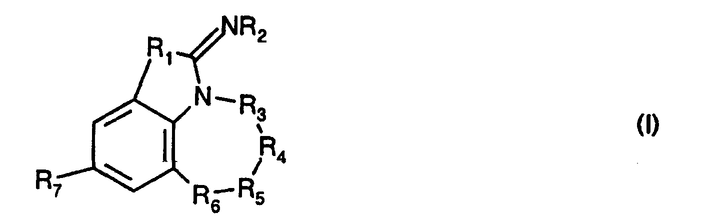 leurs isomères, racémiques, énantiomères, leurs sels, leurs procédés de préparation et les médicaments les contenant.

Dans la formule (I),
R₁ représente un atome de soufre ou de sélénium,
R₂ représente un atome d'hydrogène ou un radical alkyle,
   -R₃-R₄-R₅-R₆- représente une chaîne de formule -CH₂-CH₂-CH₂-CH₂-, -CH₂-CH₂-CH₂-CO-, -CH₂-CH₂-CH₂-CH(R₈)-, -CH₂-CH₂-CH₂-Se-, -CH₂-CH₂-Se-CH₂-, -CH₂-CH₂-CH₂-S-, -CH₂-CH₂-CH₂-SO-, -CH₂-CH₂-CH₂-SO₂-, -CH₂-CH₂-CH₂-O-, -CH₂-CH₂-CH₂-N(R₉)-, -CH₂-CH₂-CO-CH₂-, -CH₂-CH₂-CH(R₈)-CH₂-, -CH₂-CH₂-S-CH₂-, -CH₂-CH₂-SO-CH₂-, -CH₂-CH₂-SO₂-CH₂-, -CH₂-C(alk)(alk')-S-CH₂, -CH₂-C(alk)(alk')-SO-CH₂, -CH₂-C(alk)(alk')-SO₂-CH₂, -CH₂-CH(R₁₀)-S-CH₂-, -CH₂-CH(R₁₀)-SO-CH₂-, -CH₂-CH(R₁₀)-SO₂-CH₂-, -CH₂-CH₂-O-CH₂-, -CH₂-CH₂-N(R₉)-CH₂- ou -CH₂-CO-N(R₉)-CH₂-,
R₇ représente un radical polyfluoroalkyle ou polyfluoroalcoxy,
R₈ représente un radical hydroxy,
R₉ représente un atome d'hydrogène ou un radical alkyle ou benzyle,
R₁₀ représente un radical alkyle, -CH₂OH, -COOalk, -COOH ou -CONH₂,
alk représente un radical alkyle,
alk' représente un radical alkyle.

Dans les définitions qui précédent et celles qui seront citées ci-après, sauf mention contraire, les radicaux et portions alkyle contiennent 1 à 6 atomes de carbone en chaîne droite ou ramifiée.

Parmi les radicaux polyfluoroalkyle on peut citer les radicaux trifluorométhyle, 2,2,2-trifluoroéthyle, 1,1,2,2-tétrafluoroéthyle, perfluoroéthyle, perfluoropropyle, perfluorobutyle.

Parmi les radicaux polyfluoroalcoxy on peut citer les radicaux trifluorométhoxy, perfluoroéthoxy, 2,2,2-trifluoroéthoxy, 1,1,2,2-tétrafluoroéthoxy, 2,2,3,3,3-pentafluoropropoxy, perfluoropropoxy, perfluorobutoxy.

Les radicaux polyfluoroalkyle et polyfluoroalcoxy préférés sont les radicaux trifluorométhyle, trifluorométhoxy et pentafluoroéthoxy.

L'invention concerne également les sels d'addition des composés de formule (I) avec les acides minéraux ou organiques.

Les composés de formule (I) qui comportent un ou plusieurs centres asymétriques présentent des formes isomères, ces isomères et mélanges font partie de l'invention. Les racémiques et les énantiomères de ces composés font également partie de l'invention.

Les composés de formule (I) pour lesquels R₁ représente un atome de soufre ou de sélénium, R₂ représente un atome d'hydrogène, -R₃-R₄-R₅-R₆- représente une chaîne de formule -CH₂-CH₂-CH₂-CH₂-, -CH₂-CH₂-CH₂-CO-, -CH₂-CH₂-CH₂-CH(R₈)-, -CH₂-CH₂-CH₂-Se-, -CH₂-CH₂-Se-CH₂-, -CH₂-CH₂-CH₂-S-, -CH₂-CH₂-CH₂-O-, -CH₂-CH₂-CH₂-N(R₉)-, -CH₂-CH₂-CO-CH₂-, -CH₂-CH₂-CH(R₈)-CH₂-, -CH₂-CH₂-S-CH₂-, -CH₂-C(alk)(alk')-S-CH₂, -CH₂-CH(R₁₀)-S-CH₂-, -CH₂-CH₂-O-CH₂-, -CH₂-CH₂-N(R₉)-CH₂- ou -CH₂-CO-N(R₉)-CH₂-, R₈ représente un radical hydroxy, R₉ représente un atome d'hydrogène ou un radical alkyle ou benzyle et R₁₀ représente un radical alkyle, COOalk ou CONH₂ peuvent être préparés par action de thiocyanate de métal alcalin ou de sélénocyanate de métal alcalin avec un dérivé de formule : 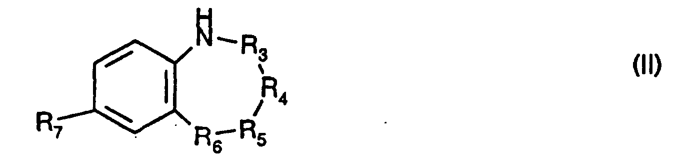 dans laquelle R₇ a les mêmes significations que dans la formule (I) et -R₃-R₄-R₅-R₆- représente une chaîne de formule -CH₂-CH₂-CH₂-CH₂-, -CH₂-CH₂-CH₂-CO-, -CH₂-CH₂-CH₂-CH(R₈)-, -CH₂-CH₂-CH₂-Se-, -CH₂-CH₂-Se-CH₂-, -CH₂-CH₂-CH₂-S-, -CH₂-CH₂-CH₂-O-, -CH₂-CH₂-CH₂-N(R₉)-, -CH₂-CH₂-CO-CH₂-, -CH₂-CH₂-CH(R₈)-CH₂-, -CH₂-CH₂-S-CH₂-, -CH₂-C(alk)(alk')-S-CH₂, -CH₂-CH(R₁₀)-S-CH₂-, -CH₂-CH₂-O-CH₂-, -CH₂-CH₂-N(R₉)-CH₂- ou -CH₂-CO-N(R₉)-CH₂-, R₈ représente un radical hydroxy, R₉ représente un atome d'hydrogène ou un radical alkyle ou benzyle et R₁₀ représente un radical alkyle, COOalk ou CONH₂, alk et alk' représentent un radical alkyle.

Cette réaction s'effectue généralement en présence de brome, de chlore, de chloramide ou de chlorure cuivrique, au sein d'un solvant organique tel que l'acide acétique, à une température comprise entre 15°C et la température d'ébullition du milieu réactionnel. Comme thiocyanate de métal alcalin ou sélénocyanate de métal alcalin, il est préférable d'utiliser le thiocyanate de potassium ou le sélénocyanate de potassium.

Les dérivés de formule (II) sont nouveaux et en tant que tels font partie de l'invention.

Les composés de formule (I) pour lesquels R₂ représente un radical alkyle peuvent être préparés par alkylation d'un composé de formule (I) correspondant pour lequel R₂ représente un atome d'hydrogène.

Cette alkylation s'effectue par toute méthode permettant d'alkyler une fonction imine. De préférence, on opère au moyen d'un dérivé Ra-X dans lequel Ra représente un radical alkyle et X représente un groupe réactif tel qu'un atome d'halogène (de préférence chlore, brome ou iode) ou un radical tosyloxy, au sein d'un solvant organique inerte tel qu'un alcool aliphatique (1-6C) (éthanol, propanol, butanol par exemple), une cétone (acétone, méthyléthylcétone par exemple) ou le diméthylformamide, en présence d'une base telle qu'un carbonate de métal alcalin (carbonate de potassium par exemple), à une température comprise entre 20°C et la température d'ébullition du milieu réactionnel.

Les composés de formule (I) pour lesquels R₂ représente un atome d'hydrogène ou un radical alkyle, -R₃-R₄-R₅-R₆- représente une chaîne de formule -CH₂-CH₂-CH(R₈)-CH₂- ou -CH₂-CH₂-CH₂-CH(R₈)-, et R₈ représente un radical hydroxy peuvent également être obtenus par réduction d'un composé de formule (I) correspondant pour lequel R₂ représente un atome d'hydrogène ou un radical alkyle et -R₃-R₄-R₅-R₆- représente une chaîne de formule -CH₂-CH₂-CO-CH₂- ou -CH₂-CH₂-CH₂-CO-.

Cette réaction s'effectue par toute méthode permettant de passer d'une cétone à un alcool. On opère généralement au moyen de borohydrure de sodium, au sein d'un alcool tel que le méthanol ou l'éthanol, à une température comprise entre 0 et 25°C.

Les composés de formule (I) pour lesquels R₂ représente un atome d'hydrogène ou un radical alkyle et -R₃-R₄-R₅-R₆- représente une chaîne de formule -CH₂-CH₂-CH₂-SO-, -CH₂-CH₂-CH₂-SO₂-, -CH₂-CH₂-SO-CH₂-, -CH₂-CH₂-SO₂-CH₂-, -CH₂-C(alk)(alk')-SO-CH₂, -CH₂-C(alk)(alk')-SO₂-CH₂, -CH₂-CH(R₁₀)-SO-CH₂- ou -CH₂-CH(R₁₀)-SO₂-CH₂- peuvent être préparés par oxydation d'un composé de formule (I) correspondant pour lequel la chaîne -R₃-R₄-R₅-R₆- représente une chaîne de formule -CH₂-CH₂₋CH₂-S-, -CH₂-CH₂-S-CH₂-, -CH₂-C(alk)(alk')-S-CH₂ ou -CH₂-CH(R₁₀)-S-CH₂-.

Cette oxydation s'effectue selon les méthodes connues d'oxydation des dérivés soufrés comme celles décrites par M. HUDLICKY, Oxidations in Organic Chemistry, ACS Monograph, 186, 252-263 (1990). Par exemple, on opère par action d'un peracide organique ou un sel d'un tel acide (acide percarboxylique ou persulfonique, notamment l'acide perbenzoïque, l'acide 3-chloroperbenzoïque, l'acide 4-nitroperbenzoïque, l'acide peracétique, l'acide pertrifluoracétique, l'acide performique, l'acide monoperphtalique) ou les peracides minéraux ou un sel d'un tel acide (par exemple l'acide periodique ou persulfurique), au sein d'un solvant inerte tel qu'un solvant chloré (chloroforme, dichlorométhane par exemple), à une température comprise entre 0 et 25°C. On peut utiliser également le peroxyde d'hydrogène ou un périodate (périodate de sodium par exemple), au sein d'un solvant inerte tel qu'un alcool aliphatique inférieur, l'eau ou un mélange de ces solvants, à une température comprise entre 0 et 20°C. Il est également possible d'opérer au moyen de tertiobutylhydroperoxyde en présence de tétraisopropylate de titane ou d'oxone^{R} (peroxymonosulfate de potassium) au sein d'un alcool aliphatique inférieur ou un mélange eau-alcool, à une température voisine de 25°C.

Les composés de formule (I) pour lesquels R₂ représente un atome d'hydrogène ou un radical alkyle, -R₃-R₄-R₅-R₆- représente une chaîne de formule -CH₂-CH(R₁₀)-S-CH₂ dans laquelle R₁₀ représente un radical -CH₂OH peuvent être préparés par réduction d'un composé de formule (I) correspondant pour lequel R₂ représente un atome d'hydrogène ou un radical alkyle, -R₃-R₄-R₅-R₆- représente une chaîne de formule -CH₂-CH(R₁₀)-S-CH₂ dans laquelle R₁₀ représente un radical -COOalk.

Cette réaction s'effectue par toute méthode connue permettant d'obtenir un alcool à partir de l'ester correspondant. De préférence, on opère au moyen d'hydroborure de sodium, au sein d'un alcool tel que l'éthanol, à la température d'ébullition du milieu réactionnel.

Les composés de formule (I) pour lesquels R₂ représente un atome d'hydrogène ou un radical alkyle, -R₃-R₄-R₅-R₆- représente une chaîne de formule -CH₂-CH(R₁₀)-S-CH₂ dans laquelle R₁₀ représente un radical -COOH peuvent être préparés par hydrolyse d'un composé de formule (I) correspondant pour lesquels R₂ représente un atome d'hydrogène ou un radical alkyle, -R₃-R₄-R₅-R₆- représente une chaîne de formule -CH₂-CH(R₁₀)-S-CH₂ dans laquelle R₁₀ représente un radical -COOalk.

Cette réaction s'effectue par toute méthode permettant de passer d'un ester à l'acide correspondant. Généralement on opère au moyen d'un hydroxyde de métal alcalin (soude par exemple), au sein d'un solvant inerte tel qu'un alcool (méthanol, éthanol par exemple), à une température comprise entre 15°C et la température d'ébullition du milieu réactionnel.

Les dérivés de formule (II) pour lesquels la chaîne -R₃-R₄-R₅-R₆- représente une chaîne de formule -CH₂-CH₂-CH₂-CH₂- et R₇ représente un radical polyfluoroalkyle ou polyfluoroalcoxy peuvent être obtenus par action de 1,4-dihalogénobutane sur le lithien d'une 4-polyfluoroalkylaniline ou 4-polyfluoroalcoxyaniline dont la fonction amine est protégée, suivie de la déprotection du NH.

Cette réaction s'effectue généralement au sein du tétrahydrofuranne, à une température de -78°C. Il est préférable de protéger la fonction amine sous forme d'un carbamate de tert-butyle; dans ce cas, la déprotection s'effectue au moyen d'acide trifluoroacétique, au sein d'un solvant organique inerte tel qu'un solvant chloré (chloroforme, dichlorométhane par exemple), à une température voisine de 20°C. De préférence, on utilise le 1-chloro-4-iodobutane. Le lithien est obtenu par action de tert-butyllithium dans le pentane sur une 4-polyfluoroalkylaniline ou 4-polyfluoroalcoxyaniline dont la fonction amine est protégée, au sein du tétrahydrofuranne, à une température de -78°C.

Les 4-polyfluoroalkylaniline et 4-polyfluoroalcoxyaniline sont commercialisées ou peuvent être obtenues par application ou adaptation des méthodes décrites dans J. Org. Chem., 29, 1 (1964), et dans les brevets US 3920444, US 2436100, DE 2606982, EP 205821 et EP546391.

Les dérivés de formule (II) pour lesquels -R₃-R₄-R₅-R₆- représente une chaîne de formule -CH₂-CH₂-CH₂-S-, -CH₂-CH₂-CH₂-Se-, -CH₂-CH₂-CH₂-O-, -CH₂-CH₂-CH₂-N(R₉)-, -CH₂-CH₂-S-CH₂-, -CH₂-C(alk)(alk')-S-CH₂, -CH₂-CH(R₁₀)-S-CH₂- dans laquelle R₁₀ représente un radical alkyle, -CH₂-CH₂-Se-CH₂-, -CH₂-CH₂-O-CH₂-, -CH₂-CH₂-N(R₉)-CH₂- peuvent être obtenus par réduction d'un dérivé de formule : 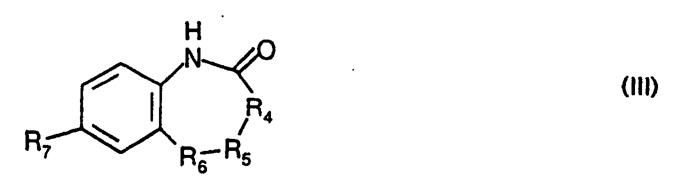 dans laquelle R₇ a les mêmes significations que dans la formule (I) et -R₄-R₅-R₆- représente une chaîne de formule -CH₂-CH₂-S-, -CH₂-CH₂-Se-, -CH₂-CH₂-O-, -CH₂-CH₂-N(R₉)-, -CH₂-S-CH₂-, -C(alk)(alk')-S-CH₂, -CH(R₁₀)-S-CH₂- dans laquelle R₁₀ représente un radical alkyle, -CH₂-Se-CH₂-, -CH₂-O-CH₂-, -CH₂-N(R₉)-CH₂- dans laquelle R₉ a les mêmes significations que dans la formule (I).

Cette réaction s'effectue généralement au moyen d'un agent réducteur tel que le tétrahydroaluminate de lithium, au sein d'un solvant organique inerte tel que le tétrahydrofuranne, à une température voisine de 20°C ou le complexe borane-diméthylsulfure, au sein d'un solvant inerte tel que le toluène, à la température d'ébullition du milieu réactionnel.

Les dérivés de formule (III) pour lesquels la chaîne -R₄-R₅-R₆- représente une chaîne de formule -CH₂-S-CH₂-, -C(alk)(alk')-S-CH₂, -CH(R₁₀)-S-CH₂- dans laquelle R₁₀ représente un radical alkyle ou -CH₂-Se-CH₂- peuvent être obtenus par cyclisation d'un dérivé de formule : 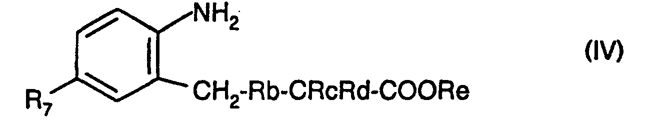 dans laquelle la fonction amino est éventuellement protégée et soit Rb représente un atome de soufre, Rc, Rd et Re représentent chacun un atome d'hydrogène ou un radical alkyle, et R₇ a les mêmes significations que dans la formule (I), soit Rb représente un atome de sélénium, Rc et Rd représentent chacun un atome d'hydrogène et Re représente un radical alkyle.

De préférence, la fonction amino est protégée sous forme de carbamate de tert-butyle. Lorsque Re représente un radical alkyle, lacyclisation s'effectuegénéralement au moyen d'acide trifluoroacétique, au sein d'un solvant organique inerte tel qu'un solvant chloré (chloroforme, dichlorométhane par exemple), à une température voisine de 20°C ou bien au moyen d'acide paratoluènesulfonique, au sein du toluène, à la température d'ébullition du milieu réactionnel. Lorsque Re représente un atome d'hydrogène, la cyclisation s'effectue, de préférence, au sein du xylène, par chauffage à la température d'ébullition du milieu réactionnel.

Les dérivés de formule (IV) pour lesquels Re représente un radical alkyle peuvent être obtenus par action de soufre ou de sélénium puis d'un dérivé Hal-CRcRd-COOalk pour lequel Hal représente un atome d'halogène, Rc et Rd ont les mêmes significations que précédemment sur le lithien d'une 2-méthyl-4-polyfluoroalkylaniline ou 2-méthyl-4-polyfluoroalcoxyaniline dont la fonction amino est protégée, de préférence sous forme de carbamate de tert-butyle, au sein du tétrahydrofuranne, à une température variant d'environ -70°C à voisine de 20°C. Le lithien de la 2-méthyl-4-polyfluoroalkylaniline ou 2-méthyl-4-polyfluoroalcoxyaniline dont la fonction amino est protégée peut être obtenu par action de tert-butyllithium sur une 2-méthyl-4-polyfluoroalkylaniline ou 2-méthyl-4-polyfluoroalcoxyaniline dont la fonction amino est protégée, au sein du tétrahydrofuranne, à une température d'environ -70°C. Les dérivés de formule (IV) pour lesquels Re représente un atome d'hydrogène peuvent être obtenus par hydrolyse d'un dérivé de formule (IV) correspondant pour lequel Re représente un radical alkyle. Cette hydrolyse s'effectue généralement au moyen de soude, au sein de l'éthanol, à une température comprise entre 15°C et la tempéraure d'ébullition du milieu réactionnel.

Les 2-méthyl-4-polyfluoroalkylaniline ou 2-méthyl-4-polyfluoroalcoxyaniline dont la fonction amino est protégée peuvent être obtenues par action d'iodométhane sur le lithien d'une 4-polyfluoroalkylaniline ou 4-polyfluoroalcoxyaniline dont la fonction amino est protégée au sein du tétrahydrofuranne, à une température variant d'environ -70°C à environ 20°C.

Les dérivés de formule (III) pour lesquels la chaîne -R₄-R₅-R₆- représente une chaîne de formule -CH₂-N(R₉)-CH₂- et les dérivés de formule (II) pour lesquels -R₃-R₄-R₅-R₆- représente une chaîne -CH₂-CO-N(R₉)-CH₂- peuvent être obtenus par action de chlorure de chloracétyle sur une aniline de formule: 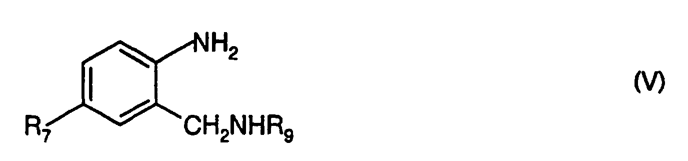 dans laquelle R₇ et R₉ ont les mêmes significations que dans la formule (I), et séparation des 2 dérivés.

Cette réaction s'effectue généralement au sein d'un solvant organique inerte tel qu'un éther (éther diéthylique par exemple), en présence d'hydrogénocarbonate de sodium, à une température voisine de 20°C.

Les anilines de formule (V) sont obtenues selon le schéma réactionnel suivant :

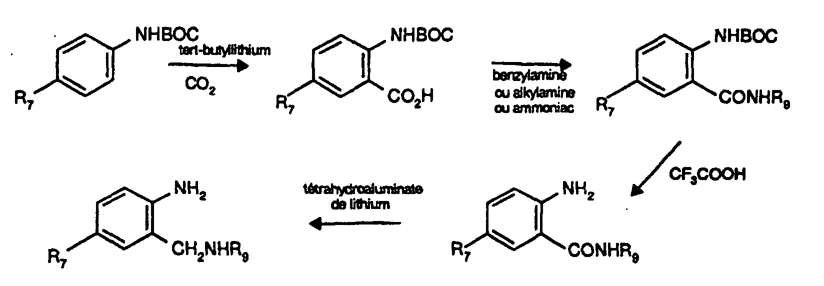

Dans ces formules, R₇ et R₉ ont les mêmes significations que dans la formule (I) et BOC représente le radical tert-butoxycarbonyle. Les conditions opératoires sont définies plus en détail dans l'exemple 8.

Les dérivés de formule (III) pour lesquels la chaîne -R₄-R₅-R₆- représente une chaîne -CH₂-O-CH₂- peuvent être obtenus par application ou adaptation de la méthode décrite par E. TESTA et L. FONTANELLA, II Farmaco, 1965, 20, 323-335 selon le schéma réactionnel suivant :

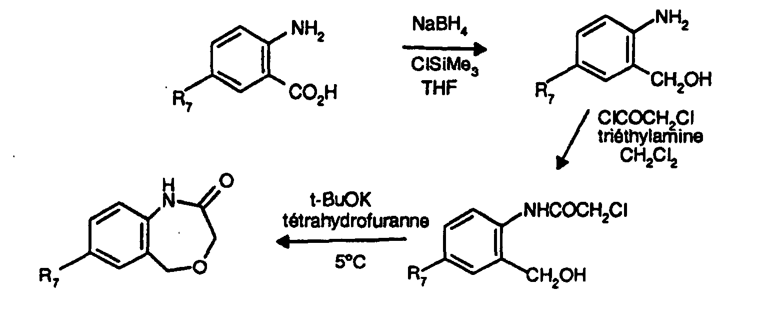

Dans ces formules R₇ a les mêmes significations que dans la formule (I), Me représente un radical méthyle et Bu représente un radical butyle.

Les dérivés de formule (III) pour lesquels la chaîne -R₄-R₅-R₆- représente une chaîne de formule -CH₂-CH₂-S-, -CH₂-CH₂-Se-, -CH₂-CH₂-O-, -CH₂-CH₂-N(R₉)- peuvent être obtenus à partir d'un dérivé de formule : 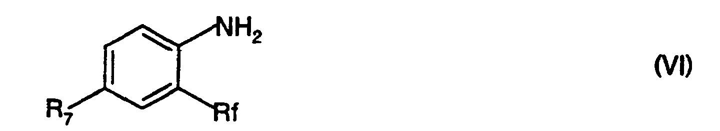 dans laquelle Rf représente un radical OH, SH, SeH ou NH(R₉), R₇ et R₉ ont les mêmes significations que dans la formule (I), par application ou adaptation des méthodes décrites dans les exemples et par X. HUANG, Synthesis, 851-852 (1984), W.C. LUMMA et coll., J. Med. Chem., 24, 93-101 (1981) et E.J. JACOBSEN et coll., J. Med. Chem., 39, 158-175 (1996).

Les dérivés de formule (VI) peuvent être obtenus par application ou adaptation des méthodes décrites par R. BELCHER et coll., J. Chem. Soc., 3846 (1954); B.L. MYLARY, J. Med. Chem., 34, 108-122 (1991); D.W. COMBS et coll., J. Med. Chem., 35, 172-176 (1992), W.C. LUMMA et coll., J. Med. Chem., 24, 93-101 (1981) et A.V. ZEIGER et coll., J. Org. Chem., 42 (3), 542 (1977).

Les dérivés de formule (II) pour lesquels -R₃-R₄-R₅-R₆- représente un radical -CH₂-CH₂-CH₂-CO- peuvent être obtenus par décarboxylation puis déprotection d'un dérivé de formule : 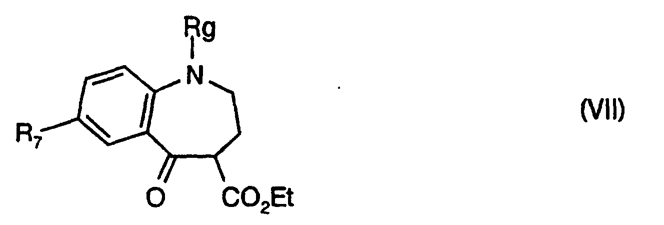 dans laquelle R₇ a les mêmes significations que dans la formule (I), Rg représente un radical p-toluènesulfonyle et Et représente un radical éthyle.

Cette réaction s'effectue généralement au moyen d'acide chlorhydrique au sein de l'acide acétique, à la température d'ébullition du milieu réactionnel. La déprotection s'effectue généralement au moyen de tournures de magnésium, au sein d'un mélange tétrahydrofuranne et méthanol, à une température voisine de 20°C.

Les dérivés de formule (VII) peuvent être obtenus selon le schéma réactionnel suivant :

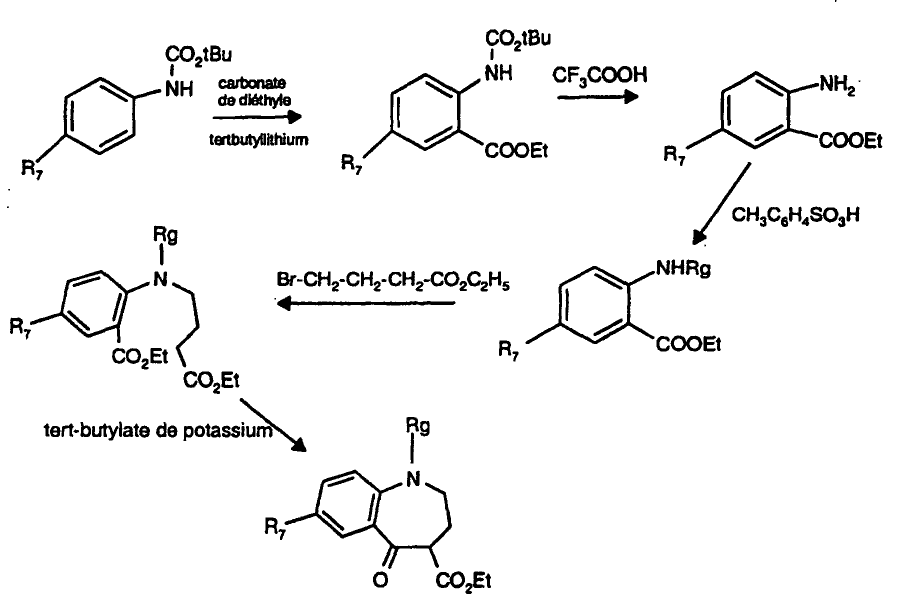

Dans ces formules, R₇ a les mêmes significations que dans la formule (I), Rg représente un radical p-toluènesulfonyle, Et représente un radical éthyle et tBu un radical tert-butyle. Les conditions opératoires sont définies plus en détail dans l'exemple 1.

Les dérivés de formule (II) pour lesquels -R₃-R₄-R₅-R₆- représente un radical -CH₂-CH₂-CH₂-CH(R₈)- ou -CH₂-CH₂-CH(R₈)-CH₂- et R₈ représente un radical hydroxy peuvent être obtenus par réduction d'un dérivé de formule (II) correspondant pour lequel R₂ représente un atome d'hydrogène ou un radical alkyle et -R₃-R₄-R₅-R₆- représente une chaîne de formule -CH₂-CH₂-CO-CH₂- ou -CH₂-CH₂-CH₂-CO-.

Cette réaction s'effectue par toute méthode permettant de passer d'une cétone à un alcool. On opère généralement au moyen de borohydrure de sodium, au sein d'un alcool tel que le méthanol ou l'éthanol, à une température comprise entre 0 et 25°C.

Les dérivés de formule (II) pour lesquels -R₃-R₄-R₅-R₆- représente un radical -CH₂-CH₂-CO-CH₂- peuvent être obtenus par décarboxylation-déprotection d'un dérivé de formule : 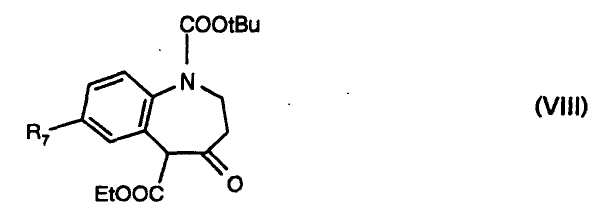 dans laquelle R₇ a les mêmes significations que dans la formule (I), tBu représente un radical tert-butyle et Et représente un radical éthyle.

Cette réaction s'effectue généralement au moyen d'acide chlorhydrique au sein de l'acide acétique, à la température d'ébullition du milieu réactionnel.

Les dérivés de formule (VIII) peuvent être obtenus selon le schéma réactionnel suivant :

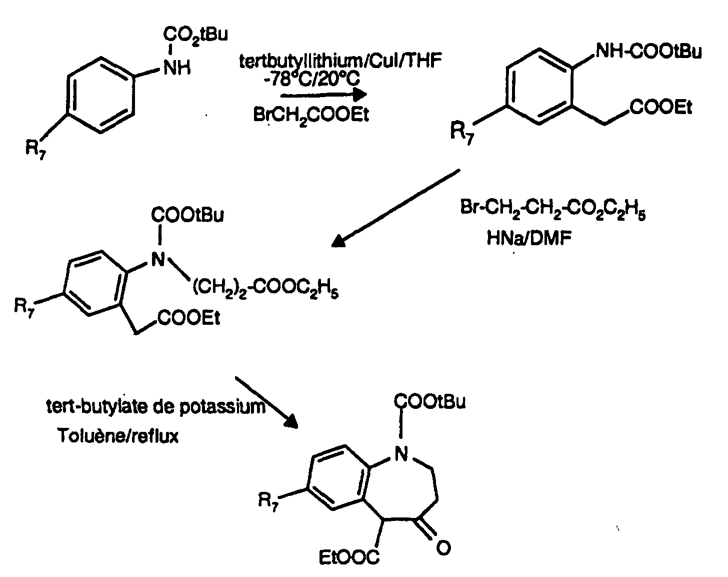

Dans ces formules, R₇ a les mêmes significations que dans la formule (I), Et représente un radical éthyle et tBu un radical tert-butyle.

Les dérivés de formule (II) pour lesquels -R₃-R₄-R₅-R₆- représente un radical -CH₂-CH(R₁₀)-S-CH₂- pour lequel R₁₀ représente un radical -CONH₂ peuvent être obtenus par action d'ammoniac sur un dérivé de formule (II) correspondant pour lequel -R₃-R₄-R₅-R₆- représente un radical -CH₂-CH(R₁₀)-S-CH₂- pour lequel R₁₀ représente un radical COOalk.

Cette réaction s'effectue généralement au sein d'un solvant inerte tel qu'un alcool (éthanol par exemple), à une température voisine de 20°C.

Les dérivés de formule (II) pour lesquels -R₃-R₄-R₅-R₆- représente un radical -CH₂-CH(R₁₀)-S-CH₂- pour lequel R₁₀ représente un radical -COOalk peuvent être obtenus par réduction d'un dérivé de formule : 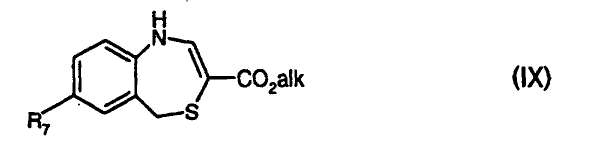 dans laquelle R₇ a les mêmes significations que dans la formule (I).

Cette réaction s'effectue de préférence au moyen de magnésium, au sein d'un solvant inerte tel qu'un alcool aliphatique (1-6C) (méthanol par exemple), à une température de 40°C.

Les dérivés de formule (IX) peuvent être obtenus selon le schéma réactionnel suivant : 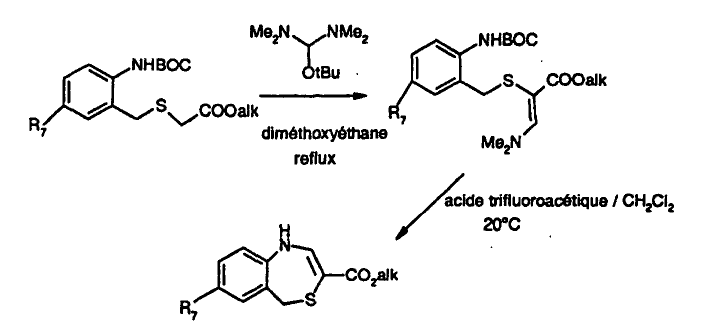 dans ces formules R₇ a les mêmes significations que dans la formule (I), alk représente un radical alkyle, BOC représente un radical tert-butoxycarbonyle.

Il est entendu pour l'homme de métier que, pour la mise en oeuvre des procédés selon l'invention décrits précédemment, il peut être nécessaire d'introduire des groupes protecteurs des fonctions amino afin d'éviter des réactions secondaires. Notamment, on opère selon les méthodes décrites par T.W. Greene, Protective Groups in Organic Synthesis, A. Wiley Interscience Publication (1981), ou par Mc Omie, Protective Groups in Organic Chemistry, Plenum Press (1973). Les fonctions amino peuvent par exemple être protégées par des radicaux méthoxycarbonyle, éthoxycarbonyle, t.butoxycarbonyle, allyloxycarbonyle, vinyloxycarbonyle, trichloréthoxycarbonyle, trichloracétyle, trifluoracétyle, chloracétyle, trityle, benzhydryle, benzyle, allyle, formyle, acétyle, benzyloxycarbonyle ou ses dérivés substitués ou sous forme de carbamates de tert-butyle ou de méthyle puis régénérées au moyen d'acide trifluoroacétique ou d'acide chlorhydrique dans le tétrahydrofuranne ou de carbamates de benzyle puis régénérées par hydrogénation après avoir mis en oeuvre le procédé selon l'invention.

Les mélanges réactionnels obtenus par les divers procédés décrits précédemment sont traités suivant des méthodes classiques physiques (évaporation, extraction, distillation, chromatographie, cristallisation par exemple) ou chimiques (formation de sels par exemple).

Les énantiomères des composés de formule (I) contenant au moins un site asymétrique peuvent être obtenus par synthèse à partir des précurseurs chiraux ou par dédoublement des racémiques par exemple par chromatographie sur phase stationnaire chirale type (*S,S*) WHELCK-01®, Chiralcel OJ® ou colonne chirale selon W. H. PIRKLE et coll., asymetric synthesis, vol 1, Academic Press (1983).

Les composés de formule (I) sous forme de base libre peuvent être éventuellement transformés en sels d'addition avec un acide minéral ou organique, par action d'un tel acide au sein d'un solvant organique tel qu'un alcool, une cétone, un éther ou un solvant chloré.

Comme exemples de sels pharmaceutiquement acceptables, peuvent être cités les sels d'addition avec les acides minéraux ou organiques tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate.

Les composés de formule (I) présentent des propriétés pharmacologiques intéressantes. Ces composés sont des anticonvulsivants et interfèrent avec la transmission glutamatergique et sont donc utiles pour traiter ou prévenir toutes les ischémies (telles l'ischémie focale ou globale) consécutives à des accidents vasculaires cérébraux tels que le stroke thromboembolique et hémorragique, un arrêt cardiaque, une hypotension artérielle, une intervention chirurgicale cardiaque, vasculaire ou pulmonaire ou une hypoglycémie sévère. Ils sont également utiles dans le traitement des effets dus à une anoxie, qu'elle soit périnatale ou consécutive à une noyade, une haute pression ou à des lésions cérébro-spinales. Ces composés peuvent également être utilisés pour traiter ou prévenir l'évolution de maladies neurodégénératives, de la chorée d'HUNTINGTON, de la maladie d'ALZHEIMER et autres démences, de la sclérose latérale amyotrophique ou d'autres maladies du motoneurone, de l'atrophie olivo-pontocérébelleuse et de la maladie de PARKINSON. Ces composés peuvent aussi être utilisés vis-à-vis des manifestations épileptogènes (épilepsie) et/ou convulsives, pour le traitement des traumatismes cérébraux ou spinaux, des traumatismes liés à la dégénérescence de l'oreille interne (R. PUJOL et coll., Neuroreport, 3, 299-302 (1992) ou de la rétine (J.L. MONSINGER et coll., Exp. Neurol., 113, 10-17 (1991), du tinnitus, de l'anxiété (KEHNE et coll., Eur. J. Pharmacol., 193, 283 (1991)), de la dépression (TRULLAS et coll.,Eur. J. Pharmacol., 185, 1 (1990)), de la schizophrénie (REYNOLDS, TIPS, 13, 116 (1992)), du syndrome de TOURETTE, des encéphalopathies hépatiques, des troubles du sommeil, des désordres du déficit attentionnel, des troubles des conditions hormonales (excès de la sécrétion de HG ou HL, sécrétion de corticostérone), en tant qu'analgésiques (DICKENSON et coll., Neurosc. Letters, 121, 263 (1991)), antiinflammatoires (SLUTA et coll., Neurosci. Letters, 149, 99-102 (1993)) antianorexiques (SORRELS et coll., Brain Res., 572, 265 (1992)), antimigraineux, antiémétiques et pour traiter les empoisonnements par des neurotoxines ou d'autres substances agonistes du récepteur NMDA ou AMPA, ainsi que les troubles neurologiques associés aux maladies virales telles que les méningites et encéphalites virales, le SIDA (LIPTON et coll., Neuron, 7, 111 (1991)), la rage, la rougeole et le tétanos (BAGETTA et coll., Br. J. Pharmacol., 101, 776 (1990)). Ces composés sont aussi utiles pour la prévention, la tolérance et la dépendance des symptômes d'abstinence aux drogues, à l'alcool et de l'inhibition de l'accoutumance et de la dépendance aux opiacés, barbituriques, amphétamine et benzodiazépines. Ils peuvent également être utilisés dans le traitement des déficits liés à des anomalies mitochondriales telles que la myopathie mitochondriale, le syndrome de LEBER, l'encéphalopathie de WERNICKE, le syndrome de RETT, l'homocystéinémie, l'hyperprolinémie, l'hydroxybutirique-aminoacidurie, l'encéphalopathie saturnine (intoxication chronique au plomb) et la déficience en sulfite oxydase.

L'activité de ces produits comme anticonvulsivant a été déterminée chez la souris selon la méthode de l'électrochoc maximal. Des souris blanches CD1 sont traitées intraveineusement avec les composés à tester en milieu salin, 10 minutes avant d'être soumis à un choc électrique (75mA; durée 0,04 seconde) au moyen d'électrodes occulaires. Normalement ce choc produit une convulsion tonique chez la souris non traitée, caractérisée par une extension des membres. Si la convulsion tonique ne survient pas, l'animal est considéré protégé. Dans ce test, les composés de formule (I) présentent une DE50 égale ou inférieure à 4 mg/kg.

L'activité de ces produits comme antiglutamate a été déterminée sur les convulsions induites par le glutamate selon une technique inspirée de celle de I. P. LAPIN, J. Neural. Transmission, 54, 229-238 (1982); l'injection du glutamate par voie intracérébroventriculaire étant effectuée selon une technique inspirée de celle de R. CHERMAT et P. SIMON, J. Pharmacol. (Paris), 6, 489-492 (1975). Leur DE₅₀ est inférieure à 10 mg/kg.

Les composés de formule (I) présentent une toxicité faible. Leur DL₅₀ est supérieure à 15 mg/kg par voie IV chez la souris.

Pour l'emploi médicinal, il peut être fait usage des composés de formule (I) tels quels ou à l'état de sels pharmaceutiquement acceptables, c'est-à-dire non toxiques aux doses d'utilisation.

Sont particulièrement intéressants, les composés de formule (I) pour lesquels R₇ représente un radical trifluorométhoxy ou trifluorométhyle.

Les composés de formule (I) préférés sont ceux pour lesquels R₁ représente un atome de soufre, R₂ représente un atome d'hydrogène, -R₃-R₄-R₅-R₆- représente une chaîne de formule -CH₂-CH₂-CH₂-CH₂-, -CH₂-CH₂-CH₂-CO-, -CH₂-CH₂-CH₂-CH(R₈)-, -CH₂-CH₂-CH₂-Se-, -CH₂-CH₂-Se-CH₂-, -CH₂-CH₂-CH₂-S-, -CH₂-CH₂-CH₂-SO-, -CH₂-CH₂-CH₂-SO₂-, -CH₂-CH₂-CH₂-O-, -CH₂-CH₂-CH₂-N(R₉)-, -CH₂-CH₂-CO-CH₂-, -CH₂-CH₂-CH(R₈)-CH₂-, -CH₂-CH₂-S-CH₂-, -CH₂-CH₂-SO-CH₂-, -CH₂-CH₂-SO₂-CH₂-,-CH₂-C(alk)(alk')-S-CH₂, -CH₂-CH(R₁₀)-S-CH₂-, -CH₂-CH₂-O-CH₂-, -CH₂-CH₂-N(R₉)-CH₂- ou -CH₂-CO-N(R₉)-CH₂-, R₇ représente un radical trifluorométhyle ou trifluorométhoxy, R₈ représente un radical hydroxy, R₉ représente un atome d'hydrogène ou un radical alkyle ou benzyle, R₁₀ représente un radical alkyle, -CH₂OH, -COOalk, -COOH ou -CONH₂,alk représente un radical alkyle et alk' représente un radical alkyle, leurs isomères, racémiques, énantiomères et leurs sels,.

Plus spécialement intéressants sont les composés de formule (I) suivants :
- 2-imino-9-trifluorométhoxy-4,5,6,7-tétrahydro-2H-thiazolo[5,4,3-jk][1]benzazépine-7-ol,
- 2-imino-9-trifluorométhoxy-4,5,6-7-tétrahydro-2H-thiazolo[5,4,3-jk][1]benzazépine,
- 2-imino-9-trifluorométhyl-4,5,6,7-tétrahydro-2H-thiazolo[5,4,3-jk][1]benzazépine,
- 7,7-dioxyde de 2-imino-9-trifluorométhoxy-5,6-dihydro-2H,4H-thiazolo[3,4,5-ef][1,5]benzothiazépine,
- 7-oxyde de 2-imino-9-trifluorométhoxy-5,6-dihydro-2H,4H-thiazolo-[3,4,5-ef][1,5]benzothiazépine,
- 2-imino-9-trifluorométhoxy-5,6-dihydro-2H,4H-thiazolo[3,4,5-ef][1,5]benzothiazépine,
- 6-benzyl-2-imino-9-trifluorométhoxy-6,7-dihydro-4H-thiazolo[3,4,5-kj][1,4] benzodiazépine-5-one,
- 6-benzyl-2-imino-9-trifluorométhoxy-4,5,6,7-tétrahydro-2H-thiazolo[3,4,5-kj][1,4]benzodiazépine,
- 2-imino-9-trifluorométhoxy-4,5-dihydro-2H,7H-thiazolo[3,4,5-de][4,1]benzothiazépine,
- 2-imino-9-trifluorométhyl-4,5-dihydro-2H,7H-thiazolo[3,4,5-de][4,1]benzothiazépine,
- 6,6-dioxyde de 2-imino-9-trifluorométhyl-4,5-dihydro-2H,7H-thiazolo[3,4,5-de][4,1]benzothiazépine,
- 7-oxyde de 2-imino-9-trifluorométhyl-5,6-dihydro-2H,4H-thiazolo[3,4,5-ef][1,5]benzothiazépine,
- 6,6-dioxyde de 2-imino-9-trifluorométhyl-5,6-dihydro-2H,4H-thiazolo[3,4,5-ef][1,5]benzothiazépine,
- 2-imino-9-trifluorométhyl-5,6-dihydro-2H,4H-thiazolo[3,4,5-ef][1,5]benzothiazépine,
- 2-imino-9-trifluorométhyl-4,5,6,7-tétrahydro-2H-thiazolo[5,4,3-jk][1]benzazépine-7-ol,- 6,6-dioxyde de 2-imino-9-trifluorométhoxy-4,5-dihydro-2H,7H-thiazolo[3,4,5-de][4,1]benzothiazépine,
- 6-oxyde de 2-imino-9-trifluorométhoxy-4,5-dihydro-2H,7H-thiazolo[3,4,5-de][4,1]benzothiazépine,
- 6-benzyl-2-imino-9-trifluorométhyl-6,7-dihydro-4H-thiazolo[3,4,5-kj][1,4] benzodiazépine-5-one,
- 6-benzyl-2-imino-9-trifluorométhyl-4,5,6,7-tétrahydro-2H-thiazolo[3,4,5-kj][1,4]benzodiazépine,
- 2-imino-5-méthyl-9-trifluorométhyl-4,5-dihydro-2H,7H-thiazolo[3,4,5-de][4,1] benzothiazépine,
- 5-carbamoyl-2-imino-9-trifluorométhyl-4,5-dihydro-2H,7H-thiazolo[3,4,5-de] [4,1]benzothiazépine,
- 5,5-diméthyl-2-imino-9-trifluorométhyl-2H,4H,7H-thiazolo[3,4,5-de][4,1]benzothiazépine,
- 5-hydroxyméthyl-2-imino-9-trifluorométhyl-4,5-dihydro-2H,7H-thiazolo[3,4,5-de][4,1]benzothiazépine,leurs isomères, racémiques, énantiomères et leurs sels.

Encore plus particulièrement préférés sont les composés suivants :
- (R,S)-6-oxyde de 2-imino-9-trifluorométhyl-4,5-dihydro-2H,7H-thiazolo[3,4,5-de][4,1]benzothiazépine,
- (+)-6-oxyde de 2-imino-9-trifluorométhyl-4,5-dihydro-2H,7H-thiazolo[3,4,5-de][4,1 ]benzothiazépine,
- (-)-6-oxyde de 2-imino-9-trifluorométhyl-4,5-dihydro-2H,7H-thiazolo[3,4,5-de][4,1 ]benzothiazépine,
- (R,S)-2-imino-5-méthyl-9-trifluorométhyl-4,5-dihydro-2H,7H-thiazolo[3,4,5-de][4,1]benzothiazépine,
- (+)-2-imino-5-méthyl-9-trifluorométhyl-4,5-dihydro-2H,7H-thiazolo[3,4,5-de][4,1]benzothiazépine,
- (-)-2-imino-5-méthyl-9-trifluorométhyl-4,5-dihydro-2H,7H-thiazolo[3,4,5-de][4,1]benzothiazépine
et leurs sels.

Les exemples suivants illustrent l'invention sans la limiter.

### EXEMPLE 1

On ajoute goutte à goutte en 10 minutes, à une température voisine de 20°C, 1,2 g de brome dilué dans 10 ml d'acide acétique à une solution de 1,7 g de thiocyanate de potassium et de 1,9 g de (R,S)-7-trifluorométhoxy-2,3,4,5-tétrahydro-1H-[1]benzazépine-5-ol dans 30 ml d'acide acétique. Le mélange réactionnel est agité pendant 20 heures à la même température, versé sur de la glace, alcalinisé par une solution d'ammoniaque à 20% et extrait par trois fois 100 ml d'acétate d'éthyle. Les phases organiques sont réunies, lavées par 100 ml d'eau distillée, séchées sur sulfate de magnésium et concentrées à sec sous pression réduite (2 kPa) à 40°C. Le résidu (0,4 g) est chromatographié sur gel de silice en éluant avec de l'acétate d'éthyle. Le produit isolé est repris dans un mélange de 4 ml d'éther isopropylique et d'éther de pétrole (50-50 en volumes) et on obtient ainsi 0,15 g de (R,S)-2-imino-9-trifluorométhoxy-4,5,6,7-tétrahydro-2H-thiazolo[5,4,3-jk][1]benzazépine-7-ol sous forme d'un solide crème fondant à 167°C [Analyse C12H11F3N2O2S, % calculé C : 47,37, H : 3,64, F : 18,73. N : 9,21, O : 10,52, S : 10,54, % trouvé C : 47,6, H : 3,5, F : 18,4, N : 9,1, S : 10,6].

Le (R,S)-7-trifluorométhoxy-2,3,4,5-tétrahydro-1H-[1]benzazépine-5-ol peut être préparé de la manière suivante : 1,08 g de tournures de magnésium est ajouté à une température voisine de 20°C à une solution de 3,6 g de (R,S)-1-(toluène-4-sulfonyl)-7-trifluorométhoxy-2,3,4,5-tétrahydro-1H-[1]benzazépine-5-ol dans 30 ml de tétrahydrofuranne et 40 ml de méthanol. Le mélange réactionnel est agité pendant 24 heures à la même température versé dans de l'eau distillée et la masse gélatineuse formée, reprise par l'éther éthylique, est filtrée puis lavée à trois reprises par 40 ml d'éther éthylique. Le filtrat jaune pâle ainsi obtenu est séché sur sulfate de magnésium, puis évaporé sous pression réduite à 50°C. On obtient ainsi 1,9 g de (R,S)-7-trifluorométhoxy-2,3,4,5-tétrahydro-1H-[1]benzazépine-5-ol sous la forme d'un solide crème fondant à 110°C.

Le (R,S)-1-(toluène-4-sulfonyl)-7-trifluorométhoxy-2,3,4,5-tétrahydro-1H-[1]benzazépine-5-ol peut être préparé de la manière suivante : à une solution de 3,7 g de 1-(toluène-4-sulfonyl)-7-trifluorométhoxy-2,3-dihydro-1H,4H-[1]benzazépine-5-one dans 40 ml d'éthanol on ajoute par petites portions 0,7 g de borohydrure de sodium et laisse sous agitation pendant 2 heures à une température voisine de 20°C. Après traitement habituel on obtient 3 g de (R,S)-1-(toluène-4-sulfonyl)-7-trifluorométhoxy-2,3,4,5-tétrahydro-1H-[1]benzazépine-5-ol sous forme d'un solide beige fondant à 160°C.

La 1-(toluène-4-sulfonyl)-7-trifluorométhoxy-2,3-dihydro-1H,4H-[1]benzazépine-5-one peut être préparée de la manière suivante : à une solution de 2,2 g de 5-oxo-1-(toluène-4-sulfonyl)-7-trifluorométhoxy-2,3,4,5-tétrahydro-1H-[1]benzazépine-4-carboxylate d'éthyle dans 20 ml d'acide acétique on ajoute 5 ml d'acide chlorhydrique. On observe la formation d'un précipité blanc et le mélange réactionnel est maintenu 4 heures à reflux. On concentre à sec sous pression réduite, reprend l'huile jaune obtenue par 300 ml d'éther éthylique et lave par une solution saturée d'hydrogénocarbonate de sodium. Après décantation, lavage à deux reprises par 50 ml d'eau distillée et par une solution saturée de chlorure de sodium, la phase organique est séchée sur du sulfate de magnésium, filtrée et concentrée à sec sous pression réduite (2,2 kPa) à 60°C. On obtient ainsi 1,58 g de 1-(toluène-4-sulfonyl)-7-trifluorométhoxy-2,3-dihydro-1H,4H-[1]benzazépine-5-one sous forme d'un solide beige fondant à 96°C.

Le 5-oxo-1-(toluène-4-sulfonyl)-7-trifluorométhoxy-2,3,4,5-tétrahydro-1H-[1]benzazépine-4-carboxylate d'éthyle peut être préparé de la manière suivante : à 275 ml de toluène anhydre porté à reflux on ajoute, sous atmosphère d'argon, 9,97 g de *tert*-butylate de potassium, puis on introduit, goutte à goutte, une solution de 23 g de 2-[(3-éthoxycarbonylpropyl)-(toluène-4-sulfonyl)amino]-5-trifluorométhoxybenzoate d'éthyle dans 300 ml de toluène anhydre. L'addition terminée on poursuit le chauffage pendant une heure. Après refroidissement on ajoute 90 ml d'une solution 1 N d'acide chlorhydrique, reprend à l'eau distillée et après décantation lave la phase organique par une solution saturée de chlorure de sodium, sèche sur sulfate de magnésium, filtre et concentre à sec sous pression réduite (2,2 kPa) à 60°C. Le résidu obtenu est dissous dans 100 ml de cyclohexane bouillant et après refroidissement le précipité apparu est séparé par filtration et séché à 40°C sous pression réduite (70 Pa). On obtient ainsi 10,75 g de 5-oxo-1-(toluène-4-sulfonyl)-7-trifluorométhoxy-2,3,4,5-tétrahydro-1H-[1]benzazépine-4-carboxylate d'éthyle sous forme d'une poudre beige fondant à 97°C.

Le 2-[(3-éthoxycarbonylpropyl)-(toluène-4-sulfonyl)amino]-5-trifluorométhoxybenzoate d'éthyle peut être préparé de la manière suivante : on chauffe à 80°C, pendant 17 heures, un mélange de 5,5 g de 2-(toluène-4-sulfonyl)amino-5-trifluorométhoxybenzoate d'éthyle, 5,6 g de carbonate de potassium, 3,18 g de 4-bromobutyrate d'éthyle dans 30 ml de dimethylformamide. On concentre à sec sous pression réduite (2,2 kPa) à 60°C. L'huile brune obtenue est dissoute dans de l'acétate d'éthyle et la solution est lavée par de l'eau distillée puis par une solution saturée de chlorure de sodium, séchée sur sulfate de magnésium et ensuite concentrée à sec sous pression réduite (2,2 kPa) à 50°C. On obtient ainsi 5,7 g de 2-[(3-éthoxycarbonylpropyl)-(toluène-4-sulfonyl)amino]-5-trifluorométhoxybenzoate d'éthyle sous forme d'une huile jaune [RMN Spectre ¹H dans DMSO-d6, T=300K, δ en ppm (300 Mhz) : 1,10 (3H, t, J=6Hz, CH₃), 1,30 (3H, t, J=6Hz, CH₃), 1,65 (2H, m, CH₂), 2,40 (5H, m, COCH₂ et PhCH₃), 3,45 et 3,70 (1H chacun, m, NCH₂), 4,00 (2H, q, J=6Hz, OCH₂), 4,25 (2H, q, J=6Hz, OCH₂), 7,05 (1H, d, J=8Hz, CH arom.), 7,40 (4H, s, 4 CH. tosyl), 7,65 (1H, dd, J= 8 et 2Hz, CH arom.), 7,70 (1H, d, J=2Hz, CH arom.)].

Le 2-(toluène-4-sulfonyl)amino-5-trifluorométhoxybenzoate d'éthyle peut être préparé de la manière suivante : à une solution de 17,5 g de 2-amino-5-trifluorométhoxybenzoate d'éthyle dans 70 ml de pyridine on ajoute sous agitation et à température voisine de 20°C 16,1 g de chlorure d'acide toluène-4-sulfonique. Après 24 heures d'agitation, on concentre à sec sous pression réduite (2,2 kPa) à 60°C. L'huile brune obtenue est dissoute dans de l'acétate d'éthyle et la solution est lavée successivement par une solution d'acide chlorhydrique (2 N), par de l'eau distillée et par une solution saturée de chlorure de sodium, puis séchée sur sulfate de magnésium et concentrée à sec sous pression réduite (2,2 kPa) à 50°C. L'huile orange obtenue reprise par l'éther de pétrole conduit à 27,8 g de 2-(toluène-4-sulfonyl)amino-5-trifluorométhoxybenzoate d'éthyle sous forme d'une poudre blanche fondant à 76°C.

Le 2-amino-5-trifluorométhoxybenzoate d'éthyle peut être préparé de la manière suivante : à 21 g de 2-*tert*-butoxycarbonylamino-5-trifluorométhoxybenzoate d'éthyle dans 150 ml de dichlorométhane sont ajoutés 55 ml d'acide trifluoroacétique. Après 4 heures à une température voisine de 20°C la solution noire obtenue est concentrée à sec. Le résidu est traité par une solution diluée d'hydrogénocarbonate de sodium et extrait par de l'éther de pétrole. La phase organique est lavée par de l'eau distillée jusqu'à pH neutre, séchée sur sulfate de magnésium et concentrée à sec sous pression réduite (2,2 kPa) à 50°C. On obtient ainsi 14 g de 2-amino-5-trifluorométhoxybenzoate d'éthyle sous forme d'huile brune [RMN Spectre ¹H dans DMSO-d6, T=300K, δ en ppm (300 Mhz) : 1,30 (3H, t, J=6Hz, CH₃), 4,30 (2H, q, J=6Hz, OCH₂), 6,85 (2H, s, NH₂), 6,87 (1H, d, J=8Hz, CH arom.), 7,30 (1H, dd, J=8 et 2Hz, CH arom.), 7,55 (1H, d, J=2Hz, CH arom.)].

Le 2-*tert*-butoxycarbonylamino-5-trifluorométhoxybenzoate d'éthyle peut être préparé de la manière suivante : sur une solution de 34,5 g de 4-trifluorométhoxyphénylcarbamate de *tert*-butyle dans 430 ml de tétrahydrofuranne anhydre, maintenue sous argon à -78°C, on coule en 1 heure 200 ml d'une solution 1,5 M de *tert*-butyllithium dans le pentane. On laisse remonter la température aux environs de -20°C et abandonne sous agitation 2,5 heures. De nouveau le milieu réactionnel est refroidi vers -78°C et on coule en une fois 75 ml de carbonate de diéthyle. Après 16 heures à une température voisine de 20°C, on ajoute 100 ml d'une solution aqueuse saturée de chlorure d'ammonium et 250 ml d'éther éthylique. Après décantation on extrait à nouveau la phase aqueuse par deux fois 200 ml d'éther éthylique. Les extraits organiques sont réunis, lavés à l'eau distillée et par une solution aqueuse saturée de chlorure de sodium, séchés sur sulfate de magnésium et concentrès à sec sous pression réduite. L'huile rouge obtenue est dissoute dans de l'éther de pétrole et la solution est filtrée sur gel de silice en lavant avec de l'éther de pétrole. Le filtrat concentré à sec livre une huile rouge qui cristallise. Après recristallisation dans 50 ml d'hexane, on obtient 21,5 g de 2-*tert*-butoxycarbonylamino-5-trifluorométhoxybenzoate d'éthyle sous forme d'un solide crème fondant à 82°C.

Le 4-trifluorométhoxyphénylcarbamate de *tert*-butyle peut être préparé de la manière suivante : on coule, en 10 minutes et à 0°C, une solution de 47 g de di-*tert*-butyldicarbonate dans 100 ml de tétrahydrofuranne anhydre sur une solution de 32,75 g de 4-trifluorométhoxyaniline dans 150 ml de tétrahydrofuranne anhydre. Le milieu réactionnel est agité à 80°C pendant 3 heures, puis concentré à sec. On obtient un produit cristallisé blanc qui est redissous dans 300 ml d'acétate d'éthyle. La solution est lavée trois fois par de l'eau distillée, séchée sur sulfate de magnésium et concentrée à sec. Par trituration dans l'éther de pétrole, filtration et séchage sous pression réduite (70 Pa) à 20°C, on obtient 35,5 g de 4-trifluorométhoxyphénylcarbamate de *tert*-butyle sous forme d'un solide blanc fondant à 110°C.

### EXEMPLE 2

On opère comme dans l'exemple 1 mais à partir de 0,7 g de brome dans 5 ml d'acide acétique, 1 g de 7-trifluorométhoxy-2,3,4,5-tétrahydro-1H-[1]benzazépine et de 1,46 g de thiocyanate de potassium dans 15 ml d'acide acétique. L'huile épaisse orange isolée est chromatographiée sur gel de silice en éluant avec un mélange d'acétate d'éthyle et d'éther de pétrole (70-30 en volumes). On obtient une huile jaune que l'on dissout dans l'éther isopropylique auquel on ajoute 0,45 ml d'une solution d'isopropanol chlorhydrique (environ 5 N). Le précipité blanc formé est filtré puis séché sous vide (70 Pa) à une température de 40°C. On obtient ainsi 0,52 g de chlorhydrate de 2-imino-9-trifluorométhoxy-4,5,6-7-tétrahydro-2H-thiazolo[5,4,3-jk][1]benzazépine sous forme d'un solide blanc fondant à 270°C (avec décomposition) [Analyse C12H12 CIF3N2OS, % calculé C : 44,38, H : 3,72, Cl : 10,92, F : 17,55, N : 8,63, O : 4,93, S : 9,87, % trouvé C : 44,3, H : 3,5, Cl : 10,9, F : 17,7, N : 9,1].

La 7-trifluorométhoxy-2,3,4,5-tétrahydro-1H-[1]benzazépine peut être préparée de la manière suivante : sur une solution de 2,3 g de 7-trifluorométhoxy-2,3,4,5-tétrahydro-1H-[1]benzazépine-1-carboxylate de *tert*-butyle dans 25 ml de dichlorométhane sont ajoutés 5 ml d'acide trifluoroacétique. Après 1 heure à température voisine de 20°C la solution rouge obtenue est concentrée à sec sous pression réduite. Le résidu est traité par une solution diluée d'hydrogénocarbonate de sodium et extrait par de l'éther éthylique. La phase organique est lavée par de l'eau distillée puis par une solution saturée de chlorure de sodium, séchée sur sulfate de magnésium et concentrée à sec sous pression réduite (2,2 kPa) à 50°C. On obtient ainsi 1,3 g d'huile brune qui est chromatographiée sur gel de silice en éluant avec un mélange d'éther de pétrole et de dichlorométhane (70-30 en volumes). Après évaporation sous pression réduite on isole 1,14 g de 7-trifluorométhoxy-2,3,4,5 -tétrahydro-1H-[1]benzazépine sous forme d'un solide pâteux utilisé tel quel dans l'étape suivante [RMN Spectre ¹H dans DMSO-d6, T=300K, δ en ppm (300 Mhz) : entre 1,50 et 1,70 (4H, m, 2 CH₂), 2,60 (2H, m, PhCH₂), 2,90 (2H, m, NCH₂), 5,40 (1H, s, NH), entre 6,80 et 7,00 (3H, m, 3 CH arom.)].

Le 7-trifluorométhoxy-2,3,4,5-tétrahydro-1H-[1]benzazépine-1-carboxylate de *tert*-butyle peut être préparé de la manière suivante : à une solution, maintenue sous atmosphère d'argon à -78°C, de 5,54 g de 4-trifluorométhoxyphénylcarbamate de *tert*-butyle dans 60 ml de tétrahydrofuranne anhydre, on ajoute en 1 heure 32 ml d'une solution 1,5 M de *tert*-butyllithium dans le pentane. Le mélange réactionnel est ensuite agité pendant 2,5 heures à une température voisine de -20°C. Il est de nouveau refroidi vers -78°C et on coule, goutte à goutte en 15 minutes, 2,65 ml de 1-chloro-4-iodobutane. Le mélange réactionnel est porté et maintenu à l'ébullition pendant 5 heures. Après refroidissement on ajoute 50 ml d'une solution aqueuse saturée de chlorure d'ammonium et extrait trois fois avec 200 ml au total d'éther éthylique. Les extraits organiques sont réunis, lavés à l'eau distillée et par une solution aqueuse saturée de chlorure de sodium, séchés sur sulfate de magnésium, puis concentrés à sec sous pression réduite. L'huile obtenue (6,65 g) est chromatographiée sur gel de silice en éluant avec un mélange d'éther de pétrole et de dichlorométhane (50-50 en volumes). On obtient ainsi 2,3 g de 7-trifluorométhoxy-2,3,4,5-tétrahydro-1H-[1]benzazépine-1-carboxylate de *tert*-butyle sous la forme d'une huile jaune [RMN Spectre ¹H dans DMSO-d6, T=393K, δ en ppm (200 Mhz) : entre 1,65 et 1,90 (4H, m, 2 CH₂), 2,70 (2H, m, PhCH₂), 3,50 (2H, t, J=6Hz, NCH₂), entre 7,05 et 7,35 (3H, m, 3 CH arom.)].

### EXEMPLE 3

On opère comme dans l'exemple 1, mais à partir de 2,38 g de brome dans 5 ml d'acide acétique, 3,2 g de 7-trifluorométhyl-2,3,4,5-tétrahydro-1H-[1]benzazépine et de 5 g de thiocyanate de potassium dans 35 ml d'acide acétique. L'huile jaune brun isolée est chromatographiée sur gel de silice en éluant avec de l'acétate d'éthyle. On obtient une huile jaune, que l'on dissout dans 15 ml d'éther isopropylique auxquels est additionné 1 ml d'une solution 1,94 N d'acide méthanesulfonique dans l'isopropanol. Le précipité blanc formé est filtré puis séché sous vide (70 Pa) à 50°C. On obtient ainsi 0,7 g de méthanesulfonate de 2-imino-9-trifluorométhyl-4,5,6,7-tétrahydro-2H-thiazolo[5,4,3-jk][1]benzazépine sous forme d'un solide blanc fondant à 226°C [Analyse C13H15F3N2O3S, % calculé C : 42,38, H : 4,1, F : 15,2, N : 7,5, O : 13,3, S : 17,41, % trouvé C : 42,4, H : 3,9, F : 15,2, N : 7,5, S :17,4].

La 7-trifluorométhyl-2,3,4,5-tétrahydro-1H-[1]benzazépine peut être préparée comme dans l'exemple 2, mais à partir de 5 g de 7-trifluorométhyl-2,3,4,5-tétrahydro-1H-[1]benzazépine-1-carboxylate de *tert*-butyle dans 50 ml de dichlorométhane et 5 ml d'acide trifluoroacétique. On obtient ainsi 3,3 g de 7-trifluorométhyl-2,3,4,5-tétrahydro-1H-[1]benzazépine sous forme d'une huile rouge utilisée telle quelle dans l'étape suivante [RMN Spectre ¹H dans DMSO-d6, T=300K, δ en ppm (300 Mhz) : entre 1,60 et 1,90 (4H, m, 2 CH₂), 2,75 (2H, t, J=6Hz, PhCH₂), 3,00 (2H, t, J=6Hz, NCH₂), 5,90 (1H, s large, NH), 6,90 (1H, d, J=8Hz, CH arom.), 7,28 (1H, dd, J=2 et 8Hz, CH arom.), 7,33 (1H, d, J=2Hz, CH arom.)].

Le 7-trifluorométhyl-2,3,4,5-tétrahydro-1H-[1]benzazépine-1-carboxylate de *tert*-butyle peut être préparé comme dans l'exemple 2, mais à partir de 26,1 g de 4-trifluorométhylphénylcarbamate de *tert*-butyle dans 300 ml de tétrahydrofuranne anhydre, 160 ml d'une solution 1,5 M de *tert*-butyllithium et 24 g de 1-chloro-4-iodobutane. On obtient 36 g d'une huile brune qui est chromatographiée sur gel de silice en éluant avec un mélange d'éther de pétrole et de dichlorométhane (70-30 en volumes). On obtient ainsi 16,2 g de 7-trifluorométhyl-2,3,4,5-tétrahydro-1H-[1 ]benzazépine-1-carboxylate de *tert*-butyle sous forme d'une huile verdâtre qui est utilisée telle quelle dans l'étape suivante.

### EXEMPLE 4

A une solution de 1 g de 2-imino-9-trifluorométhoxy-5,6-dihydro-2H,4H-thiazolo[3,4,5-ef][1,5]benzothiazépine dans 20 ml de dichlorométhane on ajoute, goutte à goutte, en 15 minutes et à une température voisine de 20°C, une solution de 1,55 g d'acide 3-chloroperbenzoïque (pureté 80%) dans 10 ml de dichlorométhane, et le mélange est ensuite agité pendant 24 heures à la même température. On ajoute ensuite 100 ml d'une solution aqueuse 1 M d'hydrogénocarbonate de sodium et on agite 1 heure à la même température. Après séparation des deux phases, on extrait la phase aqueuse deux fois par 50 ml de dichlorométhane et les extraits organiques réunis sont séchés sur sulfate de magnésium et concentrés à sec sous pression réduite (2 kPa). Le produit obtenu (1,4 g) est chromatographié sous pression d'azote (150 kPa) sur 30 g de gel de silice 20-45 µm contenus dans une colonne de 2 cm de diamètre, en éluant avec de l'acétate d'éthyle. Le produit obtenu (300 mg) est dissous dans 35 ml d'éthanol absolu, auxquels on additionne 69 µl d'acide méthanesulfonique. Après agitation pendant 1 heure à une température voisine de 20°C, la solution est concentrée à sec sous pression réduite (2kPa). Le produit obtenu est mis en suspension dans l'isopropanol, séparé par filtration, lavé avec de l'isopropanol et de l'éther isopropylique et séché sous pression réduite. On obtient ainsi 0,21 g de méthanesulfonate de 7,7-dioxyde de 2-imino-9-trifluorométhoxy-5,6-dihydro-2H,4H-thiazolo-[3,4,5-ef][1,5]benzothiazépine, sous forme d'un solide blanc fondant à une température supérieure à 260°C [Analyse C12H13F3N2O6S3, % calculé C : 33,17, H : 3,02, F : 13,12, N : 6,45, O : 22,10, S : 22,14, % trouvé C : 33,20, H : 2,71, F : 12,7, N : 6,30, S : 22,1].

### EXEMPLE 5

A une solution de 1 g de 2-imino-9-trifluorométhoxy-5,6-dihydro-2H,4H-thiazolo[3,4,5-ef][1,5]benzothiazépine dans 30 ml de dichlorométhane on ajoute, goutte à goutte, en 15 minutes et à une température voisine de 0°C, une solution de 770 mg d'acide 3-chloroperbenzoïque (pureté 80%) dans 5 ml de dichlorométhane et le mélange est ensuite agité pendant 1 heure à la même température. On ajoute ensuite 35 ml d'une solution aqueuse 1 M d'hydrogénocarbonate de sodium et on agite 1 heure à 20°C. Après séparation des deux phases, on extrait la phase aqueuse par 15 ml de dichlorométhane et les extraits organiques réunis sont séchés sur sulfate de magnésium et concentrés à sec sous pression réduite (2 kPa). Le produit obtenu est mis en suspension dans 20 ml d'éther isopropylique, séparé par filtration, lavé avec de l'éther isopropylique et séché sous pression réduite. Le produit obtenu (716 mg) est dissous dans 70 ml d'éthanol absolu et la solution est filtrée, puis additionnée de 160 µl d'acide méthanesulfonique. Après agitation pendant 1 heure à une température voisine de 20°C, la solution est concentrée à sec sous pression réduite (2kPa). Le produit obtenu est mis en suspension dans de l'isopropanol, séparé par filtration, lavé avec de l'isopropanol et de l'éther isopropylique et séché sous pression réduite. On obtient ainsi 0,70 g de méthanesulfonate de 7-oxyde de 2-imino-9-trifluorométhoxy-5,6-dihydro-2H,4H-thiazolo-[3,4,5-ef][1,5]benzothiazépine sous forme d'un solide crème fondant à une température supérieure à 260°C [Analyse C12H13F3N2O5S3, % calculé C : 34,45, H : 3,13, F : 13,62, N : 6,69, O : 19,12, S : 22,99, % trouvé C : 34,44, H : 2,86, F : 13,37, N : 6,68, S : 22,8].

### EXEMPLE 6

On opère comme à l'exemple 1, mais à partir de 0,4 ml de brome dans 5 ml d'acide acétique, 2 g de 8-trifluorométhoxy-2,3,4,5-tétrahydro[1,5]benzothiazépine et 1,71 g de thiocyanate de potassium dans 24 ml d'acide acétique. Le produit brut obtenu est chromatographié sous pression d'azote (150 kPa) sur 40 g de gel de silice 20-45 µm contenus dans une colonne de 2,5 cm de diamètre, en éluant avec un mélange d'acétate d'éthyle et de cyclohexane (50-50 en volumes). On dissout 0,5 g du produit obtenu (sur 1,5 g obtenu au total) dans 80 ml d'éthanol auxquels on ajoute 0,117 ml d'acide méthanesulfonique. Après 16 heures d'agitation à 20°C, la solution est concentrée à sec sous pression réduite (2 kPa). Le produit obtenu est mis en suspension dans 20 ml d'éther isopropylique, séparé par filtration, lavé avec de l'éther isopropylique et séché sous pression réduite (2 kPa) à 20°C. On obtient ainsi 0,56 g de méthanesulfonate de 2-imino-9-trifluorométhoxy-5,6-dihydro-2H,4H-thiazolo[3,4,5-ef][1,5]benzothiazépine, sous forme d'un solide beige fondant à une température supérieure à 260°C [Analyse C12H13F3N2O4S3, % calculé C : 35,82, H : 3,26, F : 14,16, N : 6,96, O : 15,9, S : 23,9, % trouvé C : 35,8, H : 3,0, F : 14,3, N : 7,00, S : 23,8].

La 8-trifluorométhoxy-2,3,4,5-tétrahydro-[1,5]benzothiazépine peut être préparée de la manière suivante : à 21,4 ml d'une solution environ 0,5 M de tétrahydroaluminate de lithium dans le tétrahydrofuranne, maintenue sous argon à 5°C, on ajoute goutte à goutte en 15 minutes, une solution de 2,5 g 8-trifluorométhoxy-2,3-dihydro-5H-[1,5]benzothiazépine-4-one dans 25 ml de tétrahydrofuranne. Le mélange réactionnel est agité ensuite 2 heures à 20°C et on ajoute successivement 500 ml d'eau distillée, 100 ml d'acétate d'éthyle et 100 ml d'une solution saturée de chlorure de sodium. Après décantation, la phase aqueuse est extraite trois fois avec 50 ml d'acétate d'éthyle. Les extraits organiques sont réunis, séchés sur sulfate de magnésium et concentrés à sec sous pression réduite (2kPa). On obtient ainsi 2 g de 8-trifluorométhoxy-2,3,4,5-tétrahydro-[1,5]benzothiazépine sous forme d'une huile jaune [Spectre ¹H dans DMSO-d6, T=300K, δ en ppm (300 Mhz) : 1,95 (2H, m, CH₂); 3,00 (2H, m, SCH₂); 3,30 (2H, m, NCH₂); 5,90 (1H, m, NH); 6,85 (1H, d, J=8Hz, CH arom.); 7,00 (1H, d, J=8Hz, CH arom.); 7,12 (1H, s, CH arom.)].

La 8-trifluorométhoxy-2,3-dihydro-5H-[1,5]benzothiazépine-4-one peut être préparée de la manière suivante : à une suspension de 11,5 g de 2-amino-6-trifluorométhoxybenzothiazole dans 115 ml d'eau distillée, on ajoute 70 g de potasse en pastilles par portions d'environ 10 g. Le mélange est ensuite agité pendant 16 heures au reflux. Après refroidissement vers 20°C, on ajoute 16,4 g de 3-bromopropionate d'éthyle puis 30 ml d'eau distillée et le milieu est agité pendant 16 heures à la même température. Le mélange est ensuite acidifié avec de l'acide chlorhydrique concentré à une température voisine de 5°C, extrait trois fois avec 50 ml d'acétate d'éthyle. Les extraits organiques sont réunis, lavés 3 fois avec de l'eau distillée, séchés sur sulfate de magnésium, et concentrés à sec sous pression réduite (2 kPa). Le produit brut obtenu est chromatographié sous pression d'azote (150 kPa) sur 250 g de gel de silice 20-45 µm contenus dans une colonne de 4 cm de diamètre, en éluant avec un mélange d'acétate d'éthyle et de cyclohexane (75-25 en volumes). On obtient ainsi 2,5 g d'un solide blanc qui est remis en suspension dans de l'éther isopropylique, séparé par filtration, lavé avec de l'éther isopropylique et séché sous pression réduite (2 kPa). On obtient ainsi 1,6 g 8-trifluorométhoxy-2,3-dihydro-5H-[1,5]benzothiazépine-4-one sous forme d'un solide blanc fondant à 188°C.

Le 2-amino-6-trifluorométhoxybenzothiazole peut être obtenu par la méthode décrite par L.M. YAGUPOL'SKII et coll., Zh. Obshch. Khim., 33 (7), 2301 (1963).

### EXEMPLE 7

On opère comme à l'exemple 1 mais à partir de 1,2 g de brome dans 2 ml d'acide acétique, 2,5 g de 4-benzyl-7-trifluorométhoxy-4,5-dihydro-1H,2H-[1,4]benzodiazépine-3-one et 1,6 g de thiocyanate de potassium dans 25 ml d'acide acétique. Le produit obtenu est chromatographié sous pression d'azote (150 kPa) sur 75 g de gel de silice 20-45 µm contenus dans une colonne de 2,5 cm de diamètre, en éluant avec de l'acétate d'éthyle. On dissout 0,6 g du produit obtenu (sur 2,25 g obtenus au total) dans 45 ml d'éthanol, auxquels on ajoute 0,1 ml d'acide méthanesulfonique. Après 3 heures d'agitation à 20°C, la solution est concentrée à sec sous pression réduite (2 kPa). Le produit obtenu est mis en suspension dans de l'éther éthylique, séparé par filtration, lavé avec de l'éther éthylique et séché sous pression réduite (2 kPa) à 20°C. On obtient ainsi 0,74 g de méthanesulfonate de 6-benzyl-2-imino-9-trifluorométhoxy-6,7-dihydro-4H-thiazolo[3,4,5-kj][1,4] benzodiazépine-5-one sous forme d'un solide crème fondant à une température supérieure à 260°C [Analyse C19H18F3N3O5S2, % calculé C : 46,62, H : 3,71, F : 11,64, N : 8,58, O : 16,34, S : 13,10, % trouvé C : 46,5, H : 3,4, F : 11,3, N : 8,5, S : 12,6].

### EXEMPLE 8

On opère comme à l'exemple 1 mais en utilisant 4,15 g de brome dans 5 ml d'acide acétique, 8,3 g de 4-benzyl-7-trifluorométhoxy-2,3,4,5-tétrahydro-1H-[1,4]benzodiazépine dans 120 ml d'acide acétique et 10 g de thiocyanate de potassium. On obtient 2,7 g d'une huile brune qui est chromatographiée successivement sur du gel de silice puis sur de l'alumine neutre désactivée avec 10% d'eau, en éluant dans les deux cas avec un mélange d'acétate d'éthyle et de cyclohexane (50-50 en volumes). On dissout le produit obtenu (0,68 g) dans 45 ml d'éthanol, auxquels on ajoute 0,23 ml d'acide méthanesulfonique. Après 2 heures d'agitation à 20°C, la solution est concentrée à sec sous pression réduite (2 kPa). Le produit obtenu est mis en suspension dans de l'éthanol, séparé par filtration, lavé avec de l'éthanol puis de l'éther éthylique et séché sous pression réduite (2 kPa) à 20°C. On obtient 0,32 g de diméthanesulfonate de 6-benzyl-2-imino-9-trifluorométhoxy-4,5,6,7-tétrahydro-2H-thiazolo[3,4,5-kj][1,4]benzodiazépine sous forme d'un solide beige fondant à une température supérieure à 260°C [Analyse C20H24F3N3O7S3, % calculé C : 42,02, H : 4,23, F : 9,97, N : 7,35, O : 19,59, S : 16,83, % trouvé C : 41,2, H : 4,2, F : 9,3, N : 7,2, S : 16,5].

La 4-benzyl-7-trifluorométhoxy-2,3,4,5-tétrahydro-1H-[1,4]benzodiazépine peut être préparée comme à l'exemple 6, mais en utilisant 95 ml de solution (environ 0,35 M) de tétrahydroaluminate de lithium dans le tétrahydrofuranne et de 6 g de 4-benzyl-7-trifluorométhoxy-4,5-dihydro-1H,3H-[1,4]benzodiazépine-2-one dans 40 ml de tétrahydrofuranne anhydre. On obtient 4,8 g de 4-benzyl-7-trifluorométhoxy-2,3,4,5-tétrahdro-1H-1,4-benzodiazépine sous forme d'une huile incolore [RMN Spectre ¹H dans DMSO-d6, T=300K, δ en ppm (300 Mhz) : 2,75 (2H, m, NCH₂); 3,00 (2H, m, NCH₂); 3,58 (2H, s, CH₂); 3,63 (2H, s, CH₂); 5,65 (1H, t, J=2Hz, NH); 6,80 (1H, d, J=2Hz, CH); 6,90 (1H, d, J=8Hz, CH); 7,00 (1H, dd, J=8 et 2Hz, CH); 7,30 (5H, m, 5 CH aryl)].

La 4-benzyl-7-trifluorométhoxy-4,5-dihydro-1H,3H-[1,4]benzodiazépine-2-one et la 4-benzyl-7-trifluorométhoxy-4,5-dihydro-1H,2H-[1,4]benzodiazépine-3-one peuvent être préparées de la manière suivante : à une solution, maintenue à une température voisine de 20°C, de 15,1 g de 2-benzylaminométhyl-4-trifluorométhoxyaniline dans 350 ml d'éther éthylique, on ajoute 16,5 g de chlorure de chloroacétyle puis 350 ml de solution aqueuse saturée d'hydrogénocarbonate de sodium. Le mélange réactionnel est agité pendant 1 heure à la même température. L'insoluble est ensuite éliminé par filtration et la phase organique séchée sur sulfate de magnésium, filtrée et concentrée à sec sous vide (2kPa). Le résidu d'évaporation est mis en solution dans 300 ml d'un mélange tétrahydrofuranne/alcool isopropylique (50/50 en volumes) auxquels on ajoute 17,6 g de *tert*-butoxyde de potassium et on agite 1 heure à une température voisine de 20°C. Après acidification avec 12 ml d'acide acétique et dilution avec 350 ml d'eau distillée, on extrait deux fois avec 100 ml d'acétate d'éthyle et les phases organiques réunies sont séchées sur sulfate de magnésium, filtrées et concentrées à sec sous vide (2 kPa). L'huile obtenue est reprise par 50 ml d'un mélange de cyclohexane et d'acétate d'éthyle (75/25 en volumes) où un solide cristallise. Celui est séparé par filtration, lavé avec 10 ml de ce même mélange et fournit 6,04 g de 4-benzyl-7-trifluorométhoxy-4,5-dihydro-1H,3H-[1,4]benzodiazépine-2-one sous forme d'un solide blanc fondant à 178°C. Le filtrat est concentré à sec sous vide (2 kPa) et le résidu est chromatographié sur 160 g de silice 20-45 µm contenus dans une colonne de 3,5 cm de diamètre, en éluant avec un mélange acétate d'éthyle/cyclohexane (50 / 50 en volumes). On obtient ainsi 5,72 g de 4-benzyl-7-trifluorométhoxy-4,5-dihydro-1H,2H-[1,4]benzodiazépine-3-one sous forme d'un solide blanc fondant à 124°C.

La 2-benzylaminométhyl-4-trifluorométhoxyaniline peut être préparée de la manière suivante : à 97 ml d'une solution 1 M de tétrahydroaluminate de lithium dans le tétrahydrofuranne, maintenus sous argon vers 20°C, on ajoute 100 ml de 1,4-dioxane anhydre, puis goutte à goutte une solution de 15 g de N-benzyl-2-amino-5-trifluorométhoxybenzamide dans 70 ml de 1,4-dioxane anhydre et le mélange est agité pendant 24 heures à reflux. Après hydrolyse, vers 5°C, par addition lente de 20 ml d'eau distillée, l'insoluble apparu est séparé par filtration, lavé avec de l'eau distillée puis avec de l'acétate d'éthyle et éliminé. Le filtrat est décanté et la phase organique est séchée sur sulfate de magnésium et concentrée à sec sous pression réduite (2 kPa). Le produit obtenu est chromatographié sous pression d'azote (150 kPa) sur 150 g de gel de silice 20-45 µm contenus dans une colonne de 3,5 cm de diamètre, en éluant avec un mélange de cyclohexane et d'acétate d'éthyle (75-25 en volumes). On obtient ainsi 7,39 g de 2-benzylaminométhyl-4-trifluorométhoxyaniline, sous forme d'une huile incolore [RMN Spectre ¹H dans DMSO-d6, T=300K, δ en ppm (300 Mhz) : 2,65 (1H, s, NH), 3,65 (2H, s, NCH₂), 3,72 (2H, s, NCH₂), 5,40 (2H, s, NH₂), 6,70 (1H, d, J=8Hz, CH arom.), 6,98 (1H, d, J=8Hz, CH arom.), 7,04 (1H, s, CH arom.), entre 7,20 et 7,50 (5H, m, 5CH aromatiques)].

Le N-benzyl-2-amino-5-trifluorométhoxybenzamide peut être préparé de la manière suivante : une solution de 5 g de N-benzyl-2-*tert*-butoxycarbonylamino-5-trifluorométhoxybenzamide dans 25 ml d'acide trifluoroacétique est conservée pendant 16 heures à une température voisine de 20°C, puis concentrée à sec sous pression réduite (2kPa). Le produit obtenu est dissous dans de l'acétate d'éthyle et la solution est lavée successivement avec deux fois 15 ml d'eau distillée et 15 ml de solution aqueuse saturée d'hydrogénocarbonate de sodium, puis concentrée à sec sous pression réduite (2 kPa). Le produit obtenu est mis en suspension dans du pentane, séparé par filtration et séché sous pression réduite (2 kPa). On obtient ainsi 3,55 g de N-benzyl-2-amino-5-trifluorométhoxybenzamide sous forme d'un solide crème fondant à 143°C.

Le N-benzyl-2-*tert*-butoxycarbonylamino-5-trifluorométhoxybenzamide peut être préparé de la manière suivante : à une solution, maintenue sous atmosphère d'argon à -10°C, de 20 g d'acide 2-*tert*-butoxycarbonylamino-5-trifluorométhoxybenzoïque, 16,9 g de 1-hydroxybenzotriazole et 6,8 g de benzylamine dans 400 ml de tétrahydrofuranne anhydre, on ajoute 12,9 g de N,N'-dicyclohexylcarbodiimide. Le mélange est agité pendant 2 heures à la même température puis pendant 16 heures à une température voisine de 20°C. Après refroidissement à 0°C, l'insoluble est séparé par filtration, lavé avec de l'acétate d'éthyle et le filtrat est concentré à sec sous pression réduite (2 kPa). Le produit obtenu est dissous dans 60 ml d'acétate d'éthyle et la solution est lavée deux fois par 25 ml d'une solution aqueuse saturée d'hydrogénocarbonate de sodium, séchée sur sulfate de magnésium, et concentrée à sec sous pression réduite (2kPa). Le produit obtenu est remis en suspension dans un mélange d'éther de pétrole et de pentane (50-50 en volumes), séparé par filtration, lavé avec le mélange d'éther de pétrole et de pentane et séché sous pression réduite (2 kPa). On obtient ainsi 21,7 g de N-benzyl-2-*tert*-butoxycarbonylamino-5-trifluorométhoxybenzamide sous forme d'un solide crème fondant à 144°C.

L'acide 2-*tert*-butoxycarbonylamino-5-trifluorométhoxybenzoique peut être préparé de la manière suivante : à une solution, maintenue à -70°C sous atmosphère d'argon, de 29 g de 4-trifluorométhoxy-phénylcarbamate de *tert*-butyle dans 300 ml de tétrahydrofuranne anhydre, on ajoute, goutte à goutte en 1 heure, 168 ml d'une solution 1,5 M de *tert*-butyllithium dans le pentane. Le mélange est agité pendant 3 heures 30 minutes à -20°C, refroidi de nouveau vers -70°C et un excès de dioxyde de carbone solide séché sur tétrahydrofuranne anhydre est ajouté par petites quantités. Le mélange est agité pendant 16 heures à une température voisine de 20°C puis on ajoute 500 ml d'une solution aqueuse saturée en chlorure d'ammonium et 200 ml d'acétate d'éthyle. La phase aqueuse est extraite deux fois par 200 ml d'acétate d'éthyle et les extraits organiques sont réunis, séchés sur sulfate de magnésium et concentrés à sec sous pression réduite (2kPa). Le produit obtenu est mis en suspension dans un mélange d'éther de pétrole et de pentane (50-50 en volumes), séparé par filtration, lavé avec du pentane et séché sous pression réduite (2 kPa). On obtient ainsi 31,7 g d'acide 2-*tert*-butoxycarbonylamino-5-trifluorométhoxybenzoïque sous forme d'un solide crème fondant entre 204 et 208°C.

### EXEMPLE 9

On opère comme à l'exemple 1 mais à partir de 1,5 g de brome dans 5 ml d'acide acétique, 2,34 g de 7-trifluorométhoxy-1,2,3,5-tétrahydro[4,1]benzothiazépine, 2,5 g de thiocyanate de potassium et 20 ml d'acide acétique. Le produit obtenu (3,42 g) est chromatographié sous pression d'azote (150 kPa) sur 80 g de gel de silice 20-45 µm contenus dans une colonne de 2,5 cm de diamètre, en éluant avec un mélange d'acétate d'éthyle et de cyclohexane (50-50 en volumes). Le produit obtenu est concrété par trituration dans 5 ml d'éther de pétrole, séparé par filtration et séché sous pression réduite (2 kPa). On dissout le produit obtenu (0,66 g) dans 30 ml d'éthanol, auxquels on ajoute 0,15 ml d'acide méthanesulfonique. Après 16 heures d'agitation à 20°C, la solution est concentrée à sec sous pression réduite (2 kPa). Le produit obtenu est recristallisé dans 10 ml d'un mélange d'éthanol et d'éther isopropylique (75-25 en volumes). On obtient ainsi 0,28 g de méthanesulfonate de 2-imino-9-trifluorométhoxy-4,5-dihydro-2H,7H-thiazolo[3,4,5-de][4,1]benzothiazépine sous forme d'un solide jaune fondant à une température supérieure à 260°C [Analyse C12H13F3N2O4S3, % calculé C : 35,82, H : 3,26, F : 14,16, N : 6,96, O : 15,9, S : 23,9, % trouvé C : 35,6, H : 3,0, F : 14,1, N : 6,9, S : 23,7].

La 7-trifluorométhoxy-1,2,3,5-tétrahydro-[4,1]benzothiazépine peut être préparée de la manière suivante : on opère comme à l'exemple 6, mais à partir de 3,4 g 7-trifluorométhoxy-1,5-dihydro-3H-[4,1]benzothiazépine-2-one dans 25 ml de tétrahydrofuranne anhydre, de 15,5 ml d'une solution 1 M de tétrahydroaluminate de lithium dans le tétrahydrofuranne et 15 ml de 1,4-dioxane anhydre. On obtient ainsi 2,34 g de 7-trifluorométhoxy-1,2,3,5-tétrahydro[4,1]benzothiazépine sous forme d'une huile jaunâtre [RMN Spectre ¹H dans DMSO-d6, T=300K, δ en ppm (300 Mhz) : 2,80 (2H, m, SCH₂), 3,25 (2H, m, NCH₂), 3,75 (2H, s, SCH₂-aryl), 5,60 (1H, t, J=5Hz, NH), 7,05 (2H, m, 2 CH arom.), 7,20 (1H, s, CH arom.)].

La 7-trifluorométhoxy-1,5-dihydro-3H-[4,1]benzothiazépine-2-one peut être préparée de la manière suivante : à une solution, maintenue à -70°C sous atmosphère d'argon, de 10,3 g de 2-méthyl-4-trifluorométhoxyphénylcarbamate de *tert*-butyle dans 150 ml de tétrahydrofuranne anhydre, on ajoute, goutte à goutte en 1 heure, 47 ml d'une solution 1,5 M de *tert*-butyllithium dans le pentane. Le mélange est agité pendant 2 heures à -20°C, refroidi vers -70°C, additionné de 1,1 g de soufre, puis agité pendant 1 heure à -20°C. Le mélange est refroidi vers -70°C, additionné de 5,4 g de bromoacétate de méthyle, puis agité pendant 16 heures à une température voisine de 20°C. Après hydrolyse par 50 ml d'eau distillée, on extrait par trois fois 50 ml d'acétate d'éthyle. Les extraits organiques réunis sont séchés sur sulfate de magnésium et concentrés à sec sous pression réduite (2kPa). Le produit obtenu est dissous dans 50 ml de dichlorométhane puis sont ajoutés 15 ml d'acide trifluoroacétique. Après 2 heures d'agitation à une température voisine de 20°C, le mélange est concentré à sec sous pression réduite (2 kPa). Le produit obtenu est dissous dans 40 ml d'acétate d'éthyle et la solution est lavée par 40 ml d'eau distillée, séchée sur sulfate de magnésium et concentrée à sec sous pression réduite (2 kPa). Le produit obtenu est mis en suspension dans de l'éther isopropylique, séparé par filtration, lavé avec le même solvant et séché sous pression réduite (2 kPa). On obtient ainsi 3,45 g de 7-trifluorométhoxy-1,5-dihydro-3H-[4,1]benzothiazépine-2-one sous forme d'un solide crème fondant à 190°C.

Le 2-méthyl-4-trifluorométhoxyphénylcarbamate de *tert*-butyle peut être préparé de la manière suivante : à une solution, maintenue à -70°C sous atmosphère d'argon, de 20 g de 4-trifluorométhoxyphénylcarbamate de *tert*-butyle dans 250 ml de tétrahydrofuranne anhydre, on ajoute, goutte à goutte en 1 heure, 106 ml d'une solution 1,5 M de *tert*-butyllithium dans le pentane. Le mélange est agité pendant 4 heures à -20°C, refroidi vers -70°C, additionné de 10,3 g de iodométhane, puis agité pendant 16 heures à une température voisine de 20°C. Après hydrolyse par 100 ml d'eau distillée, on extrait par trois fois 60 ml d'acétate d'éthyle. Les extraits organiques réunis sont séchés sur sulfate de magnésium et concentrés à sec sous pression réduite (2 kPa). Le produit obtenu est mis en suspension dans de l'éther de pétrole, séparé par filtration, lavé avec le même solvant et séché sous pression réduite (2 kPa). On obtient ainsi 15,1 g de 2-méthyl-4-trifluorométhoxyphénylcarbamate de *tert*-butyle sous forme d'un solide orange clair fondant à 98°C.

### EXEMPLE 10

On opère comme à l'exemple 1 mais à partir de 1,6 g de brome dans 5 ml d'acide acétique, 2,3 g de 7-trifluorométhyl-1,2,3,5-tétrahydro[4,1]benzothiazépine, 2,1 g de thiocyanate de potassium et 30 ml d'acide acétique. Après recristallisation dans l'éthanol absolu on obtient 1,15 g de méthanesulfonate de 2-imino-9-trifluorométhyl-4,5-dihydro-2H,7H-thiazolo[3,4,5-de][4,1]benzothiazépine, sous forme d'un solide blanc fondant à une température supérieure à 260°C [Analyse C12H13F3N2O3S3, % calculé C : 37,3, H : 3,39, F : 14,75, N : 7,25, O : 12,42, S : 24,89, % trouvé C : 37,2, H : 3,2, F : 14,4, N: 7,2, S : 24,6].

La 7-trifluorométhyl-1,2,3,5-tétrahydro-[4,1]benzothiazépine peut être préparée de la manière suivante : on opère comme à l'exemple 6, mais à partir de 3,6 g de 7-trifluorométhyl-1,5-dihydro-3H-[4,1]benzothiazépine-2-one dans 50 ml de tétrahydrofuranne anhydre et 50 ml de 1,4-dioxane anhydre et de 17,5 ml d'une solution 1 M de tétrahydroaluminate de lithium dans le tétrahydrofuranne. On obtient ainsi 2,4 g de 7-trifluorométhyl-1,2,3,5-tétrahydro[4,1]benzothiazépine sous forme d'un solide beige fondant à 94°C.

La 7-trifluorométhyl-1,5-dihydro-3H-[4,1]benzothiazépine-2-one peut être préparée de la manière suivante : on agite pendant 3 heures 30 minutes, à une température voisine de 20°C, un mélange de 14,3 g de (2-*tert*-butoxycarbonylamino-5-trifluorométhyl-benzylsulfanyl) acétate de méthyle dans 50 ml de dichlorométhane et de 15 ml d'acide trifluoroacétique. Le mélange est concentré à sec sous pression réduite (2 kPa) et le produit obtenu est dissous dans 40 ml de N,N-diméthylformamide et porté au reflux pendant 3 heures. Le mélange est concentré à sec sous pression réduite (2kPa). Le produit obtenu est dissous dans 50 ml d'acétate d'éthyle et la solution est lavée par deux fois 100 ml d'eau distillée, séchée sur sulfate de magnésium et concentrée à sec sous pression réduite (2 kPa). Le produit obtenu est mis en suspension dans de l'éther isopropylique, séparé par filtration, lavé avec un mélange d'acétate d'éthyle et de cyclohexane (50-50 en volumes) et séché sous pression réduite (2 kPa). On obtient 3,7 g de 7-trifluorométhyl-1,5-dihydro-3H-[4,1]benzothiazépine-2-one sous forme d'un solide beige fondant à 239°C.

Le (2-*tert*-butoxycarbonylamino-5-trifluorométhyl-benzylsulfanyl) acétate de méthyle peut être préparé de la manière suivante : à une solution, maintenue à -70°C sous atmosphère d'argon, de 13,7 g de 2-méthyl-4-trifluorométhylphénylcarbamate de *tert*-butyle dans 180 ml de tétrahydrofuranne anhydre, on ajoute, goutte à goutte en 1 heure 15 minutes, 67 ml d'une solution 1,5 M de *tert*-butyllithium dans le pentane. Le mélange est agité pendant 3 heures à -20°C, refroidi vers -70°C, additionné de 1,6 g de soufre, puis agité pendant 1 heure à -20°C. Le mélange est refroidi vers -40°C, additionné de 7,6 g de bromoacétate de méthyle, puis agité pendant 16 heures à une température voisine de 20°C. Après hydrolyse par 300 ml d'eau distillée, on extrait deux fois par 160 ml au total d'acétate d'éthyle. Les extraits organiques réunis sont séchés sur sulfate de magnésium et concentrés à sec sous pression réduite (2kPa). Le produit obtenu est dissous dans de l'éther de pétrole et la solution est filtrée puis concentrée à sec sous pression réduite (2 kPa). On obtient 14,3 g de (2-*tert*-butoxycarbonylamino-5-trifluorométhylbenzylsulfanyl) acétate de méthyle sous forme d'un solide jaune fondant à 54°C [RMN Spectre ¹H dans DMSO-d6, T=300K, δ en ppm (300 Mhz) : 1,45 (9H, s, (CH₃)₃), 3,25 (2H, s, SCH₂CO), 3,60 (3H, s, O CH₃), 4,00 (2H, s, SCH₂-aryl), 7,60 (2H, m, 2 CH arom.), 7,85 (1H, d, J=7Hz, CH arom.), 8,85 (1H, s, NH)].

Le (2-tert-butoxycarbonylamino-5-trifluorométhyl-benzylsulfanyl) acétate de méthyle peut également être préparé par la méthode suivante : sur une suspension de 5,2 g d'hydrure de sodium (80% en dispersion dans l'huile de vaseline) dans 59 ml de diméthylformamide, refroidie à 0°C et maintenue sous atmosphère d'azote, on coule 14 g de thioglycolate de méthyle et on agite ensuite 1 heure 30 mn à une température voisine de 20°C. On coule ensuite une solution de 37,8 g de 2-bromométhyl-4-trifluorométhyl phénylcarbamate de *tert*-butyle dans 30 ml de diméthylformamide et on agite ainsi 16 heures. Le milieu réactionnel est concentré à sec sous vide (2 kPa) et la pâte obtenue est reprise avec 200 ml d'eau distillée et extraite trois fois avec 50 ml d'acétate d'éthyle. Les phases organiques réunies sont séchées sur sulfate de magnésium, filtrées et concentrées à sec sous vide (2 kPa). L'huile ( 32,6 g ) est chromatographiée sur 360 g de silice 20-45 µm contenus dans une colonne de 4 cm de diamètre, en éluant avec un mélange cyclohexane/acétate d'éthyle (90/10 en volumes). On obtient ainsi 15,2 g de (2-tert-butoxycarbonylamino-5-trifluorométhyl-benzylsulfanyl) acétate de méthyle, sous forme d'un solide jaune fondant à 54°C.

Le 2-méthyl-4-trifluorométhylphénylcarbamate de *tert*-butyle est préparé de la manière suivante : à une solution, maintenue à -70°C sous atmosphère d'argon, de 30 g de 4-trifluorométhylphénylcarbamate de *tert*-butyle dans 390 ml de tétrahydrofuranne anhydre, on ajoute, goutte à goutte en 1 heure, 154 ml d'une solution 1,5 M de *tert*-butyllithium dans le pentane. Le mélange est agité pendant 4 heures à -20°C, refroidi vers -70°C, additionné de 16,4 g de iodométhane, puis agité pendant 16 heures à une température voisine de 20°C. Après hydrolyse par 300 ml d'eau distillée, on extrait par deux fois 160 ml au total d'acétate d'éthyle. Les extraits organiques réunis sont séchés sur sulfate de magnésium et concentrés à sec sous pression réduite (2kPa). Le produit obtenu est mis en suspension dans de l'éther de pétrole, séparé par filtration, lavé avec le même solvant et séché sous pression réduite (2 kPa). On obtient ainsi 20,5 g de 2-méthyl-4-trifluorométhylphénylcarbamate de *tert*-butyle sous forme d'un solide beige fondant à 101°C.

Le 2-bromométhyl-4-trifluorométhyl phénylcarbamate de *tert*-butyle peut être préparé de la manière suivantre : on porte à ébullition un mélange de 40 g de 2-méthyl-4-trifluorométhyl phénylcarbamate de *tert*-butyle, 26 g de N-bromosuccinimide et 1,5 g de peroxyde de benzoyle dans 290 ml de tétrachlorure de carbone et on illumine avec une lampe Mazdasol de 100 W pendant 4 heures. Le succinimide formé est éliminé par filtration et le filtrat est successivement lavé par 500 ml d'eau distillée, 200 ml de solution saturée en hydrogénocarbonate de sodium puis 200 ml d'eau distillée, séché sur sulfate de magnésium, filtré et concentré à sec sous vide (2 kPa). L'huile est reprise avec de l'éther de pétrole et les cristaux obtenus sont séparés par filtration. On obtient ainsi 37,9 g de 2-bromométhyl-4-trifluorométhyl phénylcarbamate de *tert*-butyle sous forme d'un solide blanc fondant à 98°C.

### EXEMPLE 11

On opère comme à l'exemple 4 mais à partir de 6,35 g de 2-imino-9-trifluorométhyl-4,5-dihydro-2H,7H-thiazolo[3,4,5-de][4,1]benzothiazépine et de 15,1 g d'acide 3-chloroperbenzoïque (pureté 80%) dans 130 ml de dichlorométhane. On obtient ainsi 4,12 g de méthanesulfonate de 6,6-dioxyde de 2-imino-9-trifluorométhyl-4,5-dihydro-2H,7H-thiazolo[3,4,5-de][4,1]benzothiazépine sous forme d'une poudre blanche fondant à une température supérieure à 260°C [Analyse C12H13F3N2O5S3, % calculé C : 34,45, H : 3,13, F : 13,62, N : 6,69, S : 22,99, % trouvé C : 34,46, H : 2,94, F : 13,17, N : 6,71, S : 23,11].

### EXEMPLE 12

On opère comme à l'exemple 5 mais à partir de 230 mg de de 2-imino-9-trifluorométhyl-4,5-dihydro-2H,7H-thiazolo[3,4,5-de][4,1]benzothiazépine et de 198 mg d'acide 3-chloroperbenzoïque (pureté 80%) dans 8 ml de dichlorométhane. On obtient ainsi 210 mg de méthanesulfonate de (R,S) 6-oxyde de 2-imino-9-trifluorométhyl-4,5-dihydro-2H,7H-thiazolo[3,4,5-de][4,1]benzothiazépine sous forme d'une poudre blanche fondant à une température supérieure à 260°C [Analyse C12H13F3N2O4S3, % calculé C : 35,82, H : 3,26, F : 14.16, N : 6,96, S : 23,9, % trouvé C : 36,2, H : 2,9, F : 13,9, N: 7,0, S : 23,5].

### EXEMPLE 13

On dissout 400 mg de (R,S) 6-oxyde de 2-imino-9-trifluorométhyl-4,5-dihydro-2H,7H-thiazolo[3,4,5-de][4,1]benzothiazépine dans 160 ml de dichlorométhane et on injecte sur 700 g de phase stationnaire chirale type (*S,S*) WHELCK-01® contenus dans une colonne de 60 mm de diamètre et de 400 mm de longueur, en éluant avec un mélange dichlorométhane/n-heptane/méthanol (50/50/2 en volumes) avec un débit de 70 ml/mn. On obtient 200 mg de (+) 6-oxyde de 2-imino-9-trifluorométhyl-4,5-dihydro-2H,7H-thiazolo[3,4,5-de][4,1]benzothiazépine ([α]_{D}²⁰=+27,7°±1° c=0,2% méthanol) et 200 mg de (-)6-oxyde de 2-imino-9-trifluorométhyl-4,5-dihydro-2H,7H-thiazolo[3,4,5-de][4,1]benzothiazépine ([α]_{D}²⁰=-28,2°±1° c=0,2% méthanol).

On dissout 200 mg de (+) 6-oxyde de 2-imino-9-trifluorométhyl-4,5-dihydro-2H,7H-thiazolo[3,4,5-de][4,1]benzothiazépine dans 30 ml d'éthanol avec 70 mg d'acide méthanesulfonique et on agite 16 heures à une température voisine de 20°C. Le milieu est ensuite concentré à sec sous vide (2 kPa) et la laque incolore est reprise avec 10 ml d'acétone. Le solide est séparé par filtration, lavé avec 2 fois 2 ml d'acétone. On obtient 240 mg de méthanesulfonate de (+) 6-oxyde de 2-imino-9-trifluorométhyl-4,5-dihydro-2H,7H-thiazolo[3,4,5-de][4,1]benzothiazépine sous forme d'un solide blanc fondant à 230°C (fusion collante) [Analyse C12H13F3N2O4S3 % calculé C : 35,82, H : 3,26, F : 14,16, N : 6,96, S : 23,9, % trouvé C : 35,51, H : 2,78, F : 14,26, N : 6,75, S : 23,95; [α]_{D}²⁰=+73,1°±1,1° c=0,5% méthanol]

On dissout 200 mg de (-) 6-oxyde de 2-imino-9-trifluorométhyl-4,5-dihydro-2H,7H-thiazolo[3,4,5-de][4,1]benzothiazépine dans 30 ml d'éthanol avec 70 mg d'acide méthanesulfonique et on agite 16 heures à une température voisine de 20°C. Le milieu est ensuite concentré à sec sous vide (2 kPa) et la laque incolore est reprise avec 10 ml d'acétone. Le solide est séparé par filtration, lavé avec 2 fois 2 ml d'acétone, et on obtient 230 mg de méthanesulfonate de (-) 6-oxyde de 2-imino-9-trifluorométhyl-4,5-dihydro-2H,7H-thiazolo[3,4,5-de][4,1]benzothiazépine sous forme d'un solide blanc fondant à 235°C (fusion collante) [Analyse C12H13F3N2O4S3 % calculé C : 35,82, H : 3,26, F : 14.16, N : 6,96, S : 23,9, % trouvé C : 35,70, H : 3,14, F : 13,30, N : 6,91, S : 24,23; [α]_{D}²⁰=-74,2°±1,2° c=0,5% dans le méthanol].

### EXEMPLE 14

A une solution, sous argon et refroidie à 0°C, de 1,2 g de 2-imino-9-trifluorométhyl-5,6-dihydro-2H,4H-thiazolo[3,4,5-ef][1,5]benzothiazépine dans 20 ml de dichlorométhane, on ajoute 0,92 g d'acide 3-chloroperbenzoïque (pureté 80%). Le mélange est agité pendant 1 heure 30 minutes à la même température. Après chromatographie en éluant avec de l'acétate d'éthyle et traitement par de l'acide méthanesulfonique, on obtient 0,88 g de méthanesulfonate de 7-oxyde de 2-imino-9-trifluorométhyl-5,6-dihydro-2H,4H-thiazolo[3,4,5-ef][1,5]benzothiazépine sous forme d'un solide blanc fondant à une température supérieure à 260°C [Analyse C12H13F3N2O4S3, % calculé C : 35,82, H : 3,26, F : 14,16, N : 6,96, O : 15,9, S : 23,9, % trouvé C : 35,7, H : 3,3, F : 13,8, N : 6,9, S : 24,0].

### EXEMPLE 15

On opère comme à l'exemple 4 mais à partir de 1 g de 2-imino-9-trifluorométhyl-5,6-dihydro-2H,4H-thiazolo[3,4,5-ef][1,5]benzothiazépine et de 2,2 g d'acide 3-chloroperbenzoïque (pureté 80%) dans 20 ml de dichlorométhane. On obtient ainsi 789 mg de méthanesulfonate de 6,6-dioxyde de 2-imino-9-trifluorométhyl-5,6-dihydro-2H,4H-thiazolo[3,4,5-ef][1,5]benzothiazépine sous forme d'une poudre blanche fondant à une température supérieure à 260°C. [Analyse C12H13F3N2O5S3, % calculé C : 34,45 , H : 3,13, F : 13,62, N : 6,69, S : 22,99, % trouvé C : 34,16, H : 3,17, F : 13,56, N : 6,75, S : 23,23].

### EXEMPLE 16

On opère comme à l'exemple 1 mais en utilisant 6,3 g de brome dans 20 ml d'acide acétique, 9 g de trifluoroacétate de 8-trifluorométhyl-2,3,4,5-tétrahydro-[1,5]benzothiazépine, 8,3 g de thiocyanate de potassium et 90 ml d'acide acétique. Le produit obtenu (6,96 g) est chromatographié sous pression d'azote (150 kPa) sur 90 g de gel de silice 20-45 µm contenus dans une colonne de 3 cm de diamètre, en éluant avec un mélange d'acétate d'éthyle et de cyclohexane (50-50 en volumes). On dissout 4,4 g du produit obtenu (sur 5,7 g obtenus au total) dans 80 ml d'éthanol, auxquels on ajoute 1,5 ml d'acide méthanesulfonique. On obtient ainsi 5 g de méthanesulfonate de 2-imino-9-trifluorométhyl-5,6-dihydro-2H,4H-thiazolo[3,4,5-ef][1,5]benzothiazépine sous forme d'un solide blanc fondant à une température supérieure à 260°C [Analyse C12H13F3N2O3S3, % calculé C : 37,3, H : 3,39, F : 14,75, N : 7,25, O : 12,42, S : 24,89, % trouvé C : 37,4, H : 3,0, F : 14,7, N : 7,3, S : 25,3].

Le trifluoroacétate de 8-trifluorométhyl-2,3,4,5-tétrahydro-[1,5]benzothiazépine peut être préparé de la manière suivante : à une solution de 13 g de 8-trifluorométhyl-2,3,4,5-tétrahydro-[1,5]benzothiazépine-5-carboxylate de *tert*-butyle dans 30 ml de dichlorométhane on ajoute une solution 15 ml d'acide trifluoroacétique. Le mélange est agité 1 heure à une température voisine de 20°C, puis concentré à sec sous pression réduite (2kPa). Le produit obtenu est dissous dans 60 ml d'acétate d'éthyle et la solution est lavée par 100 ml d'eau distillée, séchée sur sulfate de magnésium et concentrée à sec sous pression réduite (2kPa). Le produit obtenu (16,6 g) est chromatographié sous pression d'azote (150 kPa) sur 160 g de gel de silice 20-45 µm contenus dans une colonne de 4 cm de diamètre, en éluant avec un mélange de cyclohexane et d'acétate d'éthyle (75-25 en volumes). Le produit obtenu est mis en suspension dans de l'éther de pétrole et séparé par filtration. On obtient 9 g de trifluoroacétate de 8-trifluorométhyl-2,3,4,5-tétrahydro[1,5]benzothiazépine sous forme d'un solide orangé fondant à 60°C.

La 8-trifluorométhyl-2,3,4,5-tétrahydro-[1,5]benzothiazépine-5-carboxylate de *tert*-butyle peut être préparée de la manière suivante : à une solution, maintenue à -70°C sous argon, de 20 g de 4-trifluorométhyl-phénylcarbamate de *tert*-butyle dans 250 ml de tétrahydrofuranne anhydre, on ajoute, goutte à goutte en 1 heure, 102 ml d'une solution 1,5 M de *tert*-butyllithium dans le pentane. Le mélange est agité pendant 4 heures à -20°C, refroidi vers -60°C, additionné de 2,5 g de soufre, puis agité pendant 45 minutes à -20°C. Le mélange est refroidi vers -60°C, additionné de 15,6 g de 1-chloro-3-iodopropane, puis agité pendant 16 heures à une température voisine de 20°C, chauffé pendant 7 heures au reflux, puis à une température voisine de 20°C pendant 48 heures. Le mélange est hydrolysé avec 200 ml d'eau distillée et extrait deux fois avec 180 ml au total d'acétate d'éthyle. Les extraits organiques réunis sont séchés sur sulfate de magnésium et concentrés à sec sous pression réduite (2kPa). Le produit obtenu est chromatographié sous pression d'azote (150 kPa) sur 450 g de gel de silice 20-45 µm contenus dans une colonne de 6 cm de diamètre, en éluant avec un mélange de cyclohexane et d'acétate d'éthyle (90-10 en volumes). On obtient ainsi 13 g de 8-trifluorométhyl-[1,5]benzothiazépine-5-carboxylate de *tert*-butyle sous forme d'une huile jaune pâle [RMN Spectre ¹H dans DMSO-d6, T=300K, δ en ppm (300 MHz) : 1,50 (9H, s, (CH₃)₃), 1,95 (2H, m, CH₂), 3,05 (2H, t, J=6Hz, SCH₂), 3,70 (2H, t, J=6Hz, CH₂Cl), 7,60 (1H, dd, J=8 et 2 Hz, CH arom.), 7,75 (1H, d, J=2Hz, CH arom.), 7,85 (1H, d, J=8Hz, CH arom.), 8,60 (1H, s, NHCO)].

### EXEMPLE 17

On opère comme à l'exemple 1 mais à partir de 0,660 g de brome dans 5 ml d'acide acétique, 1 g de (R,S)-3-méthyl-7-trifluorométhyl-1,2,3,5-tétrahydro[4,1]benzothiazépine, 0,864 g de thiocyanate de potassium et 15 ml d'acide acétique. Avant alcalinisation à l'ammoniaque, l'insoluble est éliminé par filtration, rincé avec de l'acétate d'éthyle. Le produit obtenu est chromatographié sous pression d'azote (150 kPa) sur 40 g de gel de silice 20-45 µm contenus dans une colonne de 1,5 cm de diamètre, en éluant avec un mélange d'acétate d'éthyle et de cyclohexane (50-50 en volumes). On dissout le produit obtenu (730 mg) dans 10 ml d'éthanol, auxquels on ajoute 0,277 g d'acide méthanesulfonique. Après 16 heures d'agitation à une température voisine de 20°C, la solution est concentrée à sec sous pression réduite (2 kPa). Le produit obtenu est trituré dans de l'acétone, filtré, rincé avec de l'acétone puis de l'éther isopropylique et séché 16 heures à l'air sous hotte ventilée. On obtient ainsi 0,834 g de méthanesulfonate de (R,S)-2-imino-5-méthyl-9-trifluorométhyl-4,5-dihydro-2H,7H-thiazolo[3,4,5-de][4,1]benzothiazépine sous forme d'un solide blanc fondant à une température supérieure à 260°C [Analyse C13H15F3N2O3S3, % calculé C : 38,99, H : 3,78, F : 14,23, N : 7,00, O : 11,99, S : 24,02, % trouvé C : 39,05, H : 3,45, F : 13,94, N : 7,03, S : 24,37].

On dissout 425 mg de méthanesulfonate de (R,S)-2-imino-5-méthyl-9-trifluorométhyl-4,5-dihydro-2H,7H-thiazolo[3,4,5-de][4,1]benzothiazépine dans un mélange de 25 ml d'éthanol, 0,5 ml de triéthylamine, et 200 ml de n-heptane et on injecte sur 700 g de phase stationnaire CHIRALCEL OJ (20 µm) contenus dans une colonne de 60 mm de diamètre et de 400 mm de longueur, en éluant avec un mélange de n-heptane/ isopropanol/ triéthylamine (90/10/0,1 en volumes) avec un débit de 90 ml/mn. On obtient 150 mg de (+)-2-imino-5-méthyl-9-trifluorométhyl-4,5-dihydro-2H,7H-thiazolo[3,4,5-de][4,1]benzothiazépine sous forme d'un solide beige fondant à 102°C [([α]_{D}²⁰= +105,8° ±1,7° c=0,5% méthanol); Analyse C12H11F3N2S2, % calculé C : 47,36, H : 3,64, F : 18,73, N : 9,20, S : 21,07, % trouvé C : 47,59, H : 3,24, F : 18,44, N : 8,99, S : 20,92] et 150 mg de (-)-2-imino-5-méthyl-9-trifluorométhyl-4,5-dihydro-2H,7H-thiazolo[3,4,5-de][4,1]benzothiazépine sous forme d'un solide beige fondant à 102°C [([α]_{D}²⁰= -103,1° ±1,6° c=0,5% méthanol); Analyse C12H11F3N2S2, % calculé C : 47,36, H : 3,64, F : 18,73, N : 9,20, S : 21,07, % trouvé C : 47,64, H : 3,24, F : 18,37, N : 8,97, S : 20,93].

La (R,S)-3-méthyl-7-trifluorométhyl-1,2,3,5-tétrahydro-[4,1]benzothiazépine peut être préparée de la manière suivante : à une solution de 4,5 g de (R,S)-3-méthyl-7-trifluorométhyl-1,2,3,5-tétrahydro-[4,1]benzothiazépin-2-one dans 120 ml de toluène on ajoute goutte à goutte 16,5 ml d'une solution 2 M du complexe borane-diméthylsulfure dans le toluène et le tout est porté 1 heure 30 minutes au reflux. Après retour vers 20°C, le milieu est repris et agité 15 minutes avec une solution saturée de sodium hydrogénocarbonate puis extrait deux fois avec de l'acétate d'éthyle. Les extraits organiques réunis sont séchés sur sulfate de magnésium, filtrés et concentrés à sec sous pression réduite (2 kPa). Le produit est chromatographié sous pression d'azote (150 kPa) sur 55 g de gel de silice 20-45 µm contenus dans une colonne de 2,5 cm de diamètre, en éluant avec un mélange de cyclohexane et d'acétate d'éthyle (80-20 en volumes). On obtient ainsi 2,36 g de (R,S)-3-méthyl-7-trifluorométhyl-1,2,3,5-tétrahydro-[4,1]benzothiazépine sous forme d'une huile incolore qui cristallise sous forme de cristaux blancs fondant à 62°C.

La (R,S)-3-méthyl-7-trifluorométhyl-1,2,3,5-tétrahydro-[4,1]benzothiazépin-2-one peut être préparée de la manière suivante : une solution de 13,6 g de (R,S)-2-(2-amino-5-trifluorométhyl-benzylsulfanyl) propionate de méthyle, de 2 g d'acide toluènesulfonique-4 et de 200 ml de toluène est portée 48 heures au reflux. Après retour à une température voisine de 20°C, on ajoute une solution aqueuse saturée en sodium hydrogénocarbonate et on extrait deux fois par de l'acétate d'éthyle. Les extraits organiques réunis sont séchés sur sulfate de magnésium, filtrés et concentrés à sec sous pression réduite (2 kPa). Le produit obtenu est chromatographié sous pression d'azote (150 kPa) sur 150 g de gel de silice 20-45 µm contenus dans une colonne de 3,5 cm de diamètre, en éluant avec un mélange de cyclohexane et d'acétate d'éthyle (50-50 en volumes) puis d'acétate d'éthyle pur. On obtient ainsi 4,5 g de (R,S)-3-méthyl-7-trifluorométhyl-1,2,3,5-tétrahydro-[4,1]benzothiazépin-2-one sous forme d'un solide beige fondant à 220°C.

Le (R,S)-2-(2-amino-5-trifluorométhyl-benzylsulfanyl) propionate de méthyle peut être préparé de la manière suivante : à une solution de 17,3 g de (R,S)-2-(2-*tert*-butoxycarbonylamino-5-trifluorométhyl-benzylsulfanyl) propionate de méthyle et de 200 ml de dichlorométhane, on ajoute en une fois 20 g d'acide trifluoroacétique et on agite le tout 16 heures à une température voisine de 20°C. Après concentration à sec sous pression réduite (2 kPa) le résidu d'évaporation est repris avec de l'acétate d'éthyle et une solution aqueuse saturée en sodium hydrogénocarbonate. On réextrait une fois la phase alcaline avec de l'acétate d'éthyle et les extraits organiques réunis sont séchés sur sulfate de magnésium, filtrés et concentrés à sec sous pression réduite (2 kPa). On obtient ainsi 13,6 g de (R,S)-2-(2-amino-5-trifluorométhyl-benzylsulfanyl) propionate de méthyle sous forme d'une huile brune qui est utilisée telle quelle.

Le (R,S)-2-(2-*tert*-butoxycarbonylamino-5-trifluorométhyl-benzylsulfanyl) propionate de méthyle peut être préparé comme à l'exemple 10 mais à partir de 15 g de 2-méthyl-4-trifluorométhyl-phénylcarbamate de *tert*-butyle dans 210 ml de tétrahydrofurane anhydre, 91 ml de solution de *tert-*butyllithium 1,5 M dans le pentane, 1,75 g de soufre et 11 g de (R,S)-2-bromo propionate de méthyle. On obtient ainsi 17,3 g de (R,S)-2-(2-*tert*-butoxycarbonylamino-5-trifluorométhyl-benzylsulfanyl) propionate de méthyle sous forme d'une huile jaune limpide qui est utilisée telle quelle.

### EXEMPLE 18

On opère comme à l'exemple 1 mais à partir de 0,76 g de brome dans 6 ml d'acide acétique, 1,3 g de (R,S)-3-carbamoyl-7-trifluorométhyl-1,2,3,5-tétrahydro-[4,1]benzothiazépine, 0,912 g de thiocyanate de potassium et 11 ml d'acide acétique. Avant alcalinisation à l'ammoniaque, l'insoluble est éliminé par filtration, rincé avec de l'acétate d'éthyle. Le produit obtenu est chromatographié sous pression d'azote (150 kPa) sur 30 g de gel de silice 20-45 µm contenus dans une colonne de 2,5 cm de diamètre, en éluant avec un mélange d'acétate d'éthyle et de cyclohexane (90-10 en volumes). Le produit obtenu (560 mg) est recristallisé dans 5 ml d'acétonitrile et on isole ainsi 390 mg de (R,S)-5-carbamoyl-2-imino-9-trifluorométhyl-4,5-dihydro-2H,7H-thiazolo[3,4,5-de][4,1]benzothiazépine sous forme d'un solide blanc fondant vers 114°C [Analyse C12H10F3N3OS2, % calculé C : 43,43, H : 3,02, F : 17,1, N : 12,61, O : 4,80, S : 19,24, % trouvé C : 42,83, H : 2,79, F : 16,68, N : 12,3, S : 19,01].

La (R,S)-3-carbamoyl-7-trifluorométhyl-1,2,3,5-tétrahydro-[4,1]benzothiazépine peut être préparée de la manière suivante : on dissout 3 g de (R,S)-7-trifluorométhyl-1,2,3,5-tétrahydro-[4,1]benzothiazépine-3-carboxylate de méthyle dans 20 ml d'une solution environ 5,6 M d'ammoniac dans le méthanol et on agite à une température voisine de 20°C pendant 16 heures. Le milieu est concentré à sec sous pression réduite et l'extrait sec est chromatographié sous pression d'azote (150 kPa) sur 35 g de gel de silice 20-45 µm contenus dans une colonne de 2 cm de diamètre, en éluant avec un mélange d'acétate d'éthyle et de cyclohexane (50-50 en volumes) puis avec de l'acétate d'éthyle pur. On obtient ainsi 1,3 g (R,S)-3-carbamoyl-7-trifluorométhyl-1,2,3,5-tétrahydro-[4,1]benzothiazépine sous forme d'une huile orange [RMN spectre ¹H dans DMSO-d6, T=300K, δ en ppm (250 Mhz): 3,5 et 3,9 (1H chacun, m, NCH₂), 3,6 et 4,6 (1H chacun, d, J=16Hz, SCH₂), 3,6 (1H, dd, J=4 et 12Hz, SCH), 6,4 (1H, m, NH), 6,8 (1H, d, J=7Hz, CH arom.), 7,1 et 7,5 (1H chacun, s, CONH₂), 7,28 (1H, d, J=7Hz, CH arom.), 7,32 (1H, s, CH arom.)].

Le (R,S)-7-trifluorométhyl-1,2,3,5-tétrahydro-[4,1]benzothiazépine-3-carboxylate de méthyle peut être préparé de la manière suivante : on porte à environs 40°C un mélange de 4,7 g de 7-trifluorométhyl-1,5-dihydro[4,1]benzothiazépine-3-carboxylate de méthyle, de 15,55 g de magnésium en tournures et de 150 ml de méthanol. Lorsque la réaction démarre, le bain est retiré et la réaction entretient d'elle même le reflux. Après la fin du reflux le milieu est ramené à une température voisine de 0°C et on ajoute 270 ml d'acide chlorhydrique 4 M. L'insoluble est éliminé par filtration, rincé avec du dichlorométhane et le filtrat est décanté. L'extrait organique est séché sur du sulfate de magnésium, filtré et concentré à sec sous pression réduite (2 kPa). On obtient ainsi 3,07 g de (R,S)-7-trifluorométhyl-1,2,3,5-tétrahydro[4,1]benzothiazépine-3-carboxylate de méthyle sous forme d'une huile brune [RMN spectre dans le DMSO-d6, T=300K, δ en ppm (250 Mhz): entre 3,6 et 4,1 (7H, m, NCH₂+SCH+ ½ SCH₂+OCH₃), 4,4(1H, d, J=16Hz, ½ SCH₂), 6,5 (1H, m, NH), 6,8 (1H, d, J=7Hz, CH arom.), 7,28 (1H, d, J=7Hz, CH arom.), 7,32 (1H, s, CH arom.)].

Le 7-trifluorométhyl-1,5-dihydro-[4,1]benzothiazépine-3-carboxylate de méthyle peut être préparé de la manière suivante : à une solution de 7,05 g de 3-diméthylamino-2-(2-*tert*-butoxycarbonylamino-5-trifluorométhyl-benzylsulfanyl) acrylate de méthyle et de 75 ml de dichlorométhane on ajoute lentement 9,25 g d'acide trifluoroacétique et le tout est agité 24 heures à une température voisine de 20°C. Le milieu est concentré à sec sous pression réduite (2 kPa) et le résidu est repris par 100 ml d'une solution aqueuse saturée en sodium hydrogénocarbonate et on extrait avec une fois 200 ml d'acétate d'éthyle. L'extrait organique est séché sur du sulfate de magnésium, filtré et concentré à sec sous pression réduite (2 kPa). On obtient ainsi 6,1 g de 7-trifluorométhyl-1,5-dihydro-[4,1]benzothiazépine-3-carboxylate de méthyle sous forme d'un solide orange fondant à 260°C.

Le 3-diméthylamino-2-(2-*tert*-butoxycarbonylamino-5-trifluorométhyl-benzylsulfanyl) acrylate de méthyle peut être préparé de la manière suivante : à une solution de 11,3 g de (2-*tert*-butoxycarbonylamino-5-trifluorométhyl-benzylsulfanyl) acétate de méthyle et de 230 ml de diméthoxy-1,2-éthane anhydre, on coule 15,6 g de *tert*-butyloxy-bis-(diméthylamino) méthane. Le milieu est porté 2 heures au reflux puis agité 48 heures à une température voisine de 20°C. Le milieu est ensuite hydrolysé avec 150 ml d'une solution aqueuse saturée en sodium hydrogénocarbonate et extrait par 200 ml d'acétate d'éthyle. L'extrait organique est séché sur sulfate de magnésium, filtré et concentré à sec sous pression réduite (2 kPa). Le résidu (12,1 g) est chromatographié sous pression d'azote (150 kPa) sur 400 g de gel de silice 20-45 µm contenus dans une colonne de 4,5 cm de diamètre, en éluant avec avec un mélange de cyclohexane et d'acétate d'éthyle (80-20 en volumes). On obtient ainsi 7,05 g de 3-diméthylamino-2-(2-*tert*-butoxycarbonylamino-5-trifluorométhyl-benzylsulfanyl) acrylate de méthyle sous forme d'un solide crème [RMN spectre ¹H dans DMSO-d6, T=300K, d en ppm (250 MHz): 1,50 (9H, s, (CH₃)₃), 2,95 (6H, s, N(CH₃)₂), 3,60 (3H, s, OCH₃), 3,80 (2H, s, SCH₂), 7,30 (1H, d, J=2Hz, CH arom.), 7,50 (1H, dd, J=2 et 7Hz, CH arom.), 7,65 (1H, s, CH ethylénique), 7,90 (1H, d, J=7Hz, CH arom.), 8,90 (1H, s, NH)].

### EXEMPLE 19

On opère comme à l'exemple 1 mais à partir de 0,34 g de brome dans 3 ml d'acide acétique, 0,55 g 3,3-diméthyl-7-trifluorométhyl-1,2-dihydro-5H-[4,1]benzothiazépine, 0,45 g de thiocyanate de potassium et 10 ml d'acide acétique. Le produit obtenu est chromatographié sous pression d'azote (150 kPa) sur 40 g de gel de silice 20-45 µm contenus dans une colonne de 2,5 cm de diamètre, en éluant avec un mélange d'acétate d'éthyle et de cyclohexane (50-50 en volumes). On dissout le produit obtenu (610 mg) dans 10 ml d'éthanol, auxquels on ajoute 0,22 g d'acide méthanesulfonique. Après 16 heures d'agitation à une température voisine de 20°C, la solution est concentrée à sec sous pression réduite (2 kPa). Le produit obtenu est trituré dans un mélange d'acétone et d'éther isopropylique(65-35 en volumes), filtré, rincé avec de l'acétone puis de l'éther isopropylique et séché 16 heures à l'air sous hotte ventilée. On obtient ainsi 0,595 g de méthanesulfonate de 5,5-diméthyl-2-imino-9-trifluorométhyl-2H,4H,7H-thiazolo[3,4,5-de][4,1]benzothiazépine sous forme d'un solide blanc fondant à une température supérieure à 260°C [Analyse C14H17F3N2O3S3, % calculé C : 40,57, H : 4,13, F : 13,75, N : 6,76, O : 11,58, S : 23,21, % trouvé C : 40,23, H : 3,63, F : 13,46, N : 6,65, S : 23,6].

La 3,3-diméthyl-7-trifluorométhyl-1,2-dihydro-5H-[4,1]benzothiazépine peut être préparée de la manière suivante : à une solution de 1,6 g de 3,3-diméthyl-7-trifluorométhyl-1,5-dihydro-[4,1]benzothiazépin-2-one dans 80 ml de toluène anhydre on ajoute goutte à goutte 7,3 ml d'une solution 2 M de complexe borane-diméthylsulfure dans le toluène et le tout est porté 1 heure au reflux. Après retour vers 20°C, le milieu est repris et agité 30 mn avec une solution saturée de sodium hydrogénocarbonate puis extrait deux fois avec de l'acétate d'éthyle. Les extraits organiques réunis sont séchés sur sulfate de magnésium, filtrés et concentrés à sec sous pression réduite (2 kPa). Le produit est chromatographié sous pression d'azote (150 kPa) sur 100 g de gel de silice 20-45 µm contenus dans une colonne de 2,5 cm de diamètre, en éluant avec un mélange de cyclohexane et d'acétate d'éthyle (80-20 en volumes). On obtient ainsi 0,55 g de 3,3-diméthyl-7-trifluorométhyl-1,2-dihydro-5H-[4,1]benzothiazépine sous forme de cristaux blancs fondant à 142°C.

La 3,3-diméthyl-7-trifluorométhyl-1,5-dihydro-[4,1]benzothiazépin-2-one peut être préparée de la manière suivante : on porte au reflux pendant 72 heures une solution de 3,9 g d'acide 2-(2-amino-5-trifluorométhyl-benzylsulfanyl)-2-méthyl propionique et 40 ml de xylène. Le milieu est ensuite concentré à sec sous pression réduite (2 kPa) et le résidu est chromatographié sous pression d'azote (150 kPa) sur 55 g de gel de silice 20-45 µm contenus dans une colonne de 2,5 cm de diamètre, en éluant avec un mélange de cyclohexane et d'acétate d'éthyle (75-25 en volumes). Le produit obtenu est repris et concrété avec de l'éther isopropylique et séparé par filtration puis rincé avec de l'éther isopropylique, séché sous pression réduite. On obtient ainsi 1,47 g de 3,3-diméthyl-7-trifluorométhyl-1,5-dihydro-[4,1]benzothiazépin-2-one sous forme d'un solide crème fondant à 210°C.

L'acide 2-(2-amino-5-trifluorométhyl-benzylsulfanyl)-2-méthyl propionique peut être préparé de la manière suivante : on agite, à une température voisine de 20°C pendant 5 jours, une solution de 2,55 g de 2-(2-amino-5-trifluorométhyl-benzylsulfanyl)-2-méthyl propioniate de méthyle, 0,59 g de potassium hydroxyde en pastilles (pureté 85%) et de 30 ml d'éthanol absolu. Le milieu est ensuite acidifié par 18 ml d'une solution d'isopropanol chlorhydrique environ 5 M et le milieu est concentré à sec sous pression réduite (2 kPa). Le résidu est repris par de l'acétate d'éthyle et l'insoluble est éliminé par filtration, rincé avec de l'acétate d'éthyle. Le filtrat est concentré à sec sous pression réduite (2 kPa) et le résidu obtenu est chromatographié sous pression d'azote (150 kPa) sur 50 g de gel de silice 20-45 µm contenus dans une colonne de 2 cm de diamètre, en éluant avec un mélange d'acétate d'éthyle et de méthanol (95-5 en volumes). On isole un produit qui est repris avec de l'éther de pétrole et le produit est séparé par filtration, rincé avec de l'éther de pétrole et séché. On obtient ainsi 2,2 g d'acide 2-(2-amino-5-trifluorométhyl-benzylsulfanyl)-2-méthyl propionique sous forme d'un solide crème fondant à 113°C.

Le 2-(2-amino-5-trifluorométhyl-benzylsulfanyl)-2-méthyl propioniate de méthyle peut être préparé de la manière suivante : on agite, à une température voisine de 20°C pendant 72 heures, une solution de 9,2 g 2-(2-*tert*-butoxycarbonylamino-5-trifluorométhyl-benzylsulfanyl)-2-méthyl propioniate de méthyle, 10,3 g d'acide trifluoroacétique et de 100 ml de dichlorométhane. Le milieu est concentré à sec sous pression réduite (2 kPa) et le résidu est repris par 250 ml d'une solution aqueuse saturée en sodium hydrogénocarbonate et extrait deux fois avec de l'acétate d'éthyle. Les extraits organiques réunis sont séchés sur sulfate de magnésium, filtrés et concentrés à sec sous pression réduite (2 kPa) pour fournir une huile qui est chromatographiée sous pression d'azote (150 kPa) sur 80 g de gel de silice 20-45 µm contenus dans une colonne de 3 cm de diamètre, en éluant avec un mélange de cyclohexane et d'acétate d'éthyle (75-25 en volumes). On obtient ainsi 5 g de 2-(2-amino-5-trifluorométhyl-benzylsulfanyl)-2-méthyl propioniate de méthyle sous forme d'une huile jaune [RMN spectre ¹H dans DMSO-d6, T=300K, d en ppm (300 Mhz): 1,49 (6H, s, 2CH₃), 3,6 (3H, s, OCH₃), 3,8 (2H, s, SCH₂), 5,6 (2H, s, NH₂), 6,75 (1H, d, J=7Hz, CH arom.), 7,25 (1H, d, J=7Hz, CH arom.), 7,35 (1H, s, CH arom.)].

Le 2-(2-*tert*-butoxycarbonylamino-5-trifluorométhyl-benzylsulfanyl)-2-méthyl propioniate de méthyle peut être préparé comme à l'exemple 10 mais à partir de 15 g de 2-méthyl-4-trifluorométhyl-phénylcarbamate de *tert*-butyle dans 210 ml de tétrahydrofurane anhydre, 91 ml de solution de *tert*-butyllithium 1,5 M dans le pentane, 1,75 g de soufre et 11,9 g de 2-bromo-2-méthyl propionate de méthyle. On obtient ainsi 9,2 g de 2-(2-*tert*-butoxycarbonylamino-5-trifluorométhyl-benzylsulfanyl)-2-méthyl propionate de méthyle sous forme d'une huile incolore [RMN spectre ¹H dans DMSO-d6, T=300K, d en ppm (250 Mhz): 1,56 (6H, s, 2 CH₃), 1,57 (9H, s, (CH₃)₃), 3,6 (3H, s, OCH₃), 4,0 (2H, s, SCH₂), 7,65 (1H, d, J=7Hz, CH arom.), 7,75 (2H, m, 2CH arom.), 8,9 (1H, s, NH)].

### EXEMPLE 20

On prépare la (R,S)-5-hydroxyméthyl-2-imino-9-trifluorométhyl-4,5-dihydro-2H,7H-thiazolo[3,4,5-de][4,1]benzothiazépine de la manière suivante : à une solution de 2,5 g de (R,S)-2-imino-9-trifluorométhyl-4,5-dihydro-2H,7H-thiazolo[3,4,5-de][4,1]benzothiazépine-5-carboxylate de méthyle dans 25 ml d'éthanol absolu, maintenue sous argon, on ajoute 300 mg de sodium tétrahydruroborate et on porte le tout au reflux pendant 5 heures. Le milieu est ensuite agité 16 heures à une température voisine de 20°C, puis hydrolysé avec 50 ml d'eau distillée et extrait par 50 ml d'acétate d'éthyle. L'extrait organique est séché sur sulfate de magnésium, filtré et concentré à sec sous pression réduite (2 kPa). L'huile obtenue est chromatographiée sous pression d'azote (150 kPa) sur 30 g de gel de silice 20-45 µm contenus dans une colonne de 2,5 cm de diamètre, en éluant avec un mélange de cyclohexane et d'acétate d'éthyle (50-50 en volumes). Le solide obtenu est repris avec de l'éther isopropylique, séparé par filtration, séché sous pression réduite (2 kPa). On obtient ainsi 40 mg de (R,S)-5-hydroxyméthyl-2-imino-9-trifluorométhyl-4,5-dihydro-2H,7H-thiazolo[3,4,5-de][4,1]benzothiazépine, sous forme d'un solide écru fondant à 167°C [Analyse C12H11F3N2OS2, % calculé C : 44,99, H : 3,46, F : 17,79, N : 8,74, O : 4,99, S : 20,02, % trouvé C : 44,41, H : 3,01, F : 16,84, N : 8,51, S : 20,4].

### EXEMPLE 21

On opère comme à l'exemple 1 mais à partir de 1,66 g de brome dans 5 ml d'acide acétique, 3 g (R,S)-7-trifluorométhyl-1,2,3,5-tétrahydro-[4,1]benzothiazépine-3-carboxylate de méthyle, 2,2 g de thiocyanate de potassium et 30 ml d'acide acétique. Le produit obtenu est chromatographié sous pression d'azote (150 kPa) sur 80 g de gel de silice 20-45 µm contenus dans une colonne de 3,5 cm de diamètre, en éluant avec un mélange d'acétate d'éthyle et de cyclohexane (50-50 en volumes). On obtient ainsi 2,5 g de (R,S)-2-imino-9-trifluorométhyl-4,5-dihydro-2H,7H-thiazolo[3,4,5-de][4,1]benzothiazépine-5-carboxylate de méthyle sous forme d'une huile.

### EXEMPLE 22

On opère comme à l'exemple 1 mais à partir de 1,47 g de brome dans 5 ml d'acide acétique, 2 g de 7-trifluorométhyl-1,2,3,5-tétrahydro-[4,1]benzoxazépine, 3 g de thiocyanate de potassium et 50 ml d'acide acétique. Après chromatographie sur gel de silice en éluant avec un mélange d'acétate d'éthyle et de cyclohexane (50-50 en volumes) et traitement avec de l'acide méthanesulfonique, on obtient 1,79 g de méthanesulfonate de 2-imino-9-trifluorométhyl-4,5-dihydro-2H,7H-thiazolo[3,4,5-de][4,1]benzoxazépine sous forme d'un solide blanchâtre fondant vers 258°C [Analyse C12H13F3N2O4S2, % calculé C : 38,92, H : 3,54, F : 15,39, N : 7,56, O : 17,28, S : 17,31, % trouvé C : 38,67, H : 3,31, F : 15,02, N : 7,69, S : 17.47].

La 7-trifluorométhyl-1,2,3,5-tétrahydro-[4,1]benzoxazépine peut être préparée de la manière suivante : à une suspension de 2,22 g de 7-trifluorométhyl-1,5-dihydro-3H-[4,1]benzoxazépin-2-one dans 100 ml de toluène, on ajoute goutte à goutte, sous argon et à une température voisine de 20°C, 15 ml d'une solution 2 N dans le tétrahydrofuranne du complexe borane-méthylsulfure. Le milieu réactionnel est ensuite porté et maintenu à l'ébullition pendant 1 heure 45 minutes. Après refroidissement vers 20°C, il est hydrolysé avec 100 ml d'une solution saturée d'hydrogénocarbonate de sodium, puis extrait par 2 fois 100 ml d'acétate d'éthyle. Les phases organiques réunies sont séchées sur sulfate de magnésium, puis concentrées au rotavapor. Après trituration du résidu dans l'éther de pétrole on obtient 2 g de 7-trifluorométhyl-1,2,3,5-tétrahydro-[4,1]benzoxazépine sous forme d'un solide jaunâtre fondant vers 85°C.

La 7-trifluorométhyl-1,5-dihydro-3H-[4,1]benzoxazépin-2-one peut être préparée de la manière suivante : à une solution de 2,95 g de 2-chloro-N-(2-hydroxyméthyl-4-trifluorométhylphényl)acétamide dans 330 ml de tétrahydrofurane, on ajoute, sous argon et à une température voisine de 5°C, 2,9 g de *t*-butylate de potassium. Après 1 heure d'agitation à cette température, le milieu réactionnel est hydrolysé par 30 ml d'une solution saturée de chlorure d'ammonium, puis extrait par 150 ml d'acétate d'éthyle. La phase organique est séchée sur sulfate de magnésium, puis concentrée au rotavapor. Après trituration du résidu dans un mélange d'éther éthylique et d'éther de pétrole on obtient 2,2 g de 7-trifluorométhyl-1,5-dihydro-3H-[4,1]benzoxazépin-2-one sous forme d'un solide blanc fondant vers 183°C.

Le 2-chloro-N-(2-hydroxyméthyl-4-trifluorométhylphényl)acétamide peut être préparé de la manière suivante : à une solution de 5,54 g de 2-amino-5-trifluorométhylphénylméthanol dans 100 ml de dichlorométhane et 6 ml de triéthylamine, on ajoute, sous argon et à une température voisine de 5°C, 2,1 ml de chlorure de chloracétyle en solution dans 20 ml de dichlorométhane. Le milieu réactionnel est agité 3 heures vers 5°C, puis 18 heures à température ambiante. Il est ensuite versé sur 100 ml d'une solution saturée d'hydrogénocarbonate de sodium, puis extrait par 200 ml d'éther éthylique. La phase organique est séchée sur sulfate de magnésium, puis concentrée au rotavapor. Après chromatographie sur gel de silice en éluant avec un mélange de cyclohexane et d'acétate d'éthyle (65-35 en volumes) et trituration du résidu dans un mélange d'éther éthylique et d'éther de pétrole, on obtient 4 g de 2-chloro-N-(2-hydroxyméthyl-4-trifluorométhylphényl)acétamide sous forme d'un solide blanchâtre fondant vers 92°C.

Le 2-amino-5-trifluorométhylphénylméthanol peut être préparé de la manière suivante : à une solution de 7,3 g d'acide 2-amino-5-trifluorométhylbenzoïque dans 250 ml de tétrahydrofurane, on ajoute, sous argon et à une température voisine de 5°C, 7,3 g d'hydruroborate de sodium, puis 24 ml de chlorotriméthylsilane. Après 44 heures d'agitation à température ambiante, le milieu réactionnel est refroidi vers 5°C, puis hydrolysé par 100 ml d'eau distillée et extrait 2 fois par de l'éther éthylique (400 puis 150 ml). Les phases organiques réunies sont lavées par 100 ml d'une solution 1 N d'hydroxyde de sodium, puis séchées sur sulfate de magnésium et concentrées au rotavapor. On obtient ainsi 7,9 g de 2-amino-5-trifluorométhylphénylméthanol sous forme d'un solide blanc fondant à 70°C.

L'acide 2-amino-5-trifluorométhylbenzoïque peut être préparé selon la méthode décrite par M. L. Carmellino et coll., Eur. J. Med. Chem. Chim. Ther., 29 (10), 743 (1994).

Les médicaments selon l'invention sont constitués par un composé de formule (I) ou un sel d'un tel composé, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles pour le traitement et/ou la prévention En thérapeutique humaine, les composés selon l'invention sont particulièrement utiles pour le traitement et/ou la prévention des convulsions et des maladies liées à la transmission glutamatergique. Ils sont notamment utiles pour traiter et/ou prévenir toutes les ischémies (telles l'ischémie focale ou globale) consécutives à des accidents vasculaires cérébraux tels que le stroke thromboembolique et hémorragique, un arrêt cardiaque, une hypotension artérielle, une intervention chirurgicale cardiaque, vasculaire ou pulmonaire ou une hypoglycémie sévére, dans le traitement des effets dus à une anoxie, qu'elle soit périnatale ou consécutive à une noyade, une haute pression ou à des lésions cérébro-spinales, pour traiter ou prévenir l'évolution de maladies neurodégénératives, de la chorée d'HUNTINGTON, de la maladie d'ALZHEIMER et autres démences, de la sclérose latérale amyotrophique ou d'autres maladies du motoneurone, de l'atrophie olivo-pontocérébelleuse et de la maladie de PARKINSON, vis-à-vis des manifestations épileptogènes (épilepsie) et/ou convulsives, pour le traitement des traumatismes cérébraux ou spinaux, des traumatismes liés à la dégénérescence de l'oreille interneou de la rétine, du tinnitus, de l'anxiété, de la dépression, de la schizophrénie, du syndrome de TOURETTE, des encéphalopathies hépatiques, des troubles du sommeil, des désordres du déficit attentionnel, des troubles des conditions hormonales (excès de la sécrétion de HG ou HL, sécrétion de corticostérone), en tant qu'analgésiques, antiinflammatoires, antianorexiques, antimigraineux, antiémétiques et pour traiter les empoisonnements par des neurotoxines ainsi que les troubles neurologiques associés aux maladies virales telles que les méningites et encéphalites virales, le SIDA, la rage, la rougeole et le tétanos. Ces composés sont aussi utiles pour la prévention, la tolérance et la dépendance des symptômes d'abstinence aux drogues, à l'alcool et de l'inhibition de l'accoutumance et de la dépendance aux opiacés, barbituriques, amphétamine et benzodiazépines. Ils peuvent également être utilisés dans le traitement des déficits liés à des anomalies mitochondriales telles que la myopathie mitochondriale, le syndrome de LEBER, l'encéphalopathie de WERNICKE, le syndrome de RETT, l'homocystéinémie, l'hyperprolinémie, l'hydroxybutirique-aminoacidurie, l'encéphalopathie saturnine (intoxication chronique au plomb) et la déficience en sulfite oxydase.

Les doses dépendent de l'effet recherché, de la durée du traitement et de la voie d'administration utilisée; elles sont généralement comprises entre 10 mg et 100 mg par jour par voie orale pour un adulte avec des doses unitaires allant de 5 mg à 50 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention:

### EXEMPLE A

On prépare, selon la technique habituelle, des gélules dosées à 50 mg de produit actif ayant la composition suivante:
- Composé de formule (I) 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Composé de formule (I) 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- Composé de formule (I) 10 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau q.s.p. 4 ml

## Revendications

1. Composés de formule : 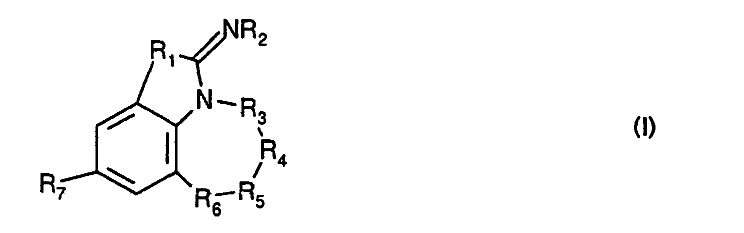 dans laquelle
R₁ représente un atome de soufre ou de sélénium,
R₂ représente un atome d'hydrogène ou un radical alkyle,
-R₃-R₄-R₅-R₆- représente une chaîne de formule -CH₂-CH₂-CH₂-CH₂-, -CH₂-CH₂-CH₂-CO-, -CH₂-CH₂-CH₂-CH(R₈)-, -CH₂-CH₂-CH₂-Se-, -CH₂-CH₂-Se-CH₂-, -CH₂-CH₂-CH₂-S-, -CH₂-CH₂-CH₂-SO-, -CH₂-CH₂-CH₂-SO₂-, -CH₂-CH₂-CH₂-O-, -CH₂-CH₂-CH₂-N(R₉)-, -CH₂-CH₂-CO-CH₂-, -CH₂-CH₂-CH(R₈)-CH₂-, -CH₂-CH₂-S-CH₂-, -CH₂-CH₂-SO-CH₂-, -CH₂-CH₂-SO₂-CH₂-, -CH₂-C(alk)(alk')-S-CH₂-, -CH₂-C(alk)(alk')-SO-CH₂-, -CH₂-C(alk)(alk')-SO₂-CH₂, -CH₂-CH(R₁₀)-S-CH₂-, -CH₂-CH(R₁₀)-SO-CH₂-, -CH₂-CH(R₁₀)-SO₂-CH₂-, -CH₂-CH₂-O-CH₂-, -CH₂-CH₂-N(R₉)-CH₂- ou -CH₂-CO-N(R₉)-CH₂-,
R₇ représente un radical polyfluoroalkyle ou polyfluoroalcoxy,
R₈ représente un radical hydroxy,
R₉ représente un atome d'hydrogène ou un radical alkyle ou benzyle,
R₁₀ représente un radical alkyle, -CH₂OH, -COOalk, -COOH ou -CONH₂,
alk représente un radical alkyle,
alk' représente un radical alkyle,
dans ces définitions, les radicaux et portions alkyle contiennent 1 à 6 atomes de carbone en chaîne droite ou ramifiée,
et lorsqu'ils comportent un ou plusieurs centres asymétriques, leurs isomères, racémiques et énantiomères et leurs sels avec un acide minéral ou organique.

2. Composés de formule (I) selon la revendication 1 pour lesquels R₇ représente un radical trifluorométhoxy ou trifluorométhyle, et lorsqu'ils comportent un ou plusieurs centres asymétriques, leurs isomères, racémiques, énantiomères et leurs sels avec un acide minéral ou organique.

3. Composés de formule (I) selon la revendication 1 pour lesquels R₁ représente un atome de soufre, R₂ représente un atome d'hydrogène, -R₃-R₄-R₅-R₆- représente une chaîne de formule -CH₂-CH₂-CH₂-CH₂-, -CH₂-CH₂-CH₂-CO-, -CH₂-CH₂-CH₂-CH(R₈)-, -CH₂-CH₂-CH₂-Se-, -CH₂-CH₂-Se-CH₂-, -CH₂-CH₂-CH₂-S-, -CH₂-CH₂-CH₂-SO-, -CH₂-CH₂-CH₂-SO₂-, -CH₂-CH₂-CH₂-O-, -CH₂-CH₂-CH₂-N(R₉)-, -CH₂-CH₂-CO-CH₂-, -CH₂-CH₂-CH(R₈)-CH₂-, -CH₂-CH₂-S-CH₂-, -CH₂-CH₂-SO-CH₂-, -CH₂-CH₂-SO₂-CH₂-, -CH₂-C(alk)(alk')-S-CH₂, -CH₂-C(alk)(alk')-SO-CH₂, -CH₂-C(alk)(alk')-SO₂-CH₂, -CH₂-CH(R₁₀)-S-CH₂-, -CH₂-CH(R₁₀)-SO-CH₂-, -CH₂-CH(R₁₀)-SO₂-CH₂-, -CH₂-CH₂-O-CH₂-, -CH₂-CH₂-N(R₉)-CH₂- ou -CH₂-CO-N(R₉)-CH₂-, R₇ représente un radical trifluorométhyle ou trifluorométhoxy, R₈ représente un radical hydroxy, R₉ représente un atome d'hydrogène ou un radical alkyle ou benzyle, R₁₀ représente un radical alkyle, -CH₂OH, -COOalk, -COOH ou -CONH₂, alk représente un radical alkyle et alk' représente un radical alkyle, et lorsqu'ils comportent un ou plusieurs centres asymétriques, leurs isomères, racémiques, énantiomères et leurs sels avec un acide minéral ou organique.

4. Composés de formule (I) selon la revendication 1 choisis parmi les composés suivants :
- 2-imino-9-trifluorométhoxy-4,5,6,7-tétrahydro-2H-thiazolo[5,4,3-jk][1]benzazépine-7-ol,
- 2-imino-9-trifluorométhoxy-4,5,6-7-tétrahydro-2H-thiazolo[5,4,3-jk][1]benzazépine,
- 2-imino-9-trifluorométhyl-4,5,6,7-tétrahydro-2H-thiazolo[5,4,3-jk][1]benzazépine,
- 7,7-dioxyde de 2-imino-9-trifluorométhoxy-5,6-dihydro-2H,4H-thiazolo[3,4,5-ef][1,5]benzothiazépine,
- 7-oxyde de 2-imino-9-trifluorométhoxy-5,6-dihydro-2H,4H-thiazolo-[3,4,5-ef][1,5]benzothiazépine,
- 2-imino-9-trifluorométhoxy-5,6-dihydro-2H,4H-thiazolo[3,4,5-ef][1,5]benzothiazépine,
- 6-benzyl-2-imino-9-trifluorométhoxy-6,7-dihydro-4H-thiazolo[3,4,5-kj][1,4] benzodiazépine-5-one,
- 6-benzyl-2-imino-9-trifluorométhoxy-4,5,6,7-tétrahydro-2H-thiazolo[3,4,5-kj][1,4]benzodiazépine,
- 2-imino-9-trifluorométhoxy-4,5-dihydro-2H,7H-thiazolo[3,4,5-de][4,1]benzothiazépine,
- 2-imino-9-trifluorométhyl-4,5-dihydro-2H,7H-thiazolo[3,4,5-de][4.1]benzothiazépine,
- 6,6-dioxyde de 2-imino-9-trifluorométhyl-4,5-dihydro-2H,7H-thiazolo[3,4,5-de][4,1]benzothiazépine,
- 7-oxyde de 2-imino-9-trifluorométhyl-5,6-dihydro-2H,4H-thiazolo[3,4,5-ef][1,5]benzothiazépine,
- 6,6-dioxyde de 2-imino-9-trifluorométhyl-5,6-dihydro-2H,4H-thiazolo[3,4,5-efl[1,5]benzothiazépine,
- 2-imino-9-trifluorométhyl-5,6-dihydro-2H,4H-thiazolo[3,4,5-ef][1,5]benzothiazépine,
- 2-imino-9-trifluorométhyl-4,5,6,7-tétrahydro-2H-thiazolo[5,4,3-jk][1]benzazépine-7-ol,- 6,6-dioxyde de 2-imino-9-trifluorométhoxy-4,5-dihydro-2H,7H-thiazolo[3,4,5-de][4,1]benzothiazépine,
- 6-oxyde de 2-imino-9-trifluorométhoxy-4,5-dihydro-2H,7H-thiazolo[3,4,5-de][4,1]benzothiazépine,
- 6-benzyl-2-imino-9-trifluorométhyl-6,7-dihydro-4H-thiazolo[3,4,5-kj][1,4] benzodiazépine-5-one,
- 6-benzyl-2-imino-9-trifluorométhyl-4,5,6,7-tétrahydro-2H-thiazolo[3,4,5-kj][1,4]benzodiazépine,
- 2-imino-5-méthyl-9-trifluorométhyl-4,5-dihydro-2H,7H-thiazolo[3,4,5-de][4,1] benzothiazépine,
- 5-carbamoyl-2-imino-9-trifluorométhyl-4,5-dihydro-2H,7H-thiazolo[3,4,5-de] [4,1]benzothiazépine,
- 5,5-diméthyl-2-imino-9-trifluorométhyl-2H,4H,7H-thiazolo[3,4,5-de][4,1]benzothiazépine,
- 5-hydroxyméthyl-2-imino-9-trifluorométhyl-4,5-dihydro-2H,7H-thiazolo[3,4,5-de][4,1]benzothiazépine,
et lorsqu'ils comportent un ou plusieurs centres asymétriques, leurs isomères, racémiques, énantiomères et leurs sels avec un acide minéral ou organique.

5. Composés de formule (I) selon la revendication 1 choisis parmi les composés suivants :
- (R,S)-6-oxyde de 2-imino-9-trifluorométhyl-4,5-dihydro-2H,7H-thiazolo[3,4,5-de][4,1]benzothiazépine,
- (+)-6-oxyde de 2-imino-9-trifluorométhyl-4,5-dihydro-2H,7H-thiazolo[3,4,5-de][4,1]benzothiazépine,
- (-)-6-oxyde de 2-imino-9-trifluorométhyl-4,5-dihydro-2H,7H-thiazolo[3,4,5-de][4,1]benzothiazépine,
- (R,S)-2-imino-5-méthyl-9-trifluorométhyl-4,5-dihydro-2H,7H-thiazolo[3,4,5-de][4,1]benzothiazépine,
- (+)-2-imino-5-méthyl-9-trifluorométhyl-4,5-dihydro-2H,7H-thiazolo[3,4,5-de][4,1]benzothiazépine,
- (-)-2-imino-5-méthyl-9-trifluorométhyl-4,5-dihydro-2H,7H-thiazolo[3,4,5-de][4,1]benzothiazépine
et leurs sels avec un acide minéral ou organique.

6. Procédé de préparation des composés de formule (I) selon la revendication 1 pour lesquels R₁ représente un atome de soufre ou de sélénium, R₂ représente un atome d'hydrogène, -R₃-R₄-R₅-R₆- représente une chaîne de formule -CH₂-CH₂-CH₂-CH₂-, -CH₂-CH₂-CH₂-CO-, -CH₂-CH₂-CH₂-CH(R₈)-, -CH₂-CH₂-CH₂-Se-, -CH₂-CH₂-Se-CH₂-, -CH₂-CH₂-CH₂-S-, -CH₂-CH₂-CH₂-O-, -CH₂-CH₂-CH₂-N(R₉)-, -CH₂-CH₂-CO-CH₂-, -CH₂-CH₂-CH(R₈)-CH₂-, -CH₂-CH₂-S-CH₂-, -CH₂-C(alk)(alk')-S-CH₂, -CH₂-CH(R₁₀)-S-CH₂-, -CH₂-CH₂-O-CH₂-, -CH₂-CH₂-N(R₉)-CH₂- ou -CH₂-CO-N(R₉)-CH₂-, R₈ représente un radical hydroxy, R₉ représente un atome d'hydrogène ou un radical alkyle ou benzyle, R₁₀ représente un radical alkyle, -COOalk ou -CONH₂, alk représente un radical alkyle, alk' représente un radical alkyle, **caractérisé en ce que** l'on fait réagir un thiocyanate de métal alcalin ou un sélénocyanate de métal alcalin sur un dérivé de formule : 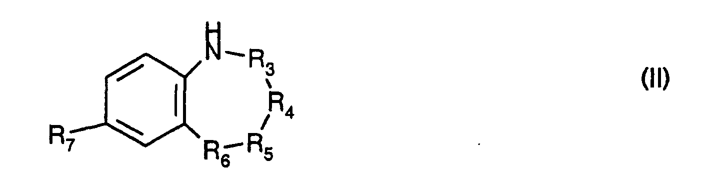 dans laquelle R₇ a les mêmes significations que dans la revendication 1 et -R₃-R₄-R₅-R₆- représente une chaîne de formule -CH₂-CH₂-CH₂-CH₂-, -CH₂-CH₂-CH₂-CO-, -CH₂-CH₂-CH₂-CH(R₈)-, -CH₂-CH₂-CH₂-Se-, -CH₂-CH₂-Se-CH₂-, -CH₂-CH₂-CH₂-S-, -CH₂-CH₂-CH₂-O-, -CH₂-CH₂-CH₂-N(R₉)-, -CH₂-CH₂-CO-CH₂-, -CH₂-CH₂-CH(R₈)-CH₂-, -CH₂-CH₂-S-CH₂-, -CH₂-C(alk)(alk')-S-CH₂, -CH₂-CH(R₁₀)-S-CH₂-, -CH₂-CH₂-O-CH₂-, -CH₂-CH₂-N(R₉)-CH₂- ou -CH₂-CO-N(R₉)-CH₂-, R₈ représente un radical hydroxy, R₉ représente un atome d'hydrogène ou un radical alkyle ou benzyle, R₁₀ représente un radical alkyle, -COOalk ou -CONH₂, alk représente un radical alkyle, alk' représente un radical alkyle, isole le produit et le transforme éventuellement en sel.

7. Procédé de préparation des composés de formule (I) selon la revendication 1 pour lesquels R₂ représente un radical alkyle **caractérisé en ce que** l'on alkyle un composé de formule (I) correspondant pour lequel R₂ représente un atome d'hydrogène, isole le produit et le transforme éventuellement en sel.

8. Procédé de préparation des composés de formule (I) selon la revendication 1 pour lesquels R₂ représente un atome d'hydrogène ou un radical alkyle, -R₃-R₄-R₅-R₆- représente une chaîne de formule -CH₂-CH₂-CH(R₈)-CH₂- ou -CH₂-CH₂-CH₂-CH(R₈)- et R₈ représente un radical hydroxy **caractérisé en ce que** l'on réduit un composé de formule (I) correspondant pour lequel R₂ représente un atome d'hydrogène ou un radical alkyle et -R₃-R₄-R₅-R₆-représente une chaîne de formule -CH₂-CH₂-CO-CH₂- ou -CH₂-CH₂-CH₂-CO-, isole le produit et le transforme éventuellement en sel.

9. Procédé de préparation des composés de formule (I) selon la revendication 1 pour lesquels R₂ représente un atome d'hydrogène ou un radical alkyle et -R₃-R₄-R₅-R₆- représente une chaîne de formule -CH₂-CH₂-CH₂-SO-, -CH₂-CH₂-CH₂-SO₂-) -CH₂-CH₂-SO-CH₂-, -CH₂-CH₂-SO₂-CH₂-, -CH₂-C(alk)(alk')-SO-CH₂-, -CH₂-C(alk)(alk')-SO₂-CH₂, -CH₂-CH(R₁₀)-SO-CH₂- ou -CH₂-CH(R₁₀)-SO₂-CH₂- **caractérisé en ce que** l'on oxyde un composé de formule (I) correspondant pour lequel la chaîne -R₃-R₄-R₅-R₆- représente une chaîne de formule -CH₂-CH₂₋CH₂-S-, -CH₂-CH₂-S-CH₂-, -CH₂-C(alk)(alk')-S-CH₂ ou -CH₂-CH(R₁₀)-S-CH₂-, isole le produit et le transforme éventuellement en sel.

10. Procédé de préparation des composés de formule (I) selon la revendication 1 pour lesquels R₂ représente un atome d'hydrogène ou un radical alkyle, -R₃-R₄-R₅-R₆- représente une chaîne de formule -CH₂-CH(R₁₀)-S-CH₂ dans laquelle R₁₀ représente un radical -COOH **caractérisé en ce que** l'on hydrolyse un composé de formule (I) correspondant pour lesquels R₂ représente un atome d'hydrogène ou un radical alkyle, -R₃-R₄-R₅-R₆- représente une chaîne de formule -CH₂-CH(R₁₀)-S-CH₂ dans laquelle R₁₀ représente un radical -COOalk, isole le produit et le transforme éventuellement en sel.

11. Médicaments contenant en tant que principe actif au moins un composé selon l'une des revendications 1 à 5 ou un sel pharmaceutiquement acceptable d'un tel composé avec un acide minéral ou organique.

12. Dérivés de formule : 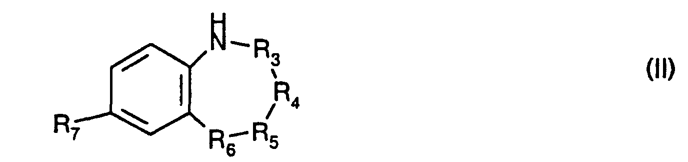 dans laquelle -R₃-R₄-R₅-R₆- représente une chaîne de formule -CH₂-CH₂-CH₂-CH₂-, -CH₂-CH₂-CH₂-CO-, -CH₂-CH₂-CH₂-CH(R₈)-, -CH₂-CH₂-CH₂-Se-, -CH₂-CH₂-Se-CH₂-, -CH₂-CH₂-CH₂-S-, -CH₂-CH₂-CH₂-O-, -CH₂-CH₂-CH₂-N(R₉)-, -CH₂-CH₂-CO-CH₂-, -CH₂-CH₂-CH(R₈)-CH₂-, -CH₂-CH₂-S-CH₂-, -CH₂-C(alk)(alk')-S-CH₂, -CH₂-CH(R₁₀)-S-CH₂-, -CH₂-CH₂-O-CH₂-, -CH₂-CH₂-N(R₉)-CH₂- ou -CH₂-CO-N(R₉)-CH₂-, R₇ représente un radical polyfluoroalkyle ou polyfluoroalcoxy, R₈ représente un radical hydroxy, R₉ représente un atome d'hydrogène ou un radical alkyle ou benzyle, R₁₀ représente un radical alkyle, -COOalk ou -CONH₂, alk représente un radical alkyle et alk' représente un radical alkyle, dans ces définitions, les radicaux et portions alkyle contiennent 1 à 6 atomes de carbone en chaîne droite ou ramifiée.

13. Les compositions pharmaceutiques renfermant un médicament tel que défini à la revendication 6 et un excipient pharmaceutiquement compatible.

## Claims

1. Compounds of formula: 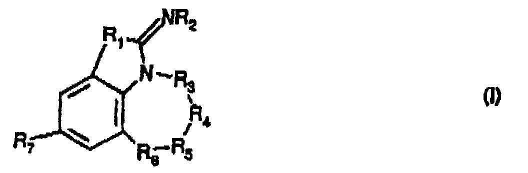 in which
R₁ represents a sulphur or selenium atom,
R₂ represents a hydrogen atom or an alkyl radical,
-R₃-R₄-R₅-R₆- represents a chain of formula -CH₂-CH₂-CH₂-CH₂-, -CH₂-CH₂-CH₂-CO-, -CH₂-CH₂-CH₂-CH(R₈)-, -CH₂-CH₂-CH₂-Se-, -CH₂-CH₂-Se-CH₂-, -CH₂-CH₂-CH₂-S-, -CH₂-CH₂-CH₂-SO-, -CH₂-CH₂-CH₂-SO₂-, -CH₂-CH₂-CH₂-O-, -CH₂-CH₂-CH₂-N(R₉)-, -CH₂-CH₂-CO-CH₂-, -CH₂-CH₂-CH(R₈)-CH₂-, -CH₂-CH₂-S-CH₂-, -CH₂-CH₂-SO-CH₂-, -CH₂-CH₂-SO₂-CH₂-, -CH₂-C(alk)(alk')-S-CH₂-, -CH₂-C(alk)(alk')-SO-CH₂-, -CH₂-C(alk)(alk')-SO₂-CH₂-, -CH₂-CH(R₁₀)-S-CH₂-, -CH₂-CH(R₁₀)-SO-CH₂-, -CH₂-CH(R₁₀)-SO₂-CH₂-, -CH₂-CH₂-O-CH₂-, -CH₂-CH₂-N(R₉)-CH₂- or -CH₂-CO-N(R₉)-CH₂-,
R₇ represents a polyfluoroalkyl or polyfluoroalkoxy radical,
R₈ represents a hydroxyl radical,
R₉ represents a hydrogen atom or an alkyl or benzyl radical,
R₁₀ represents an alkyl, -CH₂OH, -COOalk, -COOH or -CONH₂ radical,
alk represents an alkyl radical,
alk' represents an alkyl radical,
in these definitions, the alkyl radicals and portions contain 1 to 6 straight- or branched-chain carbon atoms,
and, when they include one or more asymmetric centres, their isomers, racemates and enantiomers and their salts with an inorganic or organic acid.

2. Compounds of formula (I) according to Claim 1 for which R₇ represents a trifluoromethoxy or trifluoromethyl radical, and, when they include one or more asymmetric centres, their isomers, racemates, enantiomers and their salts with an inorganic or organic acid.

3. Compounds of formula (I) according to Claim 1, for which R₁ represents a sulphur atom, R₂ represents a hydrogen atom, -R₃-R₄-R₅-R₆- represents a chain of formula -CH₂-CH₂-CH₂-CH₂-, -CH₂-CH₂-CH₂-CO-, -CH₂-CH₂-CH₂-CH(R₈)-, -CH₂-CH₂-CH₂-Se-, -CH₂-CH₂-Se-CH₂-, -CH₂-CH₂-CH₂-S-, -CH₂-CH₂-CH₂-SO-, -CH₂-CH₂-CH₂-SO₂-, -CH₂-CH₂-CH₂-O-, -CH₂-CH₂-CH₂-N(R₉)-, -CH₂-CH₂-CO-CH₂-, -CH₂-CH₂-CH(R₈)-CH₂-, -CH₂-CH₂-S-CH₂-, -CH₂-CH₂-SO-CH₂-, -CH₂-CH₂-SO₂-CH₂-, -CH₂-C (alk) (alk')-S-CH₂-, -CH₂-C(alk) (alk')-SO-CH₂-, -CH₂-C(alk) (alk')-SO₂-CH₂-, -CH₂-CH(R₁₀)-S-CH₂-, -CH₂-CH(R₁₀)-SO-CH₂-, -CH₂-CH(R₁₀)-SO₂-CH₂-, -CH₂-CH₂-O-CH₂-, -CH₂-CH₂-N(R₉)-CH₂- or -CH₂-CO-N(R₉)-CH₂-, R₇ represents a trifluoromethyl or trifluoromethoxy radical, R₈ represents a hydroxyl radical, R₉ represents a hydrogen atom or an alkyl or benzyl radical, R₁₀ represents an alkyl, -CH₂OH, -COOalk, -COOH or -CONH₂ radical, alk represents an alkyl radical and alk' represents an alkyl radical, and, when they include one or more asymmetric centres, their isomers, racemates, enantiomers and their salts with an inorganic or organic acid.

4. Compounds of formula (I) according to Claim 1, chosen from the following compounds:
- 2-imino-9-trifluoromethoxy-4,5,6,7-tetrahydro-2H-thiazolo[5,4,3-jk][1]benzazepin-7-ol,
- 2-imino-9-trifluoromethoxy-4,5,6,7-tetrahydro-2H-thiazolo[5,4,3-jk][1]benzazepine,
- 2-imino-9-trifluoromethyl-4,5,6,7-tetrahydro-2H-thiazolo[5,4,3-jk][1]benzazepine,
- 2-imino-9-trifluoromethoxy-5,6-dihydro-2H,4H-thiazolo[3,4,5-ef][1,5]benzothiazepine 7,7-dioxide,
- 2-imino-9-trifluoromethoxy-5,6-dihydro-2H,4H-thiazolo[3,4,5-ef][1,5]benzothiazepine 7-oxide,
- 2-imino-9-trifluoromethoxy-5,6-dihydro-2H,4H-thiazolo[3,4,5-ef][1,5]benzothiazepine,
- 6-benzyl-2-imino-9-trifluoromethoxy-6,7-dihydro-4H-thiazolo[3,4,5-kj] [1,4]benzodiazepin-5-one,
- 6-benzyl-2-imino-9-trifluoromethoxy-4,5,6,7-tetrahydro-2H-thiazolo[3,4,5-kj] [1,4]benzodiazepine,
- 2-imino-9-trifluoromethoxy-4,5-dihydro-2H,7H-thiazolo[3,4,5-de][4,1]benzothiazepine,
- 2-imino-9-trifluoromethyl-4,5-dihydro-2H,7H-thiazolo[3,4,5-de] [4,1]benzothiazepine,
- 2-imino-9-trifluoromethyl-4,5-dihydro-2H,7H-thiazolo[3,4,5-de][4,1]benzothiazepine 6,6-dioxide,
- 2-imino-9-trifluoromethyl-5,6-dihydro-2H,4H-thiazolo[3,4,5-ef] [1,5]benzothiazepine 7-oxide,
- 2-imino-9-trifluoromethyl-5,6-dihydro-2H,4H-thiazolo[3,4,5-ef][1,5]benzothiazepine 6,6-dioxide,
- 2-imino-9-trifluoromethyl-5,6-dihydro-2H,4H-thiazolo[3,4,5-ef][1,5]benzothiazepine,
- 2-imino-9-trifluoromethyl-4,5,6,7-tetrahydro-2H-thiazolo[5,4,3-jk] [1]benzazepin-7-ol,
- 2-imino-9-trifluoromethoxy-4,5-dihydro-2H,7H-thiazolo[3,4,5-de] [4,1]benzothiazepine 6,6-dioxide,
- 2-imino-9-trifluoromethoxy-4,5-dihydro-2H,7H-thiazolo[3,4,5-de] [4,1]benzothiazepine 6-oxide,
- 6-benzyl-2-imino-9-trifluoromethyl-6,7-dihydro-4H-thiazolo[3,4,5-kj] [1,4]benzodiazepin-5-one,
- 6-benzyl-2-imino-9-trifluoromethyl-4,5,6,7-tetrahydro-2H-thiazolo[3,4,5-kj] [1,4]benzodiazepine,
- 2-imino-5-methyl-9-trifluoromethyl-4,5-dihydro-2H,7H-thiazolo[3,4,5-de] [4,1]benzothiazepine,
- 5-carbamoyl-2-imino-9-trifluoromethyl-4,5-dihydro-2H,7H-thiazolo[3,4,5-de] [4,1]benzothiazepine,
- 5,5-dimethyl-2-imino-9-trifluoromethyl-2H,4H,7H-thiazolo[3,4,5-de][4,1]benzothiazepine,
- 5-hydroxymethyl-2-imino-9-trifluoromethyl-4,5-dihydro-2H,7H-thiazolo[3,4,5-de][4,1]benzothiazepine,
and, when they include one or more asymmetric centres, their isomers, racemates, enantiomers and their salts with an inorganic or organic acid.

5. Compounds of formula (I) according to Claim 1, chosen from the following compounds:
- (R,S)-2-imino-9-trifluoromethyl-4,5-dihydro-2H,7H-thiazolo[3,4,5-de] [4,1]benzothiazepine 6-oxide,
- (+)-2-imino-9-trifluoromethyl-4,5-dihydro-2H,7H-thiazolo[3,4,5-de] [4,1]benzothiazepine 6-oxide,
- (-)-2-imino-9-trifluoromethyl-4,5-dihydro-2H,7H-thiazolo[3,4,5-de] [4,1]benzothiazepine 6-oxide,
- (R,S)-2-imino-5-methyl-9-trifluoromethyl-4,5-dihydro-2H,7H-thiazolo[3,4,5-de] [4,1]benzothiazepine,
- (+)-2-imino-5-methyl-9-trifluoromethyl-4,5-dihydro-2H,7H-thiazolo[3,4,5-de] [4,1]benzothiazepine,
- (-)-2-imino-5-methyl-9-trifluoromethyl-4,5-dihydro-2H,7H-thiazolo[3,4,5-de][4,1]benzothiazepine
and their salts with an inorganic or organic acid.

6. Process for the preparation of the compounds of formula (I) according to Claim 1 for which R₁ represents a sulphur or selenium atom, R₂ represents a hydrogen atom, -R₃-R₄-R₅-R₆- represents a chain of formula -CH₂-CH₂-CH₂-CH₂-, -CH₂-CH₂-CH₂-CO-, -CH₂-CH₂-CH₂-CH(R₈)-, -CH₂-CH₂-CH₂-Se-, -CH₂-CH₂-Se-CH₂-, -CH₂-CH₂-CH₂-S-, -CH₂-CH₂-CH₂-O-, -CH₂-CH₂-CH₂-N(R₉)-, -CH₂-CH₂-CO-CH₂-, -CH₂-CH₂-CH(R₈)-CH₂-, -CH₂-CH₂-S-CH₂-, -CH₂-C(alk) (alk')-S-CH₂-, -CH₂-CH(R₁₀)-S-CH₂-, -CH₂-CH₂-O-CH₂-, -CH₂-CH₂-N(R₉)-CH₂- or -CH₂-CO-N(R₉)-CH₂-, R₈ represents a hydroxyl radical, R₉ represents a hydrogen atom or an alkyl or benzyl radical, R₁₀ represents an alkyl, -COOalk, or -CONH₂ radical, alk represents an alkyl radical and alk' represents an alkyl radical, **characterized in that** an alkali metal thiocyanate or alkali metal selenocyanate is reacted with a derivative of formula: 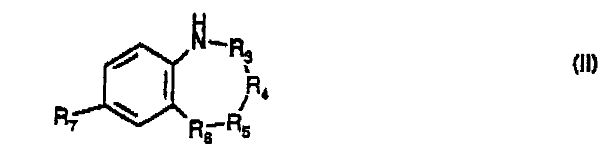 in which R₇ has the same meanings as in Claim 1 and -R₃-R₄-R₅-R₆- represents a chain of formula -CH₂-CH₂-CH₂-CH₂-, -CH₂-CH₂-CH₂-CO-, -CH₂-CH₂-CH₂-CH(R₈)-, -CH₂-CH₂-CH₂-Se-, -CH₂-CH₂-Se-CH₂-, -CH₂-CH₂-CH₂-S-, -CH₂-CH₂-CH₂-O-, CH₂-CH₂-CH₂-N(R₉)-, -CH₂-CH₂-CO-CH₂-, -CH₂-CH₂-CH(R₈)-CH₂-, -CH₂-CH₂-S-CH₂-, -CH₂-C (alk)(alk')-S-CH₂-, -CH₂-CH(R₁₀)-S-CH₂-, -CH₂-CH₂-O-CH₂-, -CH₂-CH₂-N(R₉)-CH₂- or -CH₂-CO-N(R₉)-CH₂-, R₈ represents a hydroxyl radical, R₉ represents a hydrogen atom or an alkyl or benzyl radical and R₁₀ represents an alkyl, COOalk or CONH₂ radical, alk represents an alkyl radical and alk' represents an alkyl radical, the product isolated and optionally converted to a salt.

7. Process for the preparation of the compounds of formula (I) according to Claim 1 for which R₂ represents an alkyl radical, **characterized in that** a corresponding compound of formula (I) for which R₂ represents a hydrogen atom is alkylated, the product isolated and optionally converted to a salt.

8. Process for the preparation of the compounds of formula (I) according to Claim 1 for which R₂ represents a hydrogen atom or an alkyl radical, -R₃-R₄-R₅-R₆- represents a chain of formula -CH₂-CH₂-CH(R₈)-CH₂- or -CH₂-CH₂-CH₂-CH(R₈)- and R₈ represents a hydroxyl radical, **characterized in that** a corresponding compound of formula (I) for which R₂ represents a hydrogen atom or an alkyl radical and -R₃-R₄-R₅-R₆- represents a chain of formula -CH₂-CH₂-CO-CH₂- or -CH₂-CH₂-CH₂-CO- is reduced, the product isolated and optionally converted to a salt.

9. Process for the preparation of the compounds of formula (I) according to Claim 1 for which R₂ represents a hydrogen atom or an alkyl radical and -R₃-R₄-R₅-R₆- represents a chain of formula -CH₂-CH₂-CH₂-SO-, -CH₂-CH₂-CH₂-SO₂-, -CH₂-CH₂-SO-CH₂-, -CH₂-CH₂-SO₂-CH₂-, -CH₂-C(alk)(alk')-SO-CH₂-, -CH₂-C(alk)(alk')-SO₂-CH₂-, -CH₂-CH(R₁₀)-SO-CH₂- or -CH₂-CH(R₁₀)-SO₂-CH₂-, **characterized in that** a corresponding compound of formula (I) for which the chain -R₃-R₄-R₅-R₆- represents a chain of formula -CH₂-CH₂-CH₂-S-, -CH₂-CH₂-S-CH₂-, -CH₂-C(alk)(alk')-S-CH₂- or -CH₂-CH(R₁₀)-S-CH₂- is oxidized, the product isolated and optionally converted to a salt.

10. Process for the preparation of the compounds of formula (I) according to Claim 1 for which R₂ represents a hydrogen atom or an alkyl radical, -R₃-R₄-R₅-R₆- represents a chain of formula -CH₂-CH(R₁₀)-S-CH₂- in which R₁₀ represents a -COOH radical, **characterized in that** a corresponding compound of formula (I) for which R₂ represents a hydrogen atom or an alkyl radical, -R₃-R₄-R₅-R₆- represents a chain of formula -CH₂-CH(R₁₀)-S-CH₂- in which R₁₀ represents a -COOalk radical is hydrolysed, the product isolated and optionally converted to a salt.

11. Medicaments containing, as active ingredient, at least one compound according to one of Claims 1 to 5 or a pharmaceutically acceptable salt of such a compound with an inorganic or organic acid.

12. Derivatives of formula: 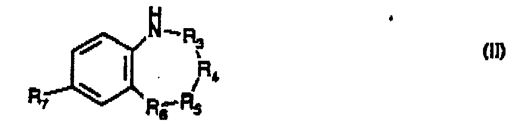 in which -R₃-R₄-R₅-R₆- represents a chain of formula -CH₂-CH₂-CH₂-CH₂-, -CH₂-CH₂-CH₂-CO-, -CH₂-CH₂-CH₂-CH(R₈)-, -CH₂-CH₂-CH₂-Se-, -CH₂-CH₂-Se-CH₂-, -CH₂-CH₂-CH₂-S-, -CH₂-CH₂-CH₂-O-, -CH₂-CH₂-CH₂-N(R₉)-, -CH₂-CH₂-CO-CH₂-, -CH₂-CH₂-CH(R₈)-CH₂-, -CH₂-CH₂-S-CH₂-, -CH₂-C(alk) (alk' )-S-CH₂-, -CH₂-CH(R₁₀)-S-CH₂-, -CH₂-CH₂-O-CH₂-, -CH₂-CH₂-N(R₉)-CH₂- or -CH₂-CO-N(R₉)-CH₂-, R₇ represents a polyfluoroalkyl or polyfluoroalkoxy radical, R₈ represents a hydroxyl radical, R₉ represents a hydrogen atom or an alkyl or benzyl radical, R₁₀ represents an alkyl, -COOalk, or -CONH₂ radical, alk represents an alkyl radical and alk' represents an alkyl radical, in these definitions, the alkyl radicals and portions contain 1 to 6 straight-chain or branched carbon atoms.

13. Pharmaceutical compositions containing a medicament as defined in Claim 6 and a pharmaceutically compatible excipient.

## Patentansprüche

1. Verbindungen der Formel (I) 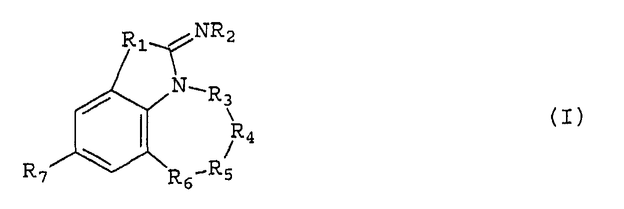 in der
R₁ ein Atom von Schwefel oder Selen darstellt,
R₂ ein Wasserstoffatom oder einen Rest Alkyl bedeutet,
-R₃-R₄-R₅-R₆- eine Kette der folgenden Formeln darstellt: -CH₂-CH₂-CH₂-CH₂-, -CH₂-CH₂-CH₂-CO-, -CH₂-CH₂-CH₂-CH(R₈)-, -CH₂-CH₂-CH₂-Se-, -CH₂-CH₂-Se-CH₂-, -CH₂-CH₂-CH₂-S-, -CH₂-CH₂-CH₂-SO-, -CH₂-CH₂-CH₂-SO₂-, -CH₂-CH₂-CH₂-O-, -CH₂-CH₂-CH₂-N(R₉)-, -CH₂-CH₂-CO-CH₂-, -CH₂-CH₂-CH(R₈)-CH₂-, -CH₂-CH₂-S-CH₂-, -CH₂-CH₂-SO-CH₂-, -CH₂-CH₂-SO₂-CH₂-, -CH₂-C(alk)(alk')-S-CH₂-, -CH₂-C(alk)(alk')-SO-CH₂-, -CH₂-C(alk)(alk')-SO₂-CH₂-, -CH₂-CH(R₁₀)-S-CH₂-, -CH₂-CH(R₁₀)-SO-CH₂-, -CH₂-CH(R₁₀)-SO₂-CH₂-, -CH₂-CH₂-O-CH₂-, -CH₂-CH₂-N(R₉)-CH₂- oder -CH₂-CO-N(R₉)-CH₂-,
R₇ einen Rest Polyfluoralkyl oder Polyfluoralkoxy bedeutet,
R₈ ein Rest Hydroxy ist,
R₉ ein Wasserstoffatom oder einen Rest Alkyl oder Benzyl darstellt,
R₁₀ einen Rest Alkyl, -CH₂OH, -COOalk, -COOH oder -CONH₂ bedeutet,
alk ein Rest Alkyl ist,
alk' ein Rest Alkyl ist,
wobei in diesen Definitionen die Reste und Teile Alkyl 1 bis 6 Kohlenstoffatome in gerader oder verzweigter Kette enthalten, und wenn sie ein oder mehrere asymmetrische Zentren umfassen,
ihre Isomeren, Racemate und Enantiomeren und ihre Salze mit einer Mineralsäure oder organischen Säure.

2. Verbindungen der Formel (I) nach Anspruch 1, worin R₇ einen Rest Trifluormethoxy oder Trifluormethyl darstellt, und wenn sie ein oder mehrere asymmetrische Zentren umfassen, ihre Isomeren, Racemate und Enantiomeren und ihre Salze mit einer Mineralsäure oder organischen Säure.

3. Verbindungen der Formel (I) nach Anspruch 1, worin R₁ ein Schwefelatom ist, R₂ ein Wasserstoffatom ist, -R₃-R₄-R₅-R₆- eine Kette der folgenden Formeln darstellt: -CH₂-CH₂-CH₂-CH₂-, -CH₂-CH₂-CH₂-CO-, -CH₂-CH₂-CH₂-CH(R₈)-, -CH₂-CH₂-CH₂-Se-, -CH₂-CH₂-Se-CH₂-, -CH₂-CH₂-CH₂-S-, -CH₂-CH₂-CH₂-SO-, -CH₂-CH₂-CH₂-SO₂-, -CH₂-CH₂-CH₂-O-, -CH₂-CH₂-CH₂-N(R₉)-, -CH₂-CH₂-CO-CH₂-, -CH₂-CH₂-CH(R₈)-CH₂-, -CH₂-CH₂-S-CH₂-, -CH₂-CH₂-SO-CH₂-, -CH₂-CH₂-SO₂-CH₂-, -CH₂-C(alk)(alk')-S-CH₂-, -CH₂-C(alk)(alk')-SO-CH₂-, -CH₂-C(alk)(alk')-SO₂-CH₂-, -CH₂-CH(R₁₀)-S-CH₂-, -CH₂-CH(R₁₀)-SO-CH₂-, -CH₂-CH(R₁₀)-SO₂-CH₂-, -CH₂-CH₂-O-CH₂-, -CH₂-CH₂-N(R₉)-CH₂- oder -CH₂-CO-N(R₉)-CH₂-, R₇ einen Rest Trifluormethyl oder Trifluormethoxy bedeutet, R₈ ein Rest Hydroxy ist, R₉ ein Wasserstoffatom oder einen Rest Alkyl oder Benzyl darstellt, R₁₀ einen Rest Alkyl, -CH₂OH, -COOalk, -COOH oder -CONH₂ bedeutet, alk ein Rest Alkyl ist und alk' ein Rest Alkyl ist,
und wenn sie ein oder mehrere asymmetrische Zentren umfassen, ihre Isomeren, Racemate und Enantiomeren und ihre Salze mit einer Mineralsäure oder organischen Säure.

4. Verbindungen der Formel (I) nach Anspruch 1, ausgewählt unter den folgenden Verbindungen:
- 2-Imino-9-trifluormethoxy-4,5,6,7-tetrahydro-2H-thiazolo[5,4,3-jk][1]-benzazepin-7-ol,
- 2-Imino-9-trifluormethoxy-4,5,6,7-tetrahydro-2H-thiazolo[5,4,3-jk][1]-benzazepin,
- 2-Imino-9-trifluormethyl-4,5,6,7-tetrahydro-2H-thiazolo[5,4,3-jk][1]-benzazepin,
- 2-Imino-9-trifluormethoxy-5,6-dihydro-2H,4H-thiazolo[3,4,5-ef]-[1,5]-benzothiazepin-7,7-dioxid,
- 2-Imino-9-trifluormethoxy-5,6-dihydro-2H,4H-thiazolo[3,4,5-ef]-[1,5]-benzothiazepin-7-oxid,
- 2-Imino-9-trifluormethoxy-5,6-dihydro-2H,4H-thiazolo[3,4,5-ef]-[1,5]-benzothiazepin,
- 6-Benzyl-2-imino-9-trifluormethoxy-6,7-dihydro-4H-thiazolo[3,4,5-kj]-[1,4]-benzodiazepin-5-on,
- 6-Benzyl-2-imino-9-trifluormethoxy-4,5,6,7-tetrahydro-2H-thiazolo-[3,4,5-kj]-[1,4]-benzodiazepin,
- 2-Imino-9-trifluormethoxy-4,5-dihydro-2H,7H-thiazolo[3,4,5-de][4,1]-benzothiazepin,
- 2-Imino-9-trifluormethyl-4,5-dihydro-2H,7H-thiazolo[3,4,5-de]-[4,1]-benzothiazepin,
- 2-Imino-9-trifluormethyl-4,5-dihydro-2H,7H-thiazolo[3,4,5-de][4,1]-benzothiazepin-6,6-dioxid,
- 2-Imino-9-trifluormethyl-5,6-dihydro-2H,4H-thiazolo[3,4,5-ef]-[1,5]-benzothiazepin-7-oxid,
- 2-Imino-9-trifluormethyl-5,6-dihydro-2H,4H-thiazolo[3,4,5-ef][1,5]-benzothiazepin-6,6-dioxid,
- 2-Imino-9-trifluormethyl-5,6-dihydro-2H,4H-thiazolo[3,4,5-ef]-[1,5]-benzothiazepin,
- 2-Imino-9-trifluormethyl-4,5,6,7-tetrahydro-2H-thiazolo[5,4,3-jk]-[1]-benzazepin-7-ol,
- 2-Imino-9-trifluormethoxy-4,5-dihydro-2H,7H-thiazolo[3,4,5-de]-[4,1]-benzothiazepin-6,6-dioxid,
- 2-Imino-9-trifluormethoxy-4,5-dihydro-2H,7H-thiazolo[3,4,5-de][4,1]-benzothiazepin-6-oxid,
- 6-Benzyl-2-imino-9-trifluormethyl-6,7-dihydro-4H-thiazolo[3,4,5-kj] [1,4]-benzodiazepin-5-on,
- 6-Benzyl-2-imino-9-trifluormethyl-4,5,6,7-tetrahydro-2H-thiazolo[3,4,5-kj] [1,4]-benzothiazepin,
- 2-Imino-5-methyl-9-trifluormethyl-4,5-dihydro-2H,7H-thiazolo[3,4,5-de] [4,1]-benzothiazepin,
- 5-Carbamoyl-2-imino-9-trifluormethyl-4,5-dihydro-2H,7H-thiazolo[3,4,5-de][4,1]-benzothiazepin,
- 5,5-Dimethyl-2-imino-9-trifluormethyl-2H,4H,7H-thiazolo[3,4,5-de][4,1]-benzothiazepin,
- 5-Hydroxymethyl-2-imino-9-trifluormethyl-4,5-dihydro-2H,7Hthiazolo-[3,4,5-de][4,1]-benzothiazepin,
und wenn sie ein oder mehrere asymmetrische Zentren umfassen, ihre Isomeren, Racemate und Enantiomeren und ihre Salze mit einer Mineralsäure oder organischen Säure.

5. Verbindungen der Formel (I) nach Anspruch 1, ausgewählt unter den folgenden Verbindungen:
- (R,S)-2-Imino-9-trifluormethyl-4,5-dihydro-2H,7H-thiazolo[3,4,5-de] [4,1] -benzothiazepin-6-oxid,
- (+)-2-Imino-9-trifluormethyl-4,5-dihydro-2H,7H-thiazolo[3,4,5-de] [4,1]-benzothiazepin-6-oxid,
- (-)-2-Imino-9-trifluormethyl-4,5-dihydro-2H,7H-thiazolo[3,4,5-de][4,1]-benzothiazepin-6-oxid,
- (R,S)-2-Imino-5-methyl-9-trifluormethyl-4,5-dihydro-2H,7Hthiazolo[3,4,5-de] [4,1]-benzothiazepin,
- (+)-2-Imino-5-methyl-9-trifluormethyl-4,5-dihydro-2H,7Hthiazolo[3,4,5-de] [4,1]-benzothiazepin,
- (-)-2-Imino-5-methyl-9-trifluormethyl-4,5-dihydro-2H,7Hthiazolo[3,4,5-de][4,1]-benzothiazepin,
und ihre Salze mit einer Mineralsäure oder organischen Säure.

6. Verfahren zur Herstellung der Verbindungen der Formel (I) nach Anspruch 1, in der R₁ ein Atom von Schwefel oder Selen darstellt, R₂ ein Wasserstoffatom bedeutet, -R₃-R₄-R₅-R₆- eine Kette der folgenden Formeln darstellt: -CH₂-CH₂-CH₂-CH₂-, -CH₂-CH₂-CH₂-CO-, -CH₂-CH₂-CH₂-CH(R₈)-, -CH₂-CH₂-CH₂-Se-, -CH₂-CH₂-Se-CH₂-, -CH₂-CH₂-CH₂-S-, -CH₂-CH₂-CH₂-O-, -CH₂-CH₂-CH₂-N(R₉)-, -CH₂-CH₂-CO-CH₂-, -CH₂-CH₂-CH(R₈)-CH₂-, -CH₂-CH₂-S-CH₂-, -CH₂-C(alk)(alk')-S-CH₂-, -CH₂-CH(R₁₀)-S-CH₂-, -CH₂-CH₂-O-CH₂-, -CH₂-CH₂-N(R₉)-CH₂- oder -CH₂-CO-N(R₉)-CH₂-, R₈ ein Rest Hydroxy ist, R₉ ein Wasserstoffatom oder einen Rest Alkyl oder Benzyl darstellt, R₁₀ einen Rest Alkyl, -COOalk oder -CONH₂ bedeutet, alk ein Rest Alkyl ist, alk' ein Rest Alkyl ist, **dadurch gekennzeichnet, daß** man ein Alkalimetallthiocyanat oder ein Alkalimetall-selenocyanat mit einem Derivat der Formel (II) 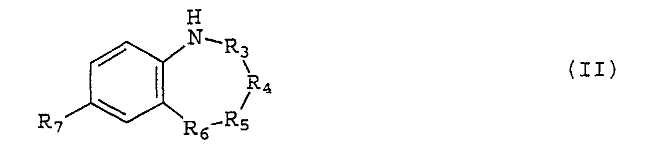 zur Reaktion bringt, in der R₇ die gleichen Bedeutungen wie in Anspruch 1 besitzt und -R₃-R₄-R₅-R₆- eine Kette der folgenden Formeln darstellt: -CH₂-CH₂-CH₂-CH₂-, -CH₂-CH₂-CH₂-CO-, -CH₂-CH₂-CH₂-CH(R₈)-, -CH₂-CH₂-CH₂-Se-, -CH₂-CH₂-Se-CH₂-, -CH₂-CH₂-CH₂-S-, -CH₂-CH₂-CH₂-O-, -CH₂-CH₂-CH₂-N(R₉)-, -CH₂-CH₂-CO-CH₂-, -CH₂-CH₂-CH(R₈)-CH₂-, -CH₂-CH₂-S-CH₂-, -CH₂-C(alk) (alk')-S-CH₂-, -CH₂-CH(R₁₀)-S-CH₂-, -CH₂-CH₂-O-CH₂-, -CH₂-CH₂-N(R₉)-CH₂- oder -CH₂-CO-N(R₉)-CH₂-, R₈ ein Rest Hydroxy ist, R₉ ein Wasserstoffatom oder einen Rest Alkyl oder Benzyl darstellt, R₁₀ einen Rest Alkyl, -COOalk oder -CONH₂ bedeutet, alk ein Rest Alkyl ist, alk' ein Rest Alkyl ist, das Produkt isoliert und gegebenenfalls in Salz überführt.

7. Verfahren zur Herstellung der Verbindungen der Formel (I) nach Anspruch 1, worin R₂ ein Rest Alkyl ist, **dadurch gekennzeichnet, daß** man eine entsprechende Verbindung der Formel (I), worin R₂ ein Wasserstoffatom ist, alkyliert, das Produkt isoliert und gegebenenfalls in Salz überführt.

8. Verfahren zur Herstellung der Verbindungen der Formel (I) nach Anspruch 1, worin R₂ ein Wasserstoffatom oder ein Rest Alkyl ist, -R₃-R₄-R₅-R₆- eine Kette der Formel -CH₂-CH₂-CH(R₈)-CH₂- oder -CH₂-CH₂-CH₂-CH(R₈) - darstellt und R₈ einen Rest Hydroxy bedeutet, **dadurch gekennzeichnet, daß** man eine entsprechende Verbindung der Formel (I), worin R₂ ein Wasserstoffatom oder ein Rest Alkyl ist und -R₃-R₄-R₅-R₆- eine Kette der Formel -CH₂-CH₂-CO-CH₂- oder -CH₂-CH₂-CH₂-CO- darstellt, reduziert, das Produkt isoliert und gegebenenfalls in Salz überführt.

9. Verfahren zur Herstellung der Verbindungen der Formel (I) nach Anspruch 1, worin R₂ ein Wasserstoffatom oder ein Rest Alkyl ist und -R₃-R₄-R₅-R₆- eine Kette der folgenden Formeln darstellt : -CH₂-CH₂-CH₂-SO-, -CH₂-CH₂-CH₂-SO₂-, -CH₂-CH₂-SO-CH₂-, -CH₂-CH₂-SO₂-CH₂-, -CH₂-C(alk) (alk')-SO-CH₂-, -CH₂-C(alk) (alk')-SO₂-CH₂-, -CH₂-CH(R₁₀)-SO-CH₂- oder -CH₂-CH(R₁₀)-SO₂-CH₂-, **dadurch gekennzeichnet, daß** man eine entsprechende Verbindung der Formel (I), worin -R₃-R₄-R₅-R₆- eine Kette der folgenden Formeln darstellt : -CH₂-CH₂-CH₂-S-, -CH₂-CH₂-S-CH₂-, -CH₂-C(alk) (alk')-S-CH₂- oder -CH₂-CH(R₁₀)-S-CH₂-, oxidiert, das Produkt isoliert und gegebenenfalls in Salz überführt.

10. Verfahren zur Herstellung der Verbindungen der Formel (I) nach Anspruch 1, worin R₂ ein Wasserstoffatom oder ein Rest Alkyl ist und -R₃-R₄-R₅-R₆- eine Kette der Formel -CH₂-CH(R₁₀)-S-CH₂- darstellt, worin R₁₀ ein Rest -COOH ist, **dadurch gekennzeichnet, daß** man eine entsprechende Verbindung der Formel (I), worin R₂ ein Wasserstoffatom oder ein Rest Alkyl ist und -R₃-R₄-R₅-R₆- eine Kette der Formel -CH₂-CH(R₁₀)-S-CH₂- darstellt, worin R₁₀ ein Rest -COOalk ist, hydrolysiert, das Produkt isoliert und gegebenenfalls in Salz überführt.

11. Arzneimittel, enthaltend als Wirkstoff mindestens eine Verbindung nach einem der Ansprüche 1 bis 5 oder ein pharmazeutisch akzeptables Salz einer derartigen Verbindung mit einer Mineralsäure oder organischen Säure.

12. Derivate der Formel (II) 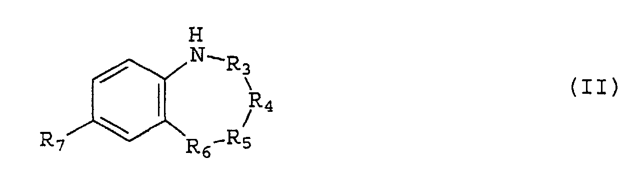 in der -R₃-R₄-R₅-R₆- eine Kette der folgenden Formeln darstellt: -CH₂-CH₂-CH₂-CH₂-, -CH₂-CH₂-CH₂-CO-, -CH₂-CH₂-CH₂-CH(R₈)-, -CH₂-CH₂-CH₂-Se-, -CH₂-CH₂-Se-CH₂-, -CH₂-CH₂-CH₂-S-, -CH₂-CH₂-CH₂-O-, -CH₂-CH₂-CH₂-N(R₉)-, -CH₂-CH₂-CO-CH₂-, -CH₂-CH₂-CH(R₈)-CH₂-, -CH₂-CH₂-S-CH₂-, -CH₂-C(alk)(alk')-S-CH₂-, -CH₂-CH(R₁₀)-S-CH₂-, -CH₂-CH₂-O-CH₂-, -CH₂-CH₂-N(R₉)-CH₂- oder -CH₂-CO-N(R₉)-CH₂-, R₇ einen Rest Polyfluoralkyl oder Polyfluoralkoxy bedeutet, R₈ ein Rest Hydroxy ist, R₉ ein Wasserstoffatom oder einen Rest Alkyl oder Benzyl darstellt, R₁₀ einen Rest Alkyl, -COOalk, oder -CONH₂ bedeutet, alk ein Rest Alkyl ist und alk' ein Rest Alkyl ist, wobei in diesen Definitionen die Reste und Teile Alkyl 1 bis 6 Kohlenstoffatome in gerader oder verzweigter Kette enthalten.

13. Pharmazeutische Zusammensetzungen, umfassend ein Arzneimittel wie in Anspruch 6 definiert und einen pharmazeutisch akzeptablen Füllstoff.
